(19) Europäisches Patentamt
European Patent Office
Office européen des brevets

(11) EP 4 095 148 A1

(12) # EUROPEAN PATENT APPLICATION
published in accordance with Art. 153(4) EPC

(43) Date of publication:
**30.11.2022 Bulletin 2022/48**

(21) Application number: **21744789.5**

(22) Date of filing: **22.01.2021**

(51) International Patent Classification (IPC):
***C07K 7/02*** *(2006.01)* ***C07K 5/062*** *(2006.01)*
***A61K 39/395*** *(2006.01)* ***A61K 31/506*** *(2006.01)*
***C07D 491/22*** *(2006.01)* ***A61P 35/00*** *(2006.01)*

(52) Cooperative Patent Classification (CPC):
**A61K 31/506; A61K 39/395; A61P 35/00; C07D 491/22; C07K 5/06017; C07K 7/02**

(86) International application number:
**PCT/CN2021/073279**

(87) International publication number:
**WO 2021/147993 (29.07.2021 Gazette 2021/30)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.01.2020 CN 202010073438**

(71) Applicants:
- **Jiangsu Hengrui Medicine Co., Ltd.**
  **Lianyungang, Jiangsu 222047 (CN)**
- **Shanghai Hengrui Pharmaceutical Co., Ltd.**
  **Shanghai 200245 (CN)**

(72) Inventors:
- **YANG, Yang**
  **Shanghai 200245 (CN)**
- **HU, Qiyue**
  **Shanghai 200245 (CN)**
- **TAO, Weikang**
  **Shanghai 200245 (CN)**

(74) Representative: **Potter Clarkson**
**The Belgrave Centre**
**Talbot Street**
**Nottingham NG1 5GG (GB)**

Remarks:
The complete document including Reference Table(s) and the Sequence Listing(s) can be downloaded from the EPO website

(54) **ANTI-TROP-2 ANTIDODY-EXATECAN ANALOG CONJUGATE AND MEDICAL USE THEREOF**

(57) Provided in the present invention are an anti-TROP-2 antibody-exatecan analog conjugate and the medical use thereof. Specifically, provided in the present invention is an anti-TROP-2 antibody-exatecan analog conjugate represented by the general formula (Pc-L-Y-D), wherein Pc is an anti-TROP-2 antibody or an antigen-binding fragment thereof.

EP 4 095 148 A1

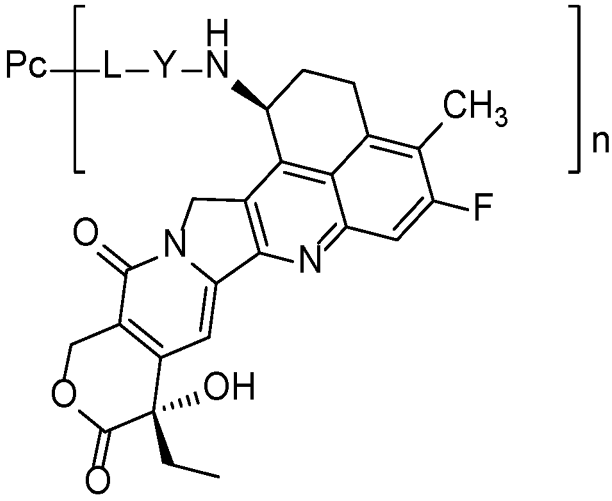

(Pc-L-Y-D)

## Description

The present application claims priority to Chinese Patent Application (Patent Application No. CN202010073438.8) filed on Jan. 22, 2020.

## TECHNICAL FIELD

The present disclosure relates to an anti-TROP-2 antibody and an anti-TROP-2 antibody-exatecan analog conjugate, a preparation method therefor, a pharmaceutical composition comprising the same, and use thereof in preparing a medicament for the treatment of a TROP-2-mediated disease or condition, especially use thereof in preparing an anti-cancer medicament.

## BACKGROUND

The statement herein merely provides background information related to the present disclosure and may not necessarily constitute the prior art.

TROP-2, human trophoblast cell surface glycoprotein antigen 2, also known as tumor-associated calcium signal transducer 2 (TACSTD2), epidermal glycoprotein 1 (EGP-1), gastrointestinal tumor-associated antigen (GA733-1) and surface marker 1 (M1S1), is a cell surface glycoprotein encoded and expressed by Tacstd2 gene in the 1p32 region of chromosome. TROP-2 is a member in GA733 protein family, and has higher structural sequence similarity to epithelial cell adhesion molecules (EpCAM, also known as Trop1 and TACSTD1) with homology of 49%.

The primary structure of TROP-2 protein is an about 36 kD polypeptide consisting of about 323 amino acids, and the primary structure is modified post-translationally by N-terminal glycosylation to form type I cell membrane glycoprotein different from EpCAM, i.e., TROP-2 protein. The TROP-2 protein spans the cell membrane and has an N-terminal extracellular domain (Trop2EC), and the extracellular domain is immobilized to the cell membrane by a unidirectional transmembrane helix (TM) connected to a short intracellular tail (Trop2IC) of a hydrophobic polypeptide consisting of 26 amino acid residues.

It is found at present that TROP-2 has important significance in the processes of embryonic development and tumor cell proliferation and metastasis. TROP-2 is originally discovered in a trophoblast cell and served as a surface marker thereof, the trophoblast cell is derived from extra-embryonic trophoblast, and TROP-2 contributes to embryo implantation and placental tissue formation, and plays an important role in the maintenance of proliferative characteristics of embryonic stem cells and the formation and development of organs. In addition, TROP-2 is also an important tumor development related factor, is highly expressed in various tumors, such as pancreatic cancer, breast cancer, colon cancer, gastric cancer, oral squamous carcinoma and ovarian cancer, can promote the processes of tumor cell proliferation, invasion, metastasis, diffusion and the like, and is closely related to the shortened survival and poor prognosis of tumor subjects, so that it is of great significance to research on an anti-tumor drug targeting TROP-2. Antibody-drug conjugate (ADC) links a monoclonal antibody or an antibody fragment to a biologically active cytotoxin via a linker compound, fully exploiting the binding specificity of the antibody to surface antigens of normal cells and tumor cells and the high-efficiency of the cytotoxic substance, and also avoiding defects of poor therapeutic effect of the antibody and serious toxic side effects of the toxic substance. This means that the antibody-drug conjugate can kill tumor cells more precisely and has a reduced effect on normal cells compared to conventional chemotherapeutic drugs in the past.

## SUMMARY

The present disclosure relates to an anti-TROP-2 antibody, an ADC thereof and use thereof, and provides an ADC drug in which an anti-TROP-2 antibody or an antigen-binding fragment is conjugated with an exatecan analog, a cytotoxic substance.

The present disclosure provides a ligand-drug conjugate of general formula (Pc-L-Y-D) or a pharmaceutically acceptable salt thereof:

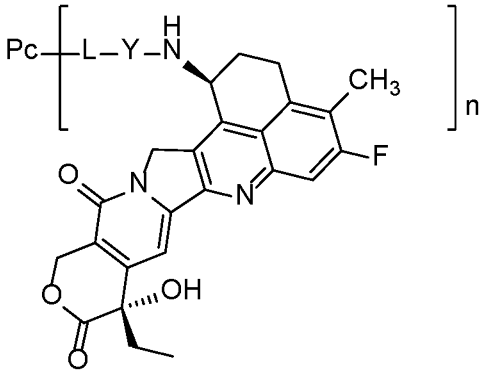

(Pc-L-Y-D)

wherein:

Y is selected from the group consisting of $-O-(CR^aR^b)_m-CR^1R^2-C(O)-$, $-O-CR^1R^2-(CR^aR^b)_m-$, $-O-CR^1R^2-$, $-NH-(CR^aR^b)_m-CR^1R^2-C(O)-$ and $-S-(CR^aR^b)_m-CR^1R^2-C(O)-$;

$R^a$ and $R^b$ are identical or different and are each independently selected from the group consisting of hydrogen, deuterium, halogen, alkyl, haloalkyl, deuterated alkyl, alkoxy, hydroxy, amino, cyano, nitro, hydroxyalkyl, cycloalkyl and heterocyclyl; or, $R^a$ and $R^b$, together with carbon atoms connected thereto, form cycloalkyl or heterocyclyl;

$R^1$ is selected from the group consisting of halogen, haloalkyl, deuterated alkyl, cycloalkyl, cycloalkylalkyl, alkoxyalkyl, heterocyclyl, aryl and heteroaryl; $R^2$ is selected from the group consisting of hydrogen, halogen, haloalkyl, deuterated alkyl, cycloalkyl, cycloalkylalkyl, alkoxyalkyl, heterocyclyl, aryl and heteroaryl; or, $R^1$ and $R^2$, together with carbon atoms connected thereto, form cycloalkyl or heterocyclyl;

or, $R^a$ and $R^2$, together with carbon atoms connected thereto, form cycloalkyl or heterocyclyl;

m is an integer from 0 to 4; non-limiting examples of m are, for example, m is selected from the group consisting of 0, 1, 2, 3 and 4;

n is a decimal or an integer from 1 to 10;

L is a linker unit;

Pc is an anti-TROP-2 antibody or an antigen-binding fragment thereof.

In some embodiments, in the ligand-drug conjugate of general formula (Pc-L-Y-D) or the pharmaceutically acceptable salt thereof as described above, the anti-TROP-2 antibody or the antigen-binding fragment thereof comprises a heavy chain variable region and a light chain variable region, wherein the heavy chain variable region comprises an HCDR1, an HCDR2 and an HCDR3 having sequences identical to those of an HCDR1, an HCDR2 and an HCDR3 of a heavy chain variable region set forth in SEQ ID NO: 3, and the light chain variable region comprises an LCDR1, an LCDR2 and an LCDR3 having sequences identical to those of an LCDR1, an LCDR2 and an LCDR3 of a light chain variable region set forth in SEQ ID NO: 4.

In some embodiments, in the ligand-drug conjugate of general formula (Pc-L-Y-D) or the pharmaceutically acceptable salt thereof according to any one of the aforementioned embodiments, the anti-TROP-2 antibody or the antigen-binding fragment thereof comprises a heavy chain variable region and a light chain variable region, wherein the heavy chain variable region comprises an HCDR1, an HCDR2 and an HCDR3 set forth in SEQ ID NO: 5, SEQ ID NO: 6 and SEQ ID NO: 7, respectively, and the light chain variable region comprises an LCDR1, an LCDR2 and an LCDR3 set forth in SEQ ID NO: 8, SEQ ID NO: 9 and SEQ ID NO: 10, respectively.

In some embodiments, in the ligand-drug conjugate of general formula (Pc-L-Y-D) or the pharmaceutically acceptable salt thereof according to any one of the aforementioned embodiments, the anti-TROP-2 antibody is a murine antibody, a chimeric antibody, a humanized antibody or a human antibody.

In some embodiments, in the ligand-drug conjugate of general formula (Pc-L-Y-D) or the pharmaceutically acceptable salt thereof according to any one of the aforementioned embodiments, the anti-TROP-2 antibody or the antigen-binding fragment thereof comprises a heavy chain variable region and a light chain variable region, wherein the heavy chain variable region has an amino acid sequence set forth in SEQ ID NO: 3 or having at least 90%-100% identity thereto including, but not limited to at least 91%, at least 92%, at least 93%, at least 94%, at least 95%, at least 96%, at least 97%, at least 98%, at least 99% or at least 100%, and the light chain variable region has an amino acid sequence set forth in SEQ ID NO: 4 or having at least 90%-100% identity thereto including, but not limited to least 91%, at least 92%, at least 93%, at least 94%, at least 95%, at least 96%, at least 97%, at least 98%, at least 99% or at least 100%.

In some embodiments, in the ligand-drug conjugate of general formula (Pc-L-Y-D) or the pharmaceutically

acceptable salt thereof according to any one of the aforementioned embodiments, the anti-TROP-2 antibody or the antigen-binding fragment thereof comprises a heavy chain variable region set forth in SEQ ID NO: 3 and a light chain variable region set forth in SEQ ID NO: 4.

In some embodiments, in the ligand-drug conjugate of general formula (Pc-L-Y-D) or the pharmaceutically acceptable salt thereof according to any one of the aforementioned embodiments, the anti-TROP-2 antibody or the antigen-binding fragment thereof comprises a heavy chain constant region and a light chain constant region; preferably, the heavy chain constant region is selected from the group consisting of human IgG1, IgG2, IgG3 and IgG4 constant regions, and the light chain constant region is selected from the group consisting of human antibody $\kappa$ and $\lambda$ chain constant regions; and more preferably, the antibody comprises a heavy chain constant region having a sequence set forth in SEQ ID NO: 11 and a light chain constant region having a sequence set forth in SEQ ID NO: 12.

In some embodiments, in the ligand-drug conjugate of general formula (Pc-L-Y-D) or the pharmaceutically acceptable salt thereof according to any one of the aforementioned embodiments, the anti-TROP-2 antibody comprises a heavy chain set forth in SEQ ID NO: 13 and a light chain set forth in SEQ ID NO: 14.

In some embodiments, in the ligand-drug conjugate of general formula (Pc-L-Y-D) or the pharmaceutically acceptable salt thereof as described above, the anti-TROP-2 antibody comprises a heavy chain having a sequence set forth in SEQ ID NO: 15 and a light chain having a sequence set forth in SEQ ID NO: 16, or comprises a heavy chain having a sequence set forth in SEQ ID NO: 17 and a light chain having a sequence set forth in SEQ ID NO: 18.

In some embodiments, in the ligand-drug conjugate of general formula (Pc-L-Y-D) or the pharmaceutically acceptable salt thereof according to any one of the aforementioned embodiments, n is a decimal or an integer from 2 to 10, preferably from 4 to 8. In some embodiments, n is an average value of 0 to 10, preferably 1 to 10, more preferably 1 to 8, or 2 to 8, or 2 to 7, or 2 to 4, or 3 to 8, or 3 to 7, or 3 to 6, or 4 to 7, or 4 to 6, or 4 to 5; in some embodiments, n is a mean of 1, 2, 3, 4, 5, 6, 7, 8, 9 and 10.

In some embodiments, in the ligand-drug conjugate of general formula (Pc-L-Y-D) or the pharmaceutically acceptable salt thereof according to any one of the aforementioned embodiments,
wherein:

- Y is $-O-(CR^aR^b)_m-CR^1R^2-C(O)-$;
- $R^a$ and $R^b$ are identical or different and are each independently selected from the group consisting of hydrogen, deuterium, halogen and alkyl;
- $R^1$ is haloalkyl or $C_{3-6}$ cycloalkyl;
- $R^2$ is selected from the group consisting of hydrogen, haloalkyl and $C_{3-6}$ cycloalkyl;
- or, $R^1$ and $R^2$, together with carbon atoms connected thereto, form $C_{3-6}$ cycloalkyl;
- m is 0 or 1.

In some embodiments, in the ligand-drug conjugate of general formula (Pc-L-Y-D) or the pharmaceutically acceptable salt thereof according to any one of the aforementioned embodiments, Y is selected from the group consisting of:

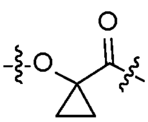, 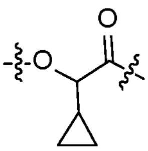, 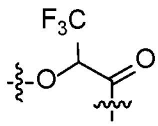, 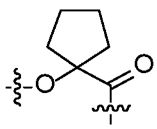, 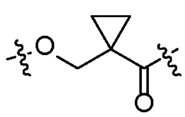, 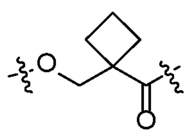,

and

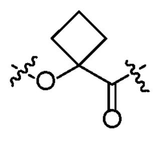;

wherein an O-terminus of Y is connected to the linker unit L.

In some embodiments, in the ligand-drug conjugate of general formula (Pc-L-Y-D) or the pharmaceutically acceptable salt thereof according to any one of the aforementioned embodiments, the linker unit -L- is $-L^1-L^2-L^3-L^4-$, wherein

$L^1$ is selected from the group consisting of -(succinimidyl-3-yl-*N*)-W-C(O)-, $-CH_2$-C(O)-$NR^3$-W-C(O)- and -C(O)-W-C(O)-, wherein W is selected from the group consisting of $C_{1-8}$ alkyl, $C_{1-8}$ alkyl-cycloalkyl and linear heteroalkyl of 1 to 8 atoms, and the heteroalkyl comprises 1 to 3 heteroatoms selected from the group consisting of N, O and S, wherein the $C_{1-8}$ alkyl, cycloalkyl and linear heteroalkyl are each independently optionally further substituted with one or more substituents selected from the group consisting of halogen, hydroxy, cyano, amino, alkyl, chloroalkyl, deuterated alkyl, alkoxy and cycloalkyl;

$L^2$ is selected from the group consisting of $-NR^4(CH_2CH_2O)_pCH_2CH_2C(O)-$, - $NR^4(CH_2CH_2O)_pCH_2C(O)-$, $-S(CH_2)_pC(O)-$ and a chemical bond, wherein p is an integer from 1 to 20;

$L^3$ is a peptide residue consisting of 2 to 7 amino acid residues, wherein the amino acid residues are selected from the group consisting of amino acid residues formed from amino acids from phenylalanine, glycine, valine, lysine, citrulline, serine, glutamic acid and aspartic acid, and are optionally further substituted with one or more substituents selected from the group consisting of halogen, hydroxy, cyano, amino, alkyl, chloroalkyl, deuterated alkyl, alkoxy and cycloalkyl;

$L^4$ is selected from the group consisting of $-NR^5(CR^6R^7)_t-$, $-C(O)NR^5$, $-C(O)NR^5(CH_2)_t-$ and a chemical bond, wherein t is an integer from 1 to 6;

$R^3$, $R^4$ and $R^5$ are identical or different and are each independently selected from the group consisting of hydrogen, alkyl, haloalkyl, deuterated alkyl and hydroxyalkyl;

$R^6$ and $R^7$ are identical or different and are each independently selected from the group consisting of hydrogen, halogen, alkyl, haloalkyl, deuterated alkyl and hydroxyalkyl.

In some embodiments, in the ligand-drug conjugate of general formula (Pc-L-Y-D) or the pharmaceutically acceptable salt thereof according to any one of the aforementioned embodiments, the linker unit -L- is $-L^1-L^2-L^3-L^4-$, wherein

$L^1$ is selected from the group consisting of -(succinimidyl-3-yl-N)-W-C(O)-, $-CH_2$-C(O)-$NR^3$-W-C(O)- and -C(O)-W-C(O)-, wherein W is selected from the group consisting of $C_{1-8}$ alkyl, $C_{1-8}$ alkyl-cycloalkyl and linear heteroalkyl of 1 to 8 chain atoms, and the heteroalkyl comprises 1 to 3 heteroatoms selected from the group consisting of N, O and S, wherein the $C_{1-8}$ alkyl, cycloalkyl and linear heteroalkyl are each independently optionally further substituted with one or more substituents selected from the group consisting of halogen, hydroxy, cyano, amino, alkyl, chloroalkyl, deuterated alkyl, alkoxy and cycloalkyl;

$L^2$ is selected from the group consisting of $-NR^4(CH_2CH_2O)_pCH_2CH_2C(O)-$, - $NR^4(CH_2CH_2O)_pCH_2C(O)-$, $-S(CH_2)_pC(O)-$ and a chemical bond, wherein p is an integer from 1 to 20;

$L^3$ is a peptide residue consisting of 2 to 7 amino acid residues, wherein the amino acid residues are selected from the group consisting of amino acid residues formed from amino acids from phenylalanine (F), glycine (G), valine (V), lysine (K), citrulline, serine (S), glutamic acid (Q) and aspartic acid (D), and are optionally further substituted with one or more substituents selected from the group consisting of halogen, hydroxy, cyano, amino, alkyl, chloroalkyl, deuterated alkyl, alkoxy and cycloalkyl;

$L^4$ is selected from the group consisting of $-NR^5(CR^6R^7)_t-$, $-C(O)NR^5-$, $-C(O)NR^5(CH_2)_t-$ and a chemical bond, wherein t is an integer from 1 to 6, and non-limiting examples of t are 1, 2, 3, 4, 5 and 6;

$R^3$, $R^4$ and $R^5$ are identical or different and are each independently selected from the group consisting of hydrogen, alkyl, haloalkyl, deuterated alkyl and hydroxyalkyl;

$R^6$ and $R^7$ are identical or different and are each independently selected from the group consisting of hydrogen, halogen, alkyl, haloalkyl, deuterated alkyl and hydroxyalkyl.

In some embodiments, in the ligand-drug conjugate of general formula (Pc-L-Y-D) or the pharmaceutically acceptable salt thereof according to any one of the aforementioned embodiments, the linker unit -L- is $-L^1-L^2-L^3-L^4-$, wherein

$L^1$ is

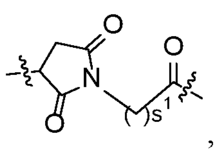
,

$s^1$ is an integer from 2 to 8, and non-limiting examples of $s^1$ are 2, 3, 4, 5, 6, 7 and 8;

$L^2$ is a chemical bond;

$L^3$ is a tetrapeptide residue, preferably a tetrapeptide residue of GGFG;

$L^4$ is $-NR^5(CR^6R^7)_t-$, wherein $R^5$, $R^6$ and $R^7$ are identical or different and are each independently hydrogen or alkyl, and t is 1 or 2;

wherein the $L^1$ terminus is connected to Pc, and the $L^4$ terminus is connected to Y.

In some embodiments, in the ligand-drug conjugate of general formula (Pc-L-Y-D) or the pharmaceutically acceptable salt thereof according to any one of the aforementioned embodiments, -L- is:

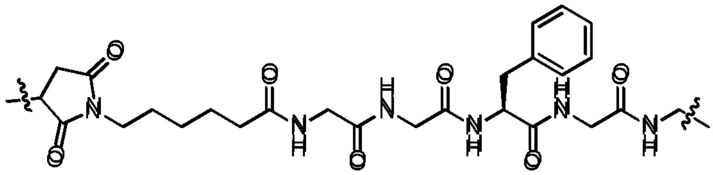

.

In some embodiments, in the ligand-drug conjugate of general formula (Pc-L-Y-D) or the pharmaceutically acceptable salt thereof according to any one of the aforementioned embodiments, -L-Y- is selected from the group consisting of:

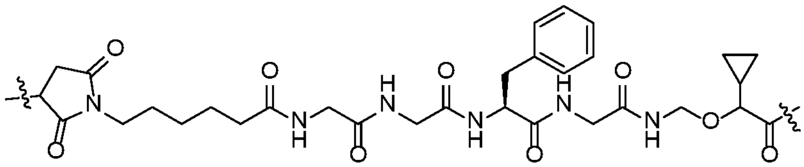

,

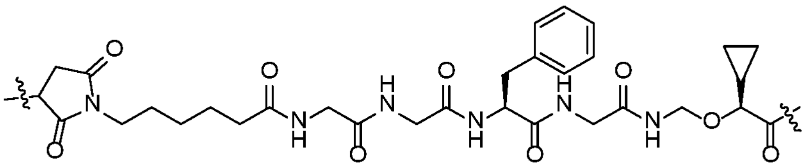

and

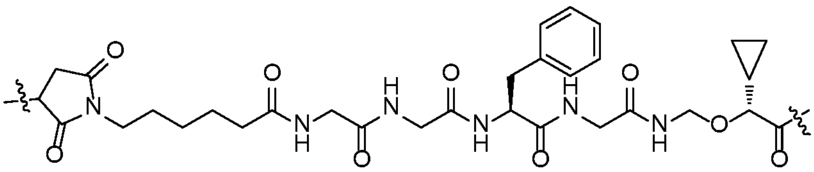

.

In some embodiments, the ligand-drug conjugate of general formula (Pc-L-Y-D) or the pharmaceutically acceptable salt thereof according to any one of the aforementioned embodiments is a ligand-drug conjugate of general formula ($Pc-L_a-Y-D$) or a pharmaceutically acceptable salt thereof,

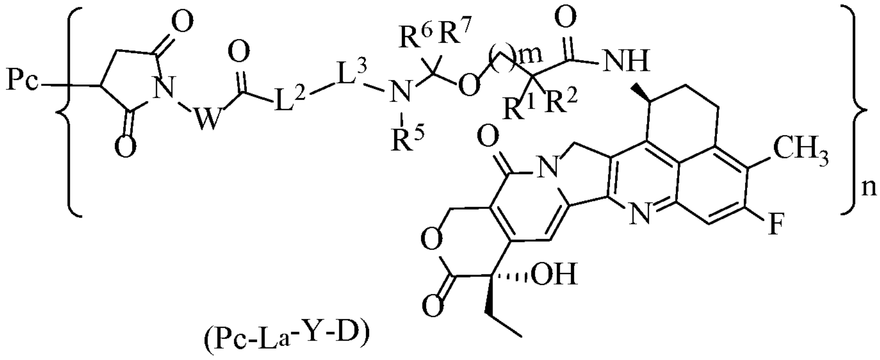

(Pc-La-Y-D)

where:

W, $L^2$, $L^3$, $R^5$, $R^6$ and $R^7$ are as defined in the aforementioned linker unit -L-; Pc, n, $R^1$, $R^2$ and m are as defined in general formula (Pc-L-Y-D).

In some embodiments, the ligand-drug conjugate of general formula (Pc-L-Y-D) or the pharmaceutically acceptable salt thereof according to any one of the aforementioned embodiments is a ligand-drug conjugate of general formula (Pc-$L_b$-T-D) or a pharmaceutically acceptable salt thereof,

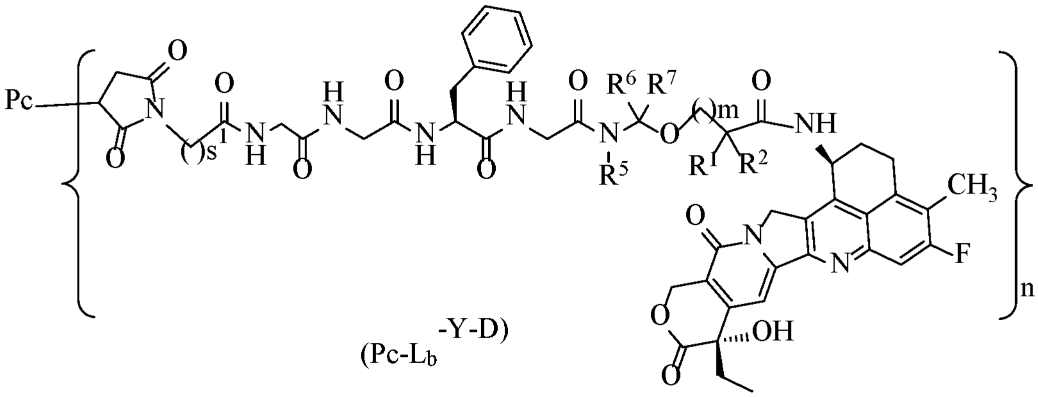

(Pc-$L_b$-Y-D)

wherein:

$s^1$ is an integer from 2 to 8;

Pc, $R^1$, $R^2$, $R^5$, $R^6$, $R^7$, m and n are as defined in general formula (Pc-$L_a$-Y-D).

In some embodiments, in the ligand-drug conjugate of general formula (Pc-L-Y-D) or the pharmaceutically acceptable salt thereof according to any one of the aforementioned embodiments, the ligand-drug conjugate is selected from the group consisting of:

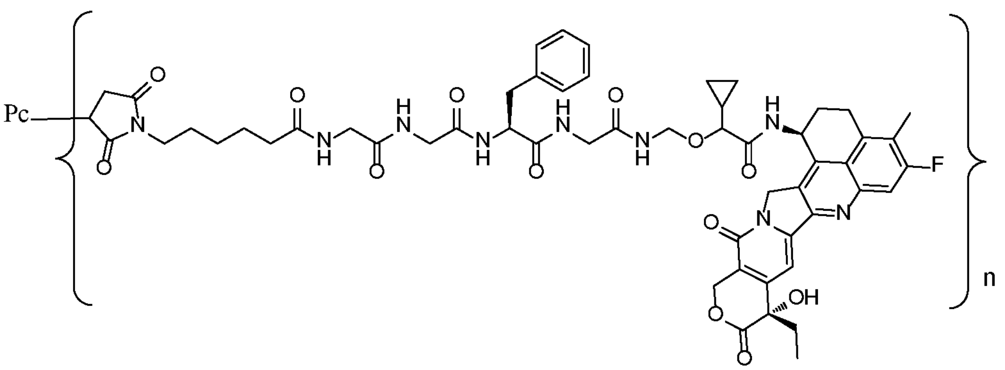

,

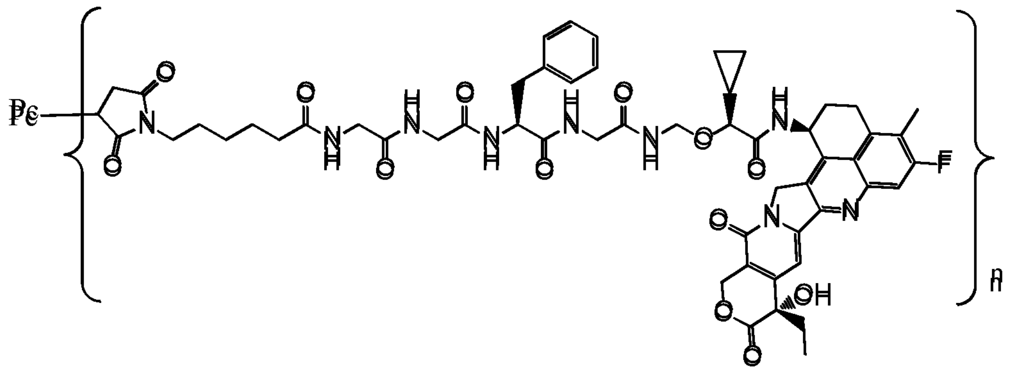

and

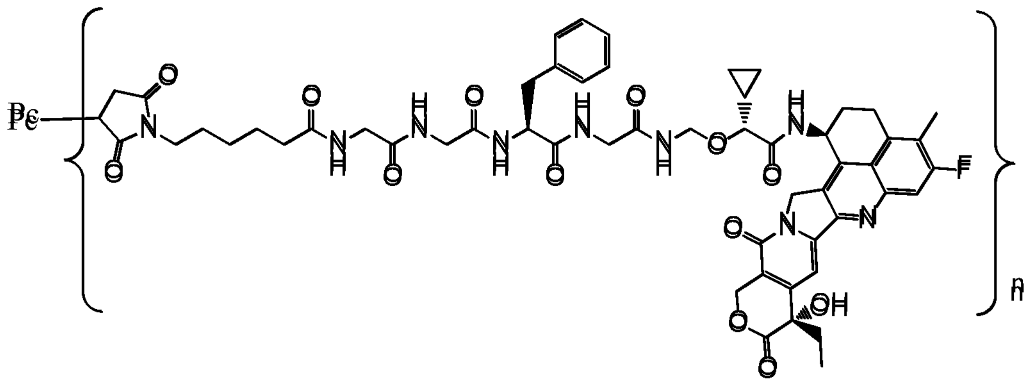

,

wherein Pc and n are as defined in general formula (Pc-L-Y-D).

In some embodiments, in the ligand-drug conjugate of general formula (Pc-L-Y-D) or the pharmaceutically acceptable salt thereof according to any one of the aforementioned embodiments, the ligand-drug conjugate is selected from the group consisting of:

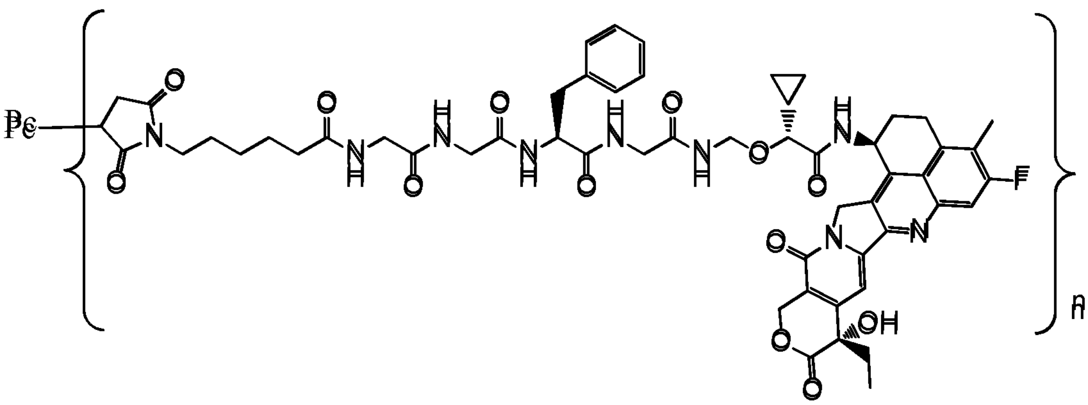

wherein:

n is a decimal or an integer from 4 to 8;
Pc is an anti-TROP-2 antibody comprising a heavy chain set forth in SEQ ID NO: 13 and a light chain set forth in SEQ ID NO: 14.

In some embodiments, in the ligand-drug conjugate of general formula (Pc-L-Y-D) or the pharmaceutically acceptable salt thereof as described above, the ligand-drug conjugate is:

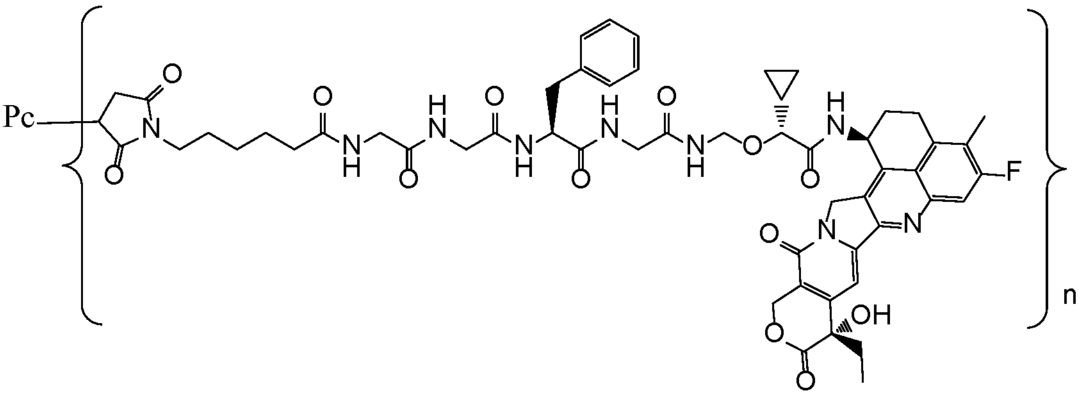

wherein:

n is a decimal or an integer from 1 to 8, preferably from 2 to 4 or 4 to 8, and more preferably from 4 to 6;
Pc is an anti-TROP-2 antibody comprising a heavy chain set forth in SEQ ID NO: 13 and a light chain set forth in SEQ ID NO: 14;
or comprising a heavy chain set forth in SEQ ID NO: 15 and a light chain set forth in SEQ ID NO: 16;
or comprising a heavy chain set forth in SEQ ID NO: 17 and a light chain set forth in SEQ ID NO: 18.

In some embodiments, in the ligand-drug conjugate of general formula (Pc-L-Y-D) or the pharmaceutically acceptable salt thereof as described above, the ligand-drug conjugate is selected from the group consisting of:

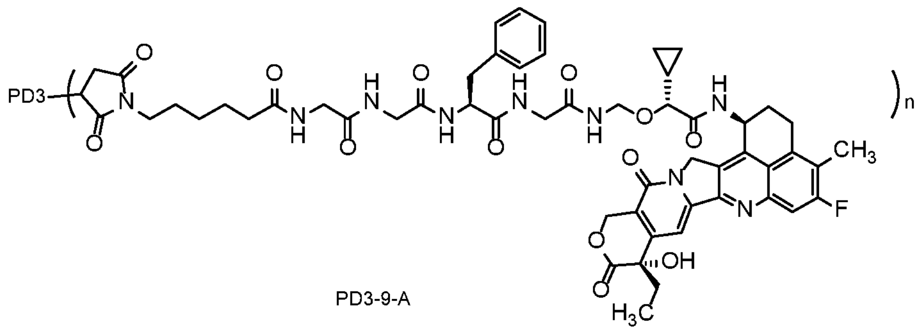

PD3-9-A

,

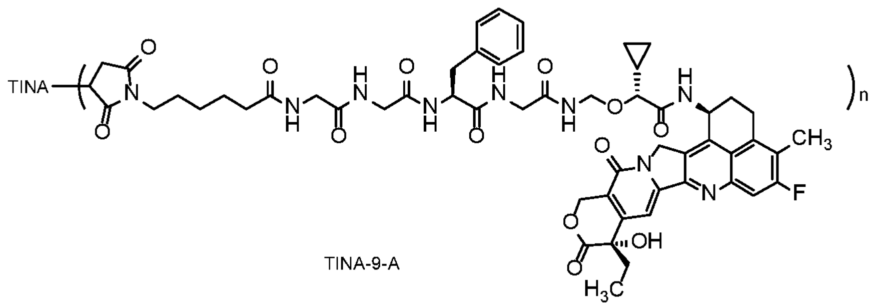

TINA-9-A

and

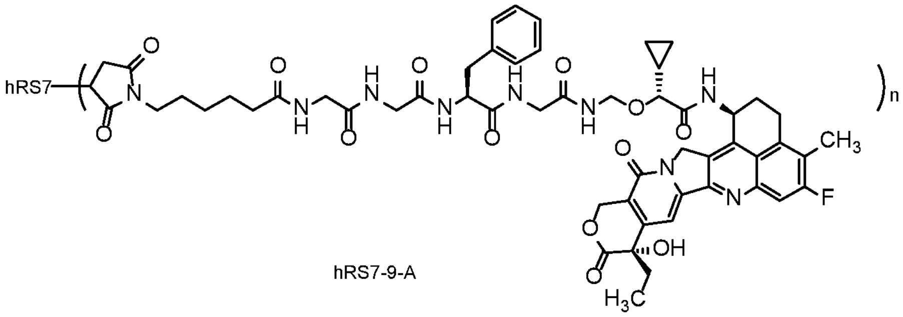

hRS7-9-A

,

wherein:

n is a decimal or an integer from 1 to 8, preferably from 2 to 4 or 4 to 8, and more preferably from 4 to 6;
PD3 is an anti-TROP-2 antibody comprising a heavy chain set forth in SEQ ID NO: 13 and a light chain set forth in SEQ ID NO: 14;
hRS7 is an anti-TROP-2 antibody comprising a heavy chain set forth in SEQ ID NO: 15 and a light chain set forth in SEQ ID NO: 16;
TINA is an anti-TROP-2 antibody comprising a heavy chain set forth in SEQ ID NO: 17 and a light chain set forth in SEQ ID NO: 18.

The present disclosure also provides an anti-TROP-2 antibody or an antigen-binding fragment thereof, comprising a heavy chain variable region and a light chain variable region of the antibody, wherein the heavy chain variable region comprises an HCDR1, an HCDR2 and an HCDR3 having sequences identical to those of an HCDR1, an HCDR2 and an HCDR3 of a heavy chain variable region set forth in SEQ ID NO: 3, and the light chain variable region comprises an LCDR1, an LCDR2 and an LCDR3 having sequences identical to those of an LCDR1, an LCDR2 and an LCDR3 of a light chain variable region set forth in SEQ ID NO: 4.

In some embodiments, in the anti-TROP-2 antibody or the antigen-binding fragment thereof as described above comprising a heavy chain variable region and a light chain variable region, the heavy chain variable region comprises an HCDR1, an HCDR2 and an HCDR3 set forth in SEQ ID NO: 5, SEQ ID NO: 6 and SEQ ID NO: 7, respectively, and the light chain variable region comprises an LCDR1, an LCDR2 and an LCDR3 set forth in SEQ ID NO: 8, SEQ ID NO: 9 and SEQ ID NO: 10, respectively.

In some embodiments, in the anti-TROP-2 antibody or the antigen-binding fragment thereof according to any one of the aforementioned embodiments, the anti-TROP-2 antibody is a murine antibody, a chimeric antibody, a humanized antibody or a human antibody.

In some embodiments, in the anti-TROP-2 antibody or the antigen-binding fragment thereof according to any one of the aforementioned embodiments comprising a heavy chain variable region and a light chain variable region, the heavy chain variable region has an amino acid sequence set forth in SEQ ID NO: 3 or having at least 90%-100% identity thereto including, but not limited to at least 91%, at least 92%, at least 93%, at least 94%, at least 95%, at least 96%, at least 97%, at least 98%, at least 99% or at least 100%, and the light chain variable region has an amino acid sequence set forth in SEQ ID NO: 4 or having at least 90%-100% identity thereto including, but not limited to least 91%, at least 92%, at least 93%, at least 94%, at least 95%, at least 96%, at least 97%, at least 98%, at least 99% or at least 100%.

In some embodiments, in the anti-TROP-2 antibody or the antigen-binding fragment thereof according to any one of the aforementioned embodiments, the anti-TROP-2 antibody or the antigen-binding fragment thereof comprises a heavy chain constant region and a light chain constant region of the antibody; preferably, the heavy chain constant region is selected from the group consisting of human IgG1, IgG2, IgG3 and IgG4 constant regions and conventional variants thereof, and the light chain constant region is selected from the group consisting of human antibody κ and λ chain constant regions and conventional variants thereof; and more preferably, the antibody comprises a heavy chain constant region set forth in SEQ ID NO: 11 and a light chain constant region set forth in SEQ ID NO: 12.

In some embodiments, in the anti-TROP-2 antibody or the antigen-binding fragment thereof according to any one of the aforementioned embodiments, the anti-TROP-2 antibody comprises a heavy chain set forth in SEQ ID NO: 13 and a light chain set forth in SEQ ID NO: 14.

In another aspect, the present disclosure provides a nucleic acid molecule encoding the anti-TROP-2 antibody as described above. In another aspect, the present disclosure provides a nucleic acid molecule encoding the anti-TROP-2 antibody or the antigen-binding fragment thereof as described above.

In another aspect, the present disclosure provides a host cell comprising the nucleic acid molecule as described above.

The present disclosure further provides a method for preparing a ligand-drug conjugate of general formula (Pc-$L_a$-Y-D) or a pharmaceutically acceptable salt thereof comprising the following steps:

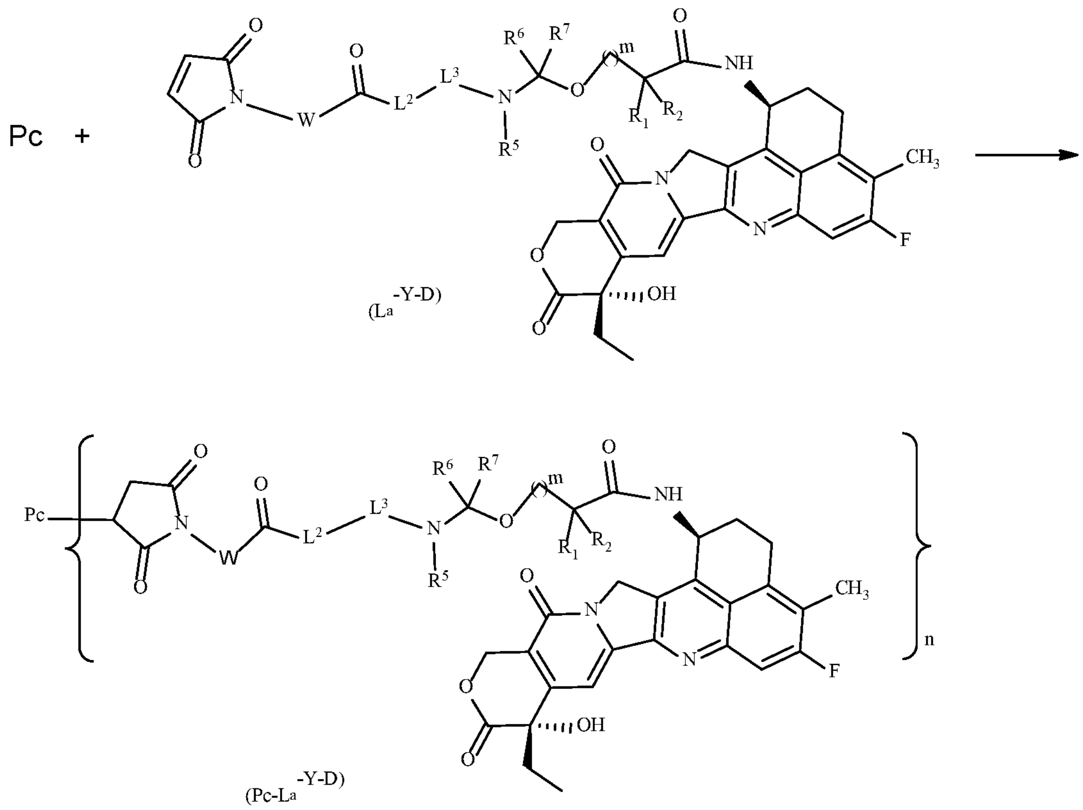

($L_a$-Y-D)

(Pc-$L_a$-Y-D)

,

subjecting reduced Pc' and a compound of general formula ($L_a$-Y-D) to a coupling reaction to give a compound of general formula (Pc-$L_a$-Y-D);
wherein:

Pc is an anti-TROP-2 antibody or an antigen-binding fragment thereof;
n, m, W, $L^2$, $L^3$, $R^1$, $R^2$, $R^5$, $R^6$ and $R^7$ are as defined in the aforementioned general formula (Pc-$L_a$-Y-D).

The present disclosure further provides a method for preparing a ligand drug conjugate of general formula (Pc-L'-D), wherein the method comprises the following step:

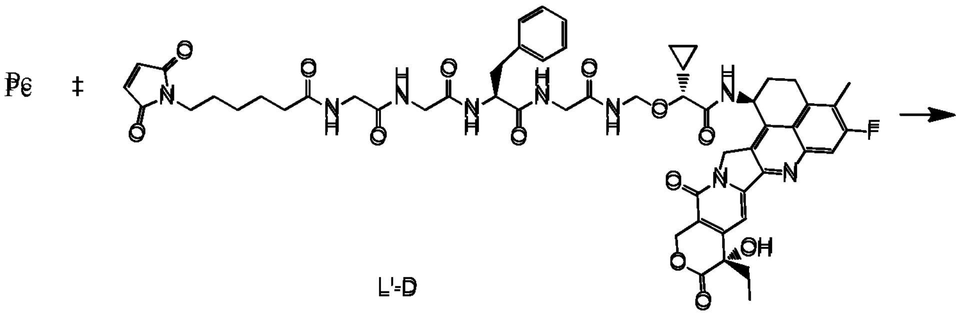

L'-D

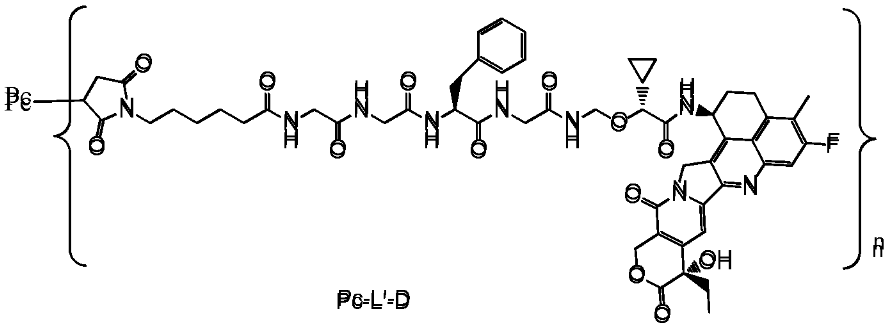

Pc-L'-D

,

subjecting reduced Pc and a compound of general formula (L'-D) to a coupling reaction to give a compound of general formula (Pc-L'-D); wherein:

Pc is the anti-TROP-2 antibody or the antigen-binding fragment thereof as described above;
n is as defined in general formula (Pc-L-Y-D).

In another aspect, the present disclosure provides the ligand-drug conjugate or the pharmaceutically acceptable salt thereof according to any one of the aforementioned embodiments, or the anti-TROP-2 antibody or the antigen-binding fragment thereof according to any one of the aforementioned embodiments, and one or more pharmaceutically acceptable excipients, diluents or carriers. In some embodiments, the unit dose of the pharmaceutical composition comprises 0.1-3000 mg or 1-1000 mg of the anti-TROP-2 antibody as described above or the antibody-drug conjugate as described above.

In another aspect, the present disclosure provides use of the ligand-drug conjugate or the pharmaceutically acceptable salt thereof according to any one of the aforementioned embodiments, or the anti-TROP-2 antibody or the antigen-binding fragment thereof according to any one of the aforementioned embodiments, or the pharmaceutical composition comprising the same as a medicament.

In another aspect, the present disclosure provides the ligand-drug conjugate or the pharmaceutically acceptable salt thereof according to any one of the aforementioned embodiments, the anti-TROP-2 antibody or the antigen-binding fragment thereof according to any one of the aforementioned embodiments, or the pharmaceutical composition comprising the same in preparing a medicament for the treatment of a TROP-2-mediated disease or condition or a tumor, wherein the TROP-2-mediated disease or condition is a cancer with high TROP-2 expression, moderate TROP-2 expression or low TROP-2 expression.

In another aspect, the present disclosure provides use of the ligand-drug conjugate or the pharmaceutically acceptable salt thereof according to any one of the aforementioned embodiments, the anti-TROP-2 antibody or the antigen-binding fragment thereof according to any one of the aforementioned embodiments, or the pharmaceutical composition comprising the same in preparing a medicament for the treatment and/or prevention of tumors and cancers, wherein the tumors and cancers are preferably head and neck squamous cell carcinoma, head and neck cancer, brain cancer, neuroglioma, glioblastoma multiforme, neuroblastoma, central nervous system carcinoma, neuroendocrine tumor, throat cancer, pharyngeal squamous cell carcinoma, oral squamous cell carcinoma, nasopharyngeal cancer, esophageal cancer, thyroid cancer, malignant pleural mesothelioma, lung cancer, breast cancer, liver cancer, hepatobiliary cancer, pancreatic cancer, stomach cancer, gastrointestinal cancer, intestinal cancer, colon cancer, colorectal cancer, kidney cancer, clear cell renal cell carcinoma, ovarian cancer, endometrial cancer, cervical cancer, bladder cancer, prostate cancer, testicular cancer, skin cancer, melanoma, leukemia, lymphoma, bone cancer, chondrosarcoma, myeloma, multiple myeloma, myelodysplastic syndrome, Krukenberg tumor, myeloproliferative tumor, squamous cell carcinoma, Ewing's sarcoma, urothelial carcinoma or Merkel cell carcinoma; more preferably, the lymphoma is selected from the group consisting of Hodgkin's lymphoma, non-Hodgkin's lymphoma, diffuse large B-cell lymphoma, follicular lymphoma, primary mediastinal large B-cell lymphoma, mantle cell lymphoma, small lymphocytic lymphoma, large B-cell lymphoma rich in T-cells/histiocytes and lymphoplasmacytic lymphoma, the lung cancer is selected from the group consisting of non-small cell lung cancer and small cell lung cancer, and the leukemia is selected from the group consisting of chronic myeloid leukemia, acute myeloid leukemia, lymphocytic leukemia, lymphoblastic leukemia, acute lymphoblastic leukemia, chronic lymphocytic leukemia and myeloid cell leukemia.

In another aspect, the present disclosure further relates to a method for treating and/or preventing a tumor, the method comprising administering to a patient in need thereof a therapeutically effective dose of the ligand-drug conjugate or the pharmaceutically acceptable salt thereof according to any one of the aforementioned embodiments, the anti-TROP-2 antibody or the antigen-binding fragment thereof according to any one of the aforementioned embodiments, or the pharmaceutical composition comprising the same; wherein the tumor is a cancer with high TROP-2 expression, moderate TROP-2 expression or low TROP-2 expression.

In another aspect, the present disclosure further relates to a method for treating or preventing tumors or cancers, the method comprising administering to a subject in need thereof a therapeutically effective dose of the ligand drug conjugate or the pharmaceutically acceptable salt thereof according to any one of the aforementioned embodiments, or the anti-TROP-2 antibody or the antigen-binding fragment thereof according to any one of the aforementioned embodiments, or the pharmaceutical composition comprising the same, wherein the tumors and cancers are preferably head and neck squamous cell carcinoma, head and neck cancer, brain cancer, neuroglioma, glioblastoma multiforme, neuroblastoma, central nervous system carcinoma, neuroendocrine tumor, throat cancer, pharyngeal squamous cell carcinoma, oral squamous cell carcinoma, nasopharyngeal cancer, esophageal cancer, thyroid cancer, malignant pleural mesothelioma, lung cancer, breast cancer, liver cancer, hepatobiliary cancer, pancreatic cancer, stomach cancer, gastrointestinal cancer, intestinal cancer, colon cancer, colorectal cancer, kidney cancer, clear cell renal cell carcinoma, ovarian cancer, endometrial cancer, cervical cancer, bladder cancer, prostate cancer, testicular cancer, skin cancer, melanoma, leukemia, lymphoma, bone cancer, chondrosarcoma, myeloma, multiple myeloma, myelodysplastic syndrome, Krukenberg tumor, myeloproliferative tumor, squamous cell carcinoma, Ewing's sarcoma, urothelial carcinoma or Merkel cell carcinoma; more preferably, the lymphoma is selected from the group consisting of Hodgkin's lymphoma, non-Hodgkin's lymphoma, diffuse large B-cell lymphoma, follicular lymphoma, primary mediastinal large B-cell lymphoma, mantle cell lymphoma, small lymphocytic lymphoma, large B-cell lymphoma rich in T-cells/histiocytes and lymphoplasmacytic lymphoma, the lung cancer is selected from the group consisting of non-small cell lung cancer and small cell lung cancer, and the leukemia is selected from the group consisting of chronic myeloid leukemia, acute myeloid leukemia, lymphocytic leukemia, lymphoblastic leukemia, acute lymphoblastic leukemia, chronic lymphocytic leukemia and myeloid cell leukemia.

In another aspect, the present disclosure further provides the aforementioned anti-TROP-2 antibody or the antibody-drug conjugate thereof for use as a medicament, preferably as a medicament for the treatment of cancers or tumors, and more preferably as a medicament for the treatment of a TROP-2-mediated cancer.

The active compound (e.g., the ligand-drug conjugate or the pharmaceutically acceptable salt thereof according to the present disclosure) may be formulated in a form suitable for administration by any suitable route, preferably in a form of a unit dose, or in a form of a unit dose that can be self-administered by a subject. The unit dose of the active compound or composition disclosed herein may be in a tablet, a capsule, a cachet, a vial, a powder, a granule, a lozenge, a suppository, a powder for reconstitution or a liquid formulation. The administration dose of the active compound or composition used in the treatment method of the present disclosure will generally vary with the severity of the disease, the weight of the subject, and the efficacy of the active compound. As a general guide, a suitable unit dose may be 0.1 mg to 1000 mg.

The pharmaceutical composition of the present disclosure may comprise, in addition to the active compound, one or more excipients selected from the group consisting of: a filler, a diluent, a binder, a wetting agent, a disintegrating agent, an excipient and the like. Depending on the method of administration, the composition may comprise 0.1 to 99 wt.% of the active compound.

The TROP-2 antibody and the antibody-drug conjugate provided by the present disclosure have good affinity for cell surface antigens, good endocytosis efficiency and high tumor inhibition efficiency as well as wider drug application windows, and are suitable for clinical drug application.

## BRIEF DESCRIPTION OF THE DRAWINGS

FIG. 1 shows results of experiments on the binding of anti-TROP-2 antibodies to cells expressing TROP-2.

FIG. 2 shows results of bystander killing activity of ADCs on BxPC3 cells and MiaPaCa2 mixed cells.

FIG. 3 shows inhibitory activity of different ADCs on FaDu xenograft tumors in mice.

FIG. 4 shows inhibitory activity of different doses of ADCs on SKOV3 xenograft tumors in mice.

FIG. 5 shows inhibitory activity of different doses of ADCs on Colo205 xenograft tumors in mice.

**DETAILED DESCRIPTION**

1. Terminology

Unless defined otherwise, all technical and scientific terms used herein have the same meaning as commonly understood by those of ordinary skill in the art to which the present disclosure belongs. Although any methods and materials similar or equivalent to those described herein can also be used to implement or test the present disclosure, preferred methods and materials are described herein. In describing and claiming the present disclosure, the following terms are used in accordance with the definitions below. When a trade name is used in the present disclosure, it is intended to include the formulation of the commercial product under the trade name, and the drug and active drug component of the commercial product under the trade name.

Unless otherwise stated, the terms used in the specification and claims have the following meanings.

The term "drug" refers to a cytotoxic drug that may have a chemical molecule within the tumor cell that is strong enough to disrupt its normal growth. The cytotoxic drug can kill cells in principle at a sufficiently high concentration; however, due to lack of specificity, the cytotoxic drug can cause apoptosis of normal cells while killing tumor cells, resulting in serious side effects. The term "cytotoxic drug" includes toxins, such as small molecule toxins or enzymatically active toxins of bacterial, fungal, plant or animal origin, radioisotopes (e.g., $At^{211}$, $I^{131}$, $I^{125}$, $Y^{90}$, $Re^{186}$, $Re^{188}$, $Sm^{153}$, $Bi^{212}$, $P^{32}$ and radioactive isotopes of Lu), chemotherapeutic drugs, antibiotics and nucleolytic enzymes.

The term "linker unit", "linker", "linking unit" or "linking fragment" refers to a chemical structural fragment or bond, which is linked to a ligand at one end and linked to a drug at the other end, and also may be linked to other linkers and then linked to the ligand or the drug.

The linker may comprise one or more linker components. Exemplary linker components include 6-maleimidocaproyl ("MC"), maleimidopropionyl ("MP"), valine-citrulline ("val-cit" or "vc"), alanine-phenylalanine ("ala-phe"), *p*-aminobenzyloxycarbonyl ("PAB"), *N*-succinimidyl 4-(2-pyridylthio)pentanoate ("SPP"), *N*-succinimidyl 4-(*N*-maleimidomethyl)cyclohexane-1 carboxylate ("SMCC", also referred to herein as "MCC"), and *N*-succinimidyl(4-iodo-acetyl)aminobenzoate ("SIAB"). The linker may be selected from the following elements or a combination thereof: an extender, a spacer and an amino acid unit. The linker may be synthesized using methods known in the art, such as those described in US2005-0238649A1. The linker may be a "cleavable linker" favoring the release of drugs in cells. For example, acid-labile linkers (e.g., hydrazones), protease-sensitive (e.g., peptidase-sensitive) linkers, photolabile linkers, dimethyl linkers or disulfide-containing linkers can be used (Chari et al., Cancer Research 52: 127-131(1992); U.S. Patent No. 5,208,020).

Linker elements include, but are not limited to:

MC = 6-maleimidocaproyl, with a structure:

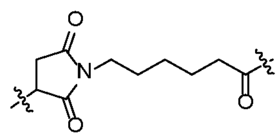
,

Val-Cit or "vc" = valine-citrulline (an exemplary dipeptide in a protease cleavable linker), citrulline = 2-amino-5-ureidopentanoic acid,
PAB = *p*-aminobenzyloxycarbonyl (an example of "self-immolative" linker elements), Me-Val-Cit = *N*-methyl-valine-citrulline (where the linker peptide bond has been modified to prevent it from being cleaved by cathepsin B),
MC(PEG)6-OH = maleimidocaproyl-polyethylene glycol (attachable to antibody cysteine),
SPP = *N*-succinimidyl 4-(2-pyridylthio)valerate,
SPDP = *N*-succinimidyl 3-(2-pyridyldithio)propionate,
SMCC = succinimidyl-4-(*N*-maleimidomethyl)cyclohexane-1-carboxylate,
IT = iminothiolane.

The term "ligand-drug conjugate" means that a ligand is linked to a biologically active drug by a linking unit, and the "ligand-drug conjugate" is preferably an "antibody-drug conjugate". In the present disclosure, "antibody-drug conjugate" (ADC) means that a monoclonal antibody or an antibody fragment is linked to a biologically active toxic drug by a linking unit. The antibody may be conjugated to the drug directly or via a linker. n is mean number of drug modules conjugated to each antibody, may be an integral or a decimal, and may range, for example, from about 0 to about 20 drug modules; in certain embodiments, from 1 to about 10 drug modules; and in certain embodiments, from 1 to about

8 drug modules, such as 2, 3, 4, 5, 6, 7 or 8 drug modules. In the composition of the mixture of the antibody-drug conjugate of the present disclosure, the mean drug loading of each antibody is about 1 to about 10, including but not limited to about 3 to about 7, about 3 to about 6, about 3 to about 5, about 1 to about 9, about 7 or about 4. The three-letter and single-letter codes for amino acids used in the present disclosure are as described in J. biol. chem, 243, p3558 (1968).

The term "antibody" refers to an immunoglobulin, which is of a tetrapeptide chain structure formed by connection between two heavy chains and two light chains by interchain disulfide bonds. According to differences in the amino acid composition and the order of arrangement of the heavy chain constant regions of immunoglobulins, immunoglobulins can be divided into five classes, otherwise called isotypes of immunoglobulins, namely IgM, IgD, IgG, IgA and IgE, with their corresponding heavy chains being $\mu$ chain, $\delta$ chain, $\gamma$ chain, $\alpha$ chain and $\varepsilon$ chain, respectively. Ig of the same class can be divided into different subclasses according to differences in the amino acid composition of the hinge regions and the number and positions of disulfide bonds of the heavy chains; for example, IgG may be divided into IgG1, IgG2, IgG3 and IgG4. Light chains are classified into $\kappa$ or $\lambda$ chain by the differences in the constant regions. Each of the five classes of Ig may have a $\kappa$ chain or $\lambda$ chain.

In the heavy and light chains of full-length antibodies, the sequences of about 110 amino acids near the N-terminus vary considerably and thus are referred to as variable regions (Fv regions); the remaining amino acid sequences near the C-terminus are relatively stable and thus are referred to as constant regions. The variable regions comprise 3 hypervariable regions (HVRs) and 4 framework regions (FRs) with relatively conservative sequences. The 3 hypervariable regions determine the specificity of the antibody and thus are also known as complementarity determining regions (CDRs). Each light chain variable region (LCVR) or heavy chain variable region (HCVR) consists of 3 CDRs and 4 FRs arranged from the amino-terminus to the carboxy-terminus in the following order: FR1, CDR1, FR2, CDR2, FR3, CDR3 and FR4. The 3 CDRs of the light chain refer to LCDR1, LCDR2 and LCDR3, and the 3 CDRs of the heavy chain refer to HCDR1, HCDR2 and HCDR3.

The terms "fully humanized antibody", "fully human antibody", "human antibody" or "completely human antibody", also known as "fully humanized monoclonal antibody", has both humanized variable region and constant region so as to eliminate immunogenicity and toxic side effects. Major relevant technologies for the preparation of fully human antibodies include: human hybridoma technology, EBV-transformed B-lymphocyte technology, phage display technology, transgenic mouse antibody preparation technology, single B-cell antibody preparation technology, and the like. The term "antigen-binding fragment" refers to one or more fragments of an antibody that retain the ability to specifically bind to an antigen. A fragment of a full-length antibody can be used to perform the antigen-binding function of the antibody. The binding fragment included in the "antigen-binding fragment" is selected from the group consisting of Fab, Fab', $F(ab')_2$, single-chain antibody (scFv), dimerized V region (diabody), disulfide-stabilized V region (dsFv) and antigen-binding fragments of peptides comprising CDRs; examples include (i) Fab fragments, monovalent fragments consisting of VL, VH, CL and CH1 domains; (ii) $F(ab')_2$ fragments, bivalent fragments comprising two Fab fragments connected by disulfide bridges in the hinge regions; (iii) Fd fragments consisting of VH and CH1 domains; (iv) Fv fragments consisting of VH and VL domains of a single arm of an antibody; (v) single domains or dAb fragments (Ward et al., (1989) Nature 341: 544-546) consisting of VH domains; and (vi) isolated complementarity determining regions (CDRs) or (vii) combinations of two or more isolated CDRs which may optionally be linked by synthetic linkers. Furthermore, although the two domains of the Fv fragment, VL and VH, are encoded by separate genes, they can be linked by a synthetic linker by recombination, so that it is capable of producing a single protein chain in which the VL and VH regions pair to form a monovalent molecule (referred to as single-chain Fv (scFv); see, e.g., Bird et al. (1988) Science 242:423-426; and Huston et al. (1988) Proc. Natl. Acad. Sci USA 85:5879-5883). Such single-chain antibodies are also intended to be included in the term "antigen-binding fragment" of an antibody. Such antibody fragments are obtained using conventional techniques known to those skilled in the art, and screened for utility in the same manner as for intact antibodies. Antigen-binding portions may be produced using recombinant DNA technology or by enzymatic or chemical cleavage of intact immunoglobulins. Antibodies may be of different isotypes, e.g., IgG (e.g., subtype IgG1, IgG2, IgG3 or IgG4), IgA1, IgA2, IgD, IgE or IgM antibody.

In general, Fab is an antibody fragment having a molecular weight of about 50,000 and having antigen-binding activity, among fragments obtained by treating an IgG antibody molecule with a protease papain (e.g., cleaving the amino acid residue at position 224 of H chain), in which a portion on the N-terminal side of H chain is combined with L chain by a disulfide bond.

In general, $F(ab')_2$ is an antibody fragment obtained by digesting a portion below the disulfide bond in the IgG hinge region with the enzyme pepsin. It has a molecular weight of about 100,000, has antigen-binding activity, and comprises two Fab regions linked at the hinge position.

In general, Fab' is an antibody fragment having a molecular weight of about 50,000 and having an antigen-binding activity, obtained by cleaving the disulfide bond in the hinge region of the $F(ab')_2$ described above.

In addition, Fab' may be produced by inserting DNA encoding the Fab' fragment into a prokaryotic or eukaryotic expression vector and introducing the vector into a prokaryote or a eukaryote to express the Fab'.

The term "single-chain antibody", "single-chain Fv" or "scFv" means a molecule comprising an antibody heavy chain variable domain (or VH) and an antibody light chain variable domain (or VL) linked by a linker. Such scFv molecules may have a general structure: $NH_2$-VL-linker-VH-COOH or $NH_2$-VH-linker-VL-COOH. Suitable linkers in the prior art consist of repeated GGGGS amino acid sequences or variants thereof, for example, 1-4 repeated variants (Holliger et al. (1993), Proc. Natl. Acad. Sci. USA 90:6444-6448). Other linkers that can be used in the present disclosure are described in Alfthan et al. (1995), Protein Eng. 8:725-731; Choi et al. (2001), Eur. J. Immunol. 31:94-106; Hu et al. (1996), Cancer Res. 56:3055-3061, Kipriyanov et al. (1999), J. Mol. Biol. 293:41-56; and Roovers et al. (2001), Cancer Immunol.

The term "CDR" refers to one of the 6 hypervariable regions within the variable domain of an antibody which primarily contribute to antigen binding. In general, there are three CDRs (HCDR1, HCDR2 and HCDR3) in each heavy chain variable region and three CDRs (LCDR1, LCDR2 and LCDR3) in each light chain variable region. The amino acid sequence boundaries of the CDRs can be determined using any of a variety of well-known schemes, including "Kabat" numbering scheme (see Kabat et al. (1991), "Sequences of Proteins of Immunological Interest", 5th edition, Public Health Service, National Institutes of Health, Bethesda, MD), "Chothia" numbering scheme (see Al-Lazikani et al. (1997) JMB 273: 927-948) and ImMunoGenTics (IMGT) numbering scheme (Lefranc M.P., Immunologist, 7, 132-136(1999); Lefranc, M.P. et al., Dev. Comp. Immunol., 27, 55-77(2003), etc.). For example, for the classical format, according to the Kabat scheme, the CDR amino acid residues in the heavy chain variable domain (VH) are numbered as 31-35(HCDR1), 50-65(HCDR2) and 95-102(HCDR3); the CDR amino acid residues in the light chain variable domain (VL) are numbered as 24-34(LCDR1), 50-56(LCDR2) and 89-97(LCDR 3). According to the Chothia scheme, the CDR amino acids in VH are numbered as 26-32(HCDR1), 52-56(HCDR2) and 95-102(HCDR3); and amino acid residues in VL are numbered as 26-32(LCDR1), 50-52(LCDR2) and 91-96(LCDR3). According to the CDR definitions by combining both the Kabat scheme and the Chothia scheme, the CDR is composed of amino acid residues 26-35(HCDR1), 50-65(HCDR2) and 95-102(HCDR3) in the human VH and amino acid residues 24-34(LCDR1), 50-56(LCDR2) and 89-97(LCDR3) in the human VL. According to the IMGT scheme, the CDR amino acid residues in VH are roughly numbered as 26-35(CDR1), 51-57(CDR2) and 93-102(CDR3), and the CDR amino acid residues in VL are roughly numbered as 27-32(CDR1), 50-52(CDR2) and 89-97(CDR3). According to the IMGT scheme, the CDRs of the antibody can be determined using the program IMGT/DomainGap Align. The term "framework region" refers to a portion of a variable domain VL or VH, which serves as a framework for the antigen-binding loops (CDRs) of the variable domain. It is essentially a variable domain without CDRs.

The term "epitope" or "antigenic determinant" refers to a site on an antigen to which an immunoglobulin or antibody binds. Epitopes typically comprise at least 3, 4, 5, 6, 7, 8, 9, 10, 11, 12, 13, 14 or 15 contiguous or non-contiguous amino acids in a unique spatial conformation. See, e.g., Epitope Mapping Protocols in Methods in Molecular B iology, volume 66, G.E.Morris, Ed. (1996).

The terms "specific binding", "selective binding", "selectively bind to" and "specifically bind to" refer to the binding of an antibody or a fragment thereof to an epitope on a predetermined antigen. In general, the antibody or the fragment thereof binds with an affinity (KD) of less than about $10^{-7}$ M, e.g., less than about $10^{-8}$ M, $10^{-9}$ M, or $10^{-10}$ M or less.

The term "KD" refers to the dissociation equilibrium constant for an antibody-antigen interaction. In general, the antibody or the antigen-binding fragment of the present disclosure binds to TROP-2 or an epitope thereof with a dissociation equilibrium constant (KD) of less than about $10^{-7}$ M, e.g., less than about $10^{-8}$ M or $10^{-9}$ M; for example, the KD value is determined using FACS method for the affinity of the antibody of the present disclosure for a cell surface antigen.

The term "nucleic acid molecule" refers to a DNA molecule or an RNA molecule. The nucleic acid molecule may be single-stranded or double-stranded, but is preferably double-stranded DNA. A nucleic acid is "operably linked" when it is placed into a functional relationship with another nucleic acid sequence. For example, a promoter or enhancer is operably linked to a coding sequence if it affects the transcription of the coding sequence.

Amino acid sequence "identity" refers to the percentage of amino acid residues shared by a first sequence and a second sequence, wherein in aligning the amino acid sequences, gaps are introduced if necessary to achieve maximum percent sequence identity, and any conservative substitution is not considered as part of sequence identity. For the purpose of determining percent amino acid sequence identity, alignments can be achieved in a variety of ways that are within the skill in the art, for example, using publicly available computer software such as BLAST, BLAST-2, ALIGN, ALIGN-2 or Megalign (DNASTAR) software. Those skilled in the art can determine parameters suitable for measuring alignment, including any algorithms required to achieve maximum alignment of the full length of the aligned sequences.

The term "expression vector" refers to a nucleic acid molecule capable of transporting another nucleic acid to which it has been linked. In one embodiment, the vector is a "plasmid" that refers to a circular double-stranded DNA loop into which additional DNA segments can be ligated. In another embodiment, the vector is a viral vector, wherein additional DNA segments may be ligated into the viral genome. The vectors disclosed herein are capable of autonomously replicating in a host cell into which they are introduced (e.g., bacterial vectors having a bacterial origin of replication and episomal mammalian vectors) or capable of integrating into the genome of a host cell after being introduced into the

host cell and thus replicating with the host genome (e.g., non-episomal mammalian vectors).

Methods of producing and purifying antibodies and antigen-binding fragments are well known in the art, for example, those described in chapters 5-8 and 15 of Antibodies: A Laboratory Manual, Cold Spring Harbor Press. The antibody or the antigen-binding fragment described in the present invention is genetically engineered to contain one or more additional human FRs in the non-human CDRs. Human FR germline sequences can be obtained at the website of ImMunoGeneTics (IMGT) or from the immunoglobulin journal, Lefranc, G., The Immunoglobulin FactsBook, Academic Press, 2001ISBN0124413 51, by comparing the IMGT human antibody variable region germline gene database with the MOE software.

The term "host cell" refers to a cell into which an expression vector has been introduced. Host cells may include microbial (e.g., bacterial), plant or animal cells. Bacteria susceptible to transformation include members of the *Enterobacteriaceae* family, such as strains of *Escherichia coli* or *Salmonella;* members of the *Bacillaceae* family, such as *Bacillus subtilis*; *Pneumococcus*; *Streptococcus* and *Haemophilus influenzae.* Suitable microorganisms include *Saccharomyces cerevisiae* and *Pichia pastoris.* Suitable animal host cell lines include CHO (Chinese hamster ovary cell line) and NS0 cells.

The engineered antibody or the antigen-binding fragment of the present disclosure can be prepared and purified using conventional methods. For example, cDNA sequences encoding the heavy and light chains can be cloned and recombined into an expression vector. Recombinant immunoglobulin expression vectors can be stably transfected into host cells. As a more recommended prior art, mammalian expression systems will result in glycosylation of the antibody, particularly at the N-terminal site of the Fc region. Positive clones are expanded in a medium in a bioreactor to produce antibodies. The culture with the secreted antibody can be purified using conventional techniques, for example, using an A or G Sepharose FF column. Non-specifically bound fractions are washed away. The bound antibody is eluted using pH gradient method, and the antibody fragments are detected using SDS-PAGE and collected. The antibody can be filtered and concentrated using conventional methods. Soluble mixtures and polymers can also be removed using conventional methods, such as molecular sieves and ion exchange. The resulting product needs to be immediately frozen, e.g., at -70 °C, or lyophilized.

The term "peptide" refers to a molecule formed by connecting 2 or more amino acid molecules by peptide bonds, and is a structural and functional fragment of the protein.

The term "alkyl" refers to a saturated aliphatic hydrocarbon group that is a linear or branched group containing 1 to 20 carbon atoms, preferably alkyl containing 1 to 12 (such as 1, 2, 3, 4, 5, 6, 7, 8, 9, 10, 11 or 12) carbon atoms, more preferably alkyl containing 1 to 10 carbon atoms, and most preferably alkyl containing 1 to 6 carbon atoms (containing 1, 2, 3, 4, 5 or 6 carbon atoms). Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, n-decyl, 3,3-diethylhexyl, 2,2-diethylhexyl, and various side-chain isomers thereof, and the like. More preferred is a lower alkyl having 1 to 6 carbon atoms, and non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl may be substituted or unsubstituted. When substituted, the substituent may be substituted at any available connection site, wherein the substituent is preferably one or more of the following groups independently selected from the group consisting of alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio and oxo.

The term "heteroalkyl" refers to alkyl containing one or more heteroatoms selected from the group consisting of N, O and S, wherein the alkyl is as defined above.

The term "alkylene" refers to a saturated linear or branched aliphatic hydrocarbon group having 2 residues derived from the parent alkane by removal of two hydrogen atoms from the same carbon atom or two different carbon atoms. It is a linear or branched group containing 1 to 20 carbon atoms, preferably alkylene containing 1 to 12 (such as 1, 2, 3, 4, 5, 6, 7, 8, 9, 10, 11 or 12) carbon atoms, more preferably alkylene containing 1 to 6 carbon atoms (containing 1, 2, 3, 4, 5 or 6 carbon atoms). Non-limiting examples of alkylene include, but are not limited to, methylene($-CH_2-$), 1,1-ethylidene($-CH(CH_3)-$), 1,2-ethylidene($-CH_2CH_2$)-, 1,1-propylidene($-CH(CH_2CH_3)-$), 1,2-propylidene($-CH_2CH(CH_3)-$), 1,3-propylidene($-CH_2CH_2CH_2-$), 1,4-butylidene($-CH_2CH_2CH_2CH_2-$), 1,5-butylidene($-CH_2CH_2CH_2CH_2CH_2-$) and the like. The alkylene may be substituted or unsubstituted. When substituted, the substituent may be substituted at any available connection site with one or more substituents preferably independently optionally selected from the group

consisting of alkyl, alkenyl, alkynyl, alkoxy, alkylthio, alkylamino, halogen, mercapto, hydroxy, nitro, cyano, cycloalkyl, heterocyclyl, aryl, heteroaryl, cycloalkoxy, heterocycloalkoxy, cycloalkylthio, heterocycloalkylthio and oxo.

The term "alkoxy" refers to -O-(alkyl) and -O-(unsubstituted cycloalkyl), wherein the alkyl or cycloalkyl is as defined above. Non-limiting examples of alkoxy include: methoxy, ethoxy, propoxy, butoxy, cyclopropyloxy, cyclobutoxy, cyclopentyloxy and cyclohexyloxy. The alkoxy may be optionally substituted or unsubstituted, and when it is substituted, the substituent is preferably one or more of the following groups independently selected from the group consisting of: alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio and heterocycloalkylthio.

The term "haloalkyl" refers to an alkyl group in which the hydrogen is substituted with one or more halogens, wherein the alkyl is as defined above.

The term "deuterated alkyl" refers to an alkyl group in which the hydrogen is substituted with one or more deuterium atoms, wherein the alkyl is as defined above.

The term "hydroxyalkyl" refers to an alkyl group in which the hydrogen is substituted with one or more hydroxy groups, wherein the alkyl is as defined above.

The term "hydroxy" refers to -OH group.

The term "halogen" refers to fluorine, chlorine, bromine or iodine.

The term "amino" refers to $-NH_2$.

The term "nitro" refers to $-NO_2$.

The term "cyano" refers to -CN.

The present disclosure also comprises various deuterated forms of the compound of formula (Pc-L-Y-D). Each available hydrogen atom connected to a carbon atom may be independently substituted with a deuterium atom. Those skilled in the art are able to synthesize the deuterated forms of the compound of general formula (Pc-L-Y-D) with reference to the relevant literature. Commercially available deuterated starting materials can be used in preparing the deuterated forms of the compound of general formula (Pc-L-Y-D), or they can be synthesized by using conventional techniques with deuterated reagents including, but not limited to, deuterated borane, tri-deuterated borane in tetrahydrofuran, deuterated lithium aluminum hydride, deuterated iodoethane, deuterated iodomethane, and the like.

The term "optional" or "optionally" means that the event or circumstance subsequently described may, but not necessarily, occur, and that the description includes instances where the event or circumstance occurs or does not occur. For example, "a heterocyclyl group optionally substituted with alkyl" means that alkyl may be, but not necessarily, present, and that the description includes instances where the heterocyclyl group is or is not substituted with alkyl.

The term "substituted" means that one or more, preferably up to 5, more preferably 1, 2 or 3 hydrogen atoms in the group are independently substituted with a substituent. A substituent is only in its possible chemical position, and those skilled in the art will be able to determine (experimentally or theoretically) possible or impossible substitution without undue efforts. For example, it may be unstable when amino or hydroxy having a free hydrogen is bound to a carbon atom having an unsaturated (e.g., olefinic) bond. The term "pharmaceutical composition" refers to a mixture containing one or more of the compounds described herein or a physiologically/pharmaceutically acceptable salt or prodrug thereof, and other chemical components, for example physiologically/pharmaceutically acceptable carriers and excipients. The purpose of the pharmaceutical composition is to promote the administration to an organism, which facilitates the absorption of the active ingredient, thereby exerting biological activity. The term "pharmaceutically acceptable salt" refers to a salt of the antibody-drug conjugates of the present disclosure. Such salts are safe and effective when used in a subject and possess the required biological activity. The ligand drug conjugate of the present disclosure at least comprises one amino group and thus may form a salt with an acid. Non-limiting examples of pharmaceutically acceptable salts include: hydrochloride, hydrobromide, hydriodate, sulphate, bisulfate, citrate, acetate, succinate, ascorbate, oxalate, nitrate, sorbate, hydrophosphate, dihydrophosphate, salicylate, hydrocitrate, tartrate, maleate, fumarate, formate, benzoate, mesylate, ethanesulfonate, benzenesulphonate and *p*-toluenesulfonate.

The term "drug loading" or "mean drug loading" refers to the mean number of cytotoxic drug loaded per ligand in ligand-drug conjugates, and may also be represented as the drug-to-antibody ratio. The drug loading may range from 0-12, preferably 1-10, cytotoxic drugs per ligand (Pc). In embodiments of the present disclosure, the drug loading is represented as n, which may also be referred to as a DAR (Drug-antibody Ratio) value, and exemplary values may be a mean of 1, 2, 3, 4, 5, 6, 7, 8, 9 and 10. The mean number of drugs per ADC molecule after coupling reactions can be characterized by conventional methods such as UV/visible spectroscopy, mass spectrometry, ELISA assays and HPLC.

In one embodiment of the present disclosure, the cytotoxic drug is conjugated to a mercapto group of the antibody by a linker unit.

The loading of the ligand-drug conjugate can be controlled by the following non-limiting methods, including:

(1) controlling a molar ratio of a linking reagent to a monoclonal antibody,
(2) controlling reaction time and temperature, and
(3) selecting different reagents.

For preparation of conventional pharmaceutical compositions, reference is made to *Chinese Pharmacopoeia.*
The term "carrier" for the drug of the present disclosure refers to a system that can alter the manner in which the drug gets into a human body and the distribution of the drug in the human body, control the release rate of the drug, and deliver the drug to a targeted organ. The drug carrier release and targeted system can reduce drug degradation and loss, reduce side effects and improve bioavailability. For example, polymeric surfactants that can be used as carriers can self-assemble due to their unique amphiphilic structures to form various forms of aggregates, such as micelles, microemulsions, gels, liquid crystals and vesicles, as preferred examples. The aggregates have the capability of encapsulating drug molecules and have good permeability for membranes, and therefore can be used as excellent drug carriers.
The term "excipient" is an addition, besides the main drug, to a pharmaceutical composition. It may also be referred to as an adjuvant. For example, binders, fillers, disintegrants, lubricants in tablets; base part in semisolid ointment and cream preparations; preservatives, antioxidants, corrigents, fragrances, cosolvents, emulsifiers, solubilizers, tonicity adjusting agents, colorants and the like in liquid formulations can all be referred to as excipients.
The term "diluent", also referred to as a filler, is used primarily to increase the weight and volume of the tablet. The addition of the diluent not only ensures a certain volume, but also reduces the dose deviation of the main ingredients, and improves the drug's compression moldability and the like. When the drug in tablet form contains oily components, an absorbent is necessarily added to absorb the oily components so as to maintain a "dry" state and thus to facilitate the preparation of the tablet. Examples include starch, lactose, inorganic salts of calcium, microcrystalline cellulose and the like.
The pharmaceutical composition may be in a form of a sterile injectable aqueous solution. Available and acceptable vehicles or solvents include water, Ringer's solution and isotonic sodium chloride solution. The sterile injectable formulation may be a sterile injectable oil-in-water microemulsion in which the active ingredient is dissolved in the oil phase. For example, the active ingredient is dissolved in a mixture of soybean oil and lecithin. The oil solution is then added to a mixture of water and glycerol and treated to form a microemulsion. The injection or microemulsion can be locally injected into the bloodstream of a subject in large quantities. Alternatively, it may be desirable to administer solutions and microemulsions in such a way as to maintain a constant circulating concentration of the compound disclosed herein. To maintain such a constant concentration, a continuous intravenous delivery device may be used. An example of such a device is a Deltec CADD-PLUS. TM. 5400 intravenous injection pump.
The pharmaceutical composition may be in a form of a sterile injectable aqueous or oily suspension for intramuscular and subcutaneous administration. The suspension can be prepared according to the prior art using those suitable dispersants or wetting agents and suspending agents mentioned above. The sterile injectable formulation may also be a sterile injection or suspension prepared in a parenterally acceptable non-toxic diluent or solvent, e.g., a solution prepared in 1,3-butanediol. In addition, a sterile fixed oil may be conventionally used as a solvent or a suspending medium. For this purpose, any blend fixed oil including synthetic monoglycerides or diglycerides can be used. In addition, fatty acids such as oleic acid may also be used in the preparation of injections.

2. Synthesis Method

For the synthesis purpose, the following technical schemes for synthesis are adopted.
A method for preparing a compound of general formula (Pc-$L_a$-Y-D) comprises the following steps:

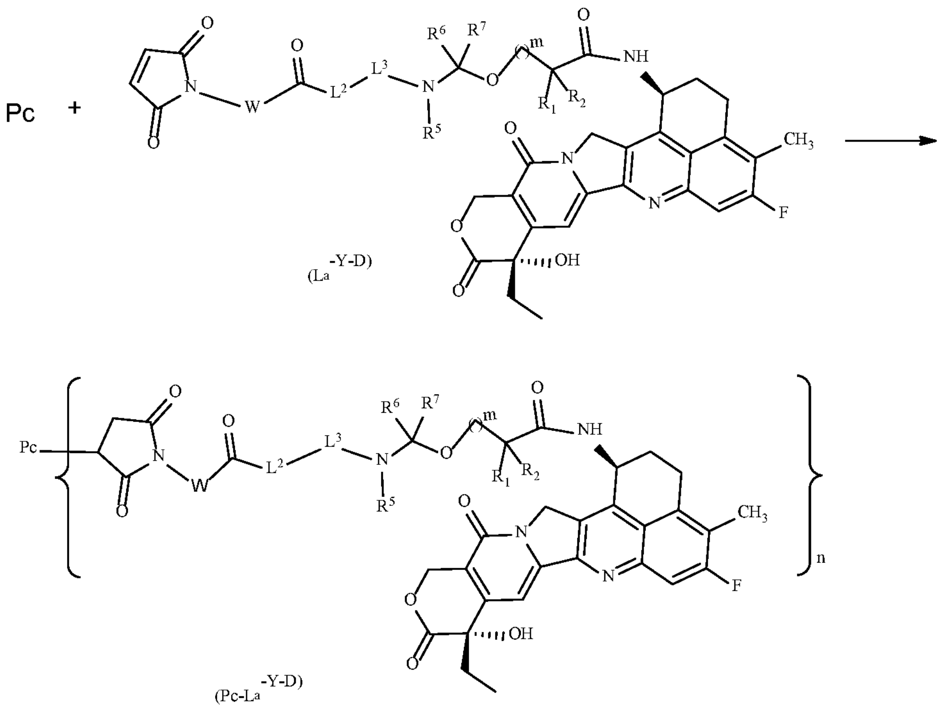

($L_a$-Y-D)

(Pc-$L_a$-Y-D)

subjecting reduced Pc and general formula ($L_a$-Y-D) to a coupling reaction to give a compound of general formula (Pc-$L_a$-Y-D), wherein the reducing agent is preferably TCEP; particularly, the disulfide bonds in the antibody are preferably reduced; wherein:

Pc, W, $L^2$, $L^3$, $R^1$, $R^2$, $R^5$, $R^6$, $R^7$, m and n are as defined in the aforementioned general formula (Pc-$L_a$-Y-D).

One or more embodiments of the present disclosure are described in detail in the specification above. Although any methods and materials similar or identical to those described herein can also be used to implement or test the present disclosure, preferred methods and materials are described below. Other features, purposes and advantages of the present disclosure will be apparent from the description and the claims. In the specification and claims, singular forms include plural referents unless otherwise indicated clearly in the context. Unless otherwise defined, all technical and scientific terms used herein have the meanings generally understood by those of ordinary skill in the art to which the present disclosure belongs. All the patents and publications cited in the specification are incorporated by reference. The following examples are set forth in order to more fully illustrate the preferred embodiments of the present disclosure. These examples should not be construed in any way as limiting the scope of the present disclosure that is defined by the claims.

## Examples

Experimental procedures without specific conditions indicated in the following examples and test examples, are generally conducted according to conventional conditions, or according to conditions recommended by the manufacturers of the starting materials or commercial products, see Sambrook et al., Molecular Cloning: A Laboratory Manual, Cold Spring Harbor Laboratory Press; CurrentProtocols in Molecular Biology, Ausubel et al., Greene Publishing Association, Wiley Interscience, NY. Reagents without specific origins indicated are commercially available conventional reagents.

### 1. Preparation of Antibody

### Example 1-1. Construction of Cell Strain with High TROP-2 Expression

pCDH-hTROP-2 lentiviral expression vector plasmids, pVSV-G and pCMV-dR8.91 lentiviral system packaging vectors were transfected into viral packaging cells 293T using Lipofectamine 3000 transfection reagent. The medium supernatant containing viruses was collected, filtered, and centrifuged at ultra-high speed. Chinese hamster ovary cells CHO-K1 was allowed to be infected with the concentrated virus, screened using puromycin for two to three weeks, and subjected to FACS single-cell sorting.

According to the TROP-2 expression level on the surface of the CHO-K1 cells infected by lentivirus determined

by FACS, CHO-K1/hTROP-2 monoclonal cell strains with high TROP-2 expression were selected.

Amino acid sequence of TROP-2 (Genbank: NP_002344.2) is as follows:

MARGPGLAPPPLRLPLLLLVLAAVTGHTAAQDNCTCPTNKMTVCSPDGPGGRC
QCRALGSGMAVDCSTLTSKCLLLKARMSAPKNARTLVRPSEHALVDNDGLYD
PDCDPEGRFKARQCNQTSVCWCVNSVGVRRTDKGDLSLRCDELVRTHHILIDL
RHRPTAGAFNHSDLDAELRRLFRERYRLHPKFVAAVHYEQPTIQIELRQNTSQK
AAGDVDIGDAAYYFERDIKGESLFQGRGGLDLRVRGEPLQVERTLIYYLDEIPP
KFSMKRLTAGLIAVIVVVVVALVAGMAVLVITNRRKSGKYKKVEIKELGELRK
EPSL

SEQ ID NO: 1;

Amino acid sequence of Trop2-His:

MARGPGLAPPPLRLPLLLLVLAAVTGHTAAQDNCTCPTNKMTVCSPDGPGGRC
QCRALGSGMAVDCSTLTSKCLLLKARMSAPKNARTLVRPSEHALVDNDGLYD
PDCDPEGRFKARQCNQTSVCWCVNSVGVRRTDKGDLSLRCDELVRTHHILIDL
RHRPTAGAFNHSDLDAELRRLFRERYRLHPKFVAAVHYEQPTIQIELRQNTSQK
AAGDVDIGDAAYYFERDIKGESLFQGRGGLDLRVRGEPLQVERTLIYYLDEIPP
KFSMKRLTAGLIAVIVVVVVALVAGMAVLVITNRRKSGKYKKVEIKELGELRK
EPSLHHHHHH

SEQ ID NO: 2.

**Example** 1-2. **Preparation of Anti-Human TROP-2 Monoclonal Antibody**

The anti-human TROP-2 monoclonal antibody in the present application was prepared according to the method disclosed in WO03074566, and the site mutation modification design was carried out on CDRs by using computer software and taking the antibody variable region gene of hRS7 as a template. The antibody variable region gene was inserted into a protein expression vector Phr-IgG (with signal peptide and constant region gene (CH1-Fc/CL) fragment) by molecular cloning and then expressed in HEK293 and Expi-CHO-S cells. Antibody purification was performed according to a conventional method. Activity verification was performed by using CHO-K1 cells and huTROP-2 protein (His27-Thr274 accession No. NP_002344.2) over-expressing huTROP-2 protein, and an antibody with better target binding activity was selected, wherein the variable region sequence of PD3 is as follows:

Heavy chain variable region of PD3:

EVQLVQSGSELKKPGASVKVSCKASGYTFTNYGMNWVKQAPGQGLKWMGWI
NTYTGEPTYTQDFKGRFAFSLDTSVSTAYLQISSLKAEDTAVYYCARGGFGSSY
WYFDVWGQGTLVTVSS

SEQ ID NO: 3;

Light chain variable region of PD3:

DIQLTQSPSSLSASVGDRVSITCKASQDVSIAVAWYQQKPGKAPKLLIYSASYRYTGVPDRFSGSGSGTDFTLTISSLQPEDFAVYYCQQHYITPLTFGAGTKVEIK
SEQ ID NO: 4;

Note: the underlined portions are CDRs determined according to Kabat numbering scheme.

Table 1. CDRs of PD3 antibodies

| **Antibodies** | **PD3** |
|---|---|
| **Heavy chain CDR1** | NYGMN (SEQ ID NO: 5) |
| **Heavy chain CDR2** | WINTYTGEPTYTQDFKG (SEQ ID NO: 6) |
| **Heavy chain CDR3** | GGFGSSYWYFDV (SEQ ID NO: 7) |
| **Light chain CDR1** | KASQDVSIAVA (SEQ ID NO: 8) |
| **Light chain CDR2** | SASYRYT (SEQ ID NO: 9) |
| **Light chain CDR3** | QQHYITPLT (SEQ ID NO: 10) |

The heavy chain constant region of the antibody may be selected from the constant regions of human IgG1, IgG2, IgG4 and variants thereof, and the light chain constant region of the antibody may be selected from the light chain constant regions of human κ and λ chains and variants thereof. Illustratively, the heavy chain constant region of the antibody is selected from the constant region of human IgG1 having a sequence set forth in SEQ ID NO: 11, and the light chain constant region of the antibody is selected from the constant region of human κ chain having a sequence set forth in SEQ ID NO: 12.

Heavy chain constant region of human IgG1:

ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK
SEQ ID NO: 11;

Humanized κ light chain constant region:

RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC
SEQ ID NO: 12;

Illustratively, the light/heavy chain constant regions described above are combined with the variable regions of the aforementioned PD3 antibody to form a complete antibody, the light/heavy chain sequences of which are as follows:

Heavy chain of PD3:

EVQLVQSGSELKKPGASVKVSCKASGYTFTNYGMNWVKQAPGQGLKWMGWI
NTYTGEPTYTQDFKGRFAFSLDTSVSTAYLQISSLKAEDTAVYYCARGGFGSSY
WYFDVWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSN
TKVDKKVEPKSCDKTHTCPPCPAPELLGGPSVFLFPPKPKDTLMISRTPEVTCVV
VDVSHEDPEVKFNWYVDGVEVHNAKTKPREEQYNSTYRVVSVLTVLHQDWL
NGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGN
VFSCSVMHEALHNHYTQKSLSLSPGK
SEQ ID NO: 13;

Light chain of PD3:

DIQLTQSPSSLSASVGDRVSITCKASQDVSIAVAWYQQKPGKAPKLLIYSASYRY
TGVPDRFSGSGSGTDFTLTISSLQPEDFAVYYCQQHYITPLTFGAGTKVEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC
SEQ ID NO: 14;

The control molecule hRS7 used in the present disclosure is constructed with reference to Patent WO03074566 and the TINA antibody is constructed with reference to Patent WO2015098099a1, the sequences of which are as follows:

Heavy chain of hRS7:

*QVQLQQSGSELKKPGASVKVSCKASGYTFTNYGMNWVKQAPGQGLKWMGWINTYT*
*GEPTYTDDFKGRFAFSLDTSVSTAYLQISSLKADDTAVYFCARGGFGSSYWYFDVWG*
*QGSLVTVSS*ASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEP
KSCDKTHTCPPCPAPELLGGPSVFLFPPKPKDTLMISRTPEVTCVVVDVSHEDPE

VKFNWYVDGVEVHNAKTKPREEQYNSTYRVVSVLTVLHQDWLNGKEYKCKV
SNKALPAPIEKTISKAKGQPREPQVYTLPPSRDELTKNQVSLTCLVKGFYPSDIA
VEWESNGQPENNYKTTPPVLDSDGSFFLYSKLTVDKSRWQQGNVFSCSVMHE
ALHNHYTQKSLSLSPGK
SEQ ID NO: 15;

Light chain of hRS7:

*DIQLTQSPSSLSASVGDRVSITCKASQDVSIAVAWYQQKPGKAPKLLIYSASYRYTGVPDRFSGSGSGTDFTLTISSLQPEDFAVYYCQQHYITPLTFGAGTKVEIK*RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC

SEQ ID NO: 16;

Heavy chain of TINA:

*QVQLVQSGAEVKKPGASVKVSCKASGYTFTTAGMQWVRQAPGQGLEWMGWINTHSGVPKYAEDFKGRVTISADTSTSTAYLQLSSLKSEDTAVYYCARSGFGSSYWYFDVWGQGTLVTVSS*ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK

SEQ ID NO: 17;

Light chain of TINA:

*DIQMTQSPSSLSASVGDRVTITCKASQDVSTAVAWYQQKPGKAPKLLIYSASYRYTGVPSRFSGSGSGTDFTLTISSLQPEDFAVYYCQQHYITPLTFGQGTKLEIK*RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC

SEQ ID NO: 18.

**2. Preparation of Compounds**

Experimental procedures without conditions specified in the Examples of the present disclosure, are generally conducted according to conventional conditions, or according to conditions recommended by the manufacturer of the starting materials or commercial products. Reagents without specific origins indicated are commercially available conventional reagents.

The structure of the compounds were determined by nuclear magnetic resonance (NMR) or mass spectrometry (MS). NMR spectra was measured using a Bruker AVANCE-400 nuclear magnetic resonance instrument, with deuterated dimethyl sulfoxide (*DMSO-d6*), deuterated chloroform ($CDCl_3$) and deuterated methanol ($CD_3OD$) as determination solvents and tetramethylsilane (TMS) as internal standard. Chemical shifts are given in unit of $10^{-6}$ (ppm).

MS analysis was performed using a FINNIGAN LCQAd (ESI) mass spectrometer (manufacturer: Thermo, model: Finnigan LCQ advantage MAX).

UPLC analysis was performed using a Waters Acquity UPLC SQD liquid chromatography-mass spectrometry system.

HPLC analysis was performed using an Agilent 1200DAD high pressure liquid chromatograph (Sunfire C18 150 × 4.6 mm chromatography column) and a Waters 2695-2996 high pressure liquid chromatograph (Gimini C18 150 × 4.6 mm chromatography column).

UV-HPLC analysis was performed using a Thermo nanodrop2000 ultraviolet spectrophotometer.

Proliferation inhibition rates and $IC_{50}$ values were measured using a PHERA starFS microplate reader (BMG, Germany).

Huanghai HSGF254 or Qingdao GF254 silica gel plates of specifications 0.15 mm to 0.2 mm were adopted for thin layer chromatography (TLC) analysis and 0.4 mm to 0.5 mm for TLC separation and purification.

Yantai Yellow Sea silica gel of 200-300 mesh is generally used as a carrier in column chromatography.

Known starting materials of the present disclosure may be synthesized using or according to methods known in the art, or may be purchased from ABCR GmbH & Co.KG, Acros Organnics, Aldrich Chemical Company, Accela ChemBio Inc, Chembee Chemicals and the like.

In the Examples, the reactions were performed in an argon atmosphere or a nitrogen atmosphere unless otherwise stated.

An argon atmosphere or a nitrogen atmosphere means that the reaction flask is connected to a balloon containing about 1 L of argon or nitrogen.

A hydrogen atmosphere means that the reaction flask is connected to a balloon containing about 1 L of hydrogen.

A Parr 3916EKX hydrogenator, a Qinglan QL-500 hydrogenator or a HC2-SS hydrogenator was used for pressurized hydrogenation reactions.

The hydrogenation reaction usually involved 3 cycles of vacuumization and hydrogen purge.

A CEM Discover-S 908860 microwave reactor was used for the microwave reaction.

In the Examples, the solution in the reaction refers to an aqueous solution unless otherwise stated.

In the Examples, the reaction temperature is room temperature unless otherwise stated. The room temperature is the optimum reaction temperature, which ranges from 20 °C to 30 °C.

Preparation of PBS buffer at pH 6.5 in Examples: 8.5 g of $KH_2PO_4$, 8.56 g of $K_2HPO_4.3H_2O$, 5.85 g of NaCl and 1.5 g of EDTA were added to a flask, and the volume was brought to 2 L. The additions were all ultrasonically dissolved, and the solution was well mixed by shaking to give the desired buffer.

The eluent system for column chromatography and the developing solvent system for thin layer chromatography used for compound purification include: A: dichloromethane and isopropanol system, B: dichloromethane and methanol system, and C: petroleum ether and ethyl acetate system. The volume ratio of solvents was adjusted according to the polarity of the compound, or by adding a small amount of triethylamine and acidic or basic reagent.

Some of the compounds of the present disclosure are characterized by Q-TOF LC/MS. Q-TOF LC/MS analysis used an Agilent 6530 accurate-mass quadrupole time-of-flight mass spectrometer and an Agilent 1290-Infinity ultra-high performance liquid chromatograph (Agilent Poroshell 300SB-C8 5 μm, 2.1 × 75 mm chromatography column).

Y-D drug portion of the antibody-drug conjugates of the present disclosure is found in PCT/CN2019/107873, and the synthesis and tests of relevant compounds are incorporated herein by reference. Non-limiting Examples of synthesis are incorporated by reference as follows:

**Example 2-1**

*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-1-hydroxycyclopropane-1-carboxamide **1**

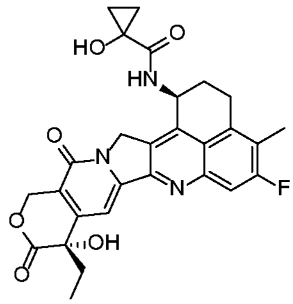

1

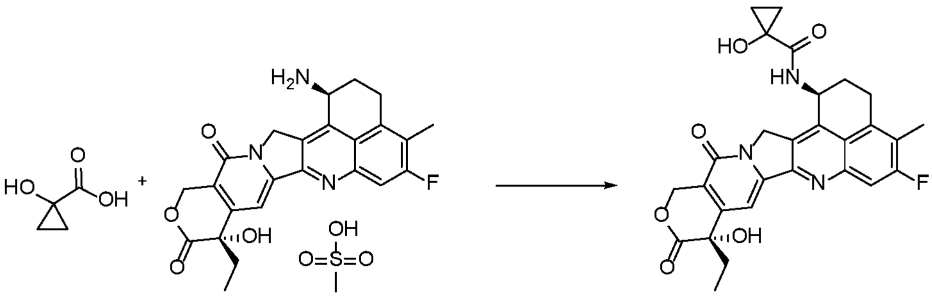

1a 1b 1

To exatecan mesylate 1b (2.0 mg, 3.76 μmol, prepared as disclosed in Patent Application "EP0737686A1") was added 1 mL of *N,N*-dimethylformamide. The mixture was cooled to 0-5 °C in an ice-water bath, and a drop of triethylamine was added. The reaction was stirred until it became clear. To the reaction mixture were successively added 1-hydroxycyclopropylcarboxylic acid 1a (1.4 mg, 3.7 μmol, prepared using known method "Tetrahedron Letters, 25(12), 1269-72; 1984") and 4-(4,6-dimethoxy-1,3,5-triazin-2-yl)-4-methylmorpholinium chloride (3.8 mg, 13.7 μmol). After addition, the reaction mixture was stirred at 0-5 °C for 2 h, quenched with 5 mL of water, and extracted with ethyl acetate (8 mL × 3). The organic phases were combined, washed with saturated sodium chloride solution (5 mL × 2), dried over anhydrous sodium sulfate, and filtered. The filtrate was concentrated under reduced pressure, and the resulting residue was purified by thin layer chromatography with developing solvent system B to give the title product 1 (1.6 mg, 82.1% yield).

MS m/z (ESI): 520.2 [M+1].

$^1H$ NMR (400 MHz, $CDCl_3$): δ 7.90-7.84 (m, 1H), 7.80-7.68(m, 1H), 5.80-5.70 (m, 1H), 5.62-5.54(m, 2H), 5.44-5.32 (m, 2H), 5.28-5.10(m, 2H), 3.40-3.15 (m, 3H), 2.44 (s, 3H), 2.23(t, 1H), 2.06-1.75 (m, 2H), 1.68-1.56 (m, 1H), 1.22-1.18 (m, 2H), 1.04-0.98 (m, 2H), 0.89 (t, 3H).

**Example 2-2**

(*S*)-2-cyclopropyl-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-2-hydroxyacetamide **2-A**

(*R*)-2-cyclopropyl-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-2-hydroxyacetamide **2-B**

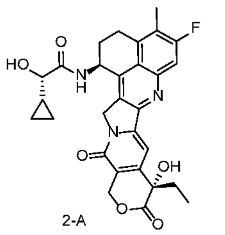
2-A

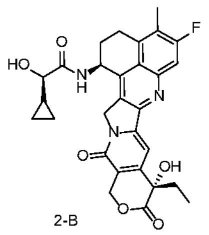
2-B

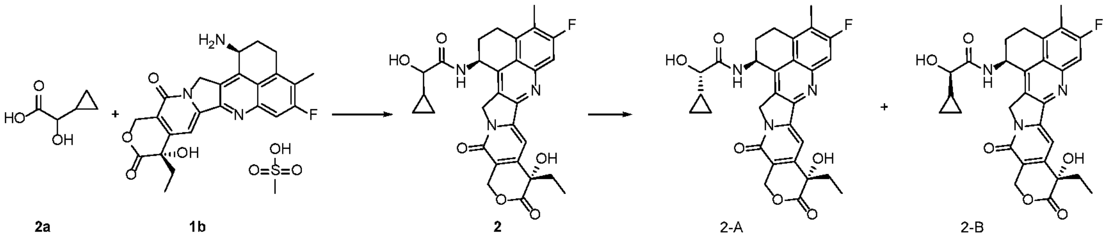
2a 1b 2 2-A 2-B

To **1b** (4 mg, 7.53 μmol) were added 2 mL of ethanol and 0.4 mL of *N,N*-dimethylformamide. The system was purged with argon three times, and the mixture was cooled to 0-5 °C in an ice-water bath, followed by dropwise addition of 0.3 mL of *N*-methylmorpholine. The reaction mixture was stirred until it became clear. To the reaction mixture were successively added 2-cyclopropyl-2-hydroxyacetic acid **2a** (2.3 mg, 19.8 μmol, prepared as disclosed in Patent Application "WO2013106717"), 1-hydroxybenzotriazole (3 mg, 22.4 μmol) and 1-(3-dimethylaminopropyl)-3-ethylcarbodiimide hydrochloride (4.3 mg, 22.4 μmol). After addition, the reaction mixture was stirred at 0-5 °C for 1 h. The ice-water bath was removed, and the reaction mixture was heated to 30 °C, stirred for 2 h, and concentrated under reduced pressure. The resulting crude compound **2** was purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient elution, flow rate: 18 mL/min), and the corresponding fractions were collected and concentrated under reduced pressure to give the title product (2-A: 1.5 mg, 2-B: 1.5 mg).

MS m/z (ESI): 534.0 [M+1].

Single-configuration compound 2-B (shorter retention time)

UPLC analysis: retention time: 1.06 min; purity: 88% (chromatography column: ACQUITY UPLC BEHC18 1.7 μm 2.1 × 50 mm; mobile phase: A-water (5 mmol of $NH_4OAc$), B-acetonitrile).

$^1$H NMR (400 MHz, DMSO-$d_6$): δ 8.37 (d, 1H), 7.76 (d, 1H), 7.30 (s, 1H), 6.51 (s, 1H), 5.58-5.56 (m, 1H), 5.48 (d, 1H), 5.41 (s, 2H), 5.32-5.29 (m, 2H), 3.60 (t, 1H), 3.19-3.13 (m, 1H), 2.38 (s, 3H), 2.20-2.14 (m, 1H), 1.98 (q, 2H), 1.87-1.83 (m, 1H), 1.50-1.40 (m, 1H), 1.34-1.28 (m, 1H), 0.86 (t, 3H), 0.50-0.39 (m, 4H).

Single-configuration compound 2-A (longer retention time)

UPLC analysis: retention time: 1.10 min; purity: 86% (chromatography column: ACQUITY UPLC BEHC18 1.7 μm 2.1 × 50 mm; mobile phase: A-water (5 mmol of $NH_4OAc$), B-acetonitrile).

$^1$H NMR (400 MHz, DMSO-$d_6$): δ 8.35 (d, 1H), 7.78 (d, 1H), 7.31 (s, 1H), 6.52 (s, 1H), 5.58-5.53 (m, 1H), 5.42 (s, 2H), 5.37 (d, 1H), 5.32 (t, 1H), 3.62 (t, 1H), 3.20-3.15 (m, 2H), 2.40 (s, 3H), 2.25-2.16 (m, 1H), 1.98 (q, 2H), 1.87-1.82 (m, 1H), 1.50-1.40 (m, 1H), 1.21-1.14 (m, 1H), 0.87 (t, 3H), 0.47-0.35 (m, 4H).

**Example 2-3**

(*S*)-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-3,3,3-trifluoro-2-hydroxypropionamide **3-A**

(*R*)-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-3,3,3-trifluoro-2-hydroxypropionamide **3-B**

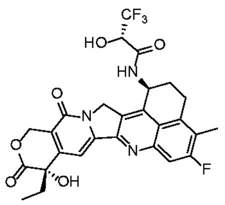

3-A

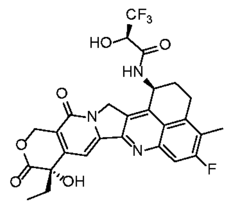

3-B

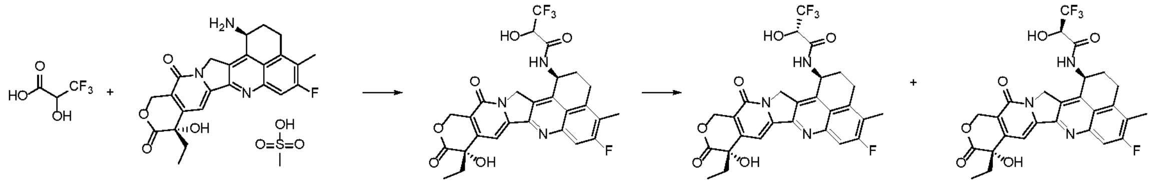

3a 1b 3 3-A 3-B

To **1b** (5.0 mg, 9.41 μmol) were added 2 mL of ethanol and 0.4 mL of *N,N*-dimethylformamide, and the mixture was cooled to 0-5 °C in an ice-water bath, followed by dropwise addition of 0.3 mL of *N*-methylmorpholine. The reaction mixture was stirred until it became clear. To the reaction mixture were successively added 3,3,3-trifluoro-2-hydroxypropionic acid **3a** (4.1 mg, 28.4 μmol, supplied by Alfa), 1-hydroxybenzotriazole (3.8 mg, 28.1 μmol) and 1-(3-dimethylaminopropyl)-3-ethylcarbodiimide hydrochloride (5.4 mg, 28.2 μmol). After addition, the reaction mixture was stirred at 0-5 °C for 10 min. The ice-water bath was removed, and the reaction mixture was heated to 30 °C, stirred for 8 h, and concentrated under reduced pressure. The resulting crude compound **3** was purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient elution, flow rate: 18 mL/min), and the corresponding fractions were collected and concentrated under reduced pressure to give the title product (1.5 mg, 1.5 mg).

MS m/z (ESI): 561.9 [M+1].

Single-configuration compound (shorter retention time)

UPLC analysis: retention time: 1.11 min; purity: 88% (chromatography column: ACQUITY UPLC BEHC18 1.7 μm 2.1 × 50 mm; mobile phase: A-water (5 mmol of $NH_4OAc$), B-acetonitrile).

$^1$H NMR (400 MHz, DMSO-$d_6$): δ 8.94 (d, 1H), 7.80 (d, 1H), 7.32 (s, 1H), 7.20 (d, 1H), 6.53 (s, 1H), 5.61-5.55 (m, 1H), 5.45-5.23 (m, 3H), 5.15-5.06 (m, 1H), 4.66-4.57 (m, 1H), 3.18-3.12 (m, 1H), 2.40 (s, 3H), 2.26-2.20 (m, 1H), 2.16-2.08 (m, 1H), 2.02-1.94 (m, 1H), 1.89-1.82 (m, 1H), 1.50-1.40 (m, 1H), 0.87 (t, 3H).

Single-configuration compound (longer retention time)

UPLC analysis: retention time: 1.19 min; purity: 90% (chromatography column: ACQUITY UPLC BEHC18 1.7 μm 2.1 × 50 mm; mobile phase: A-water (5 mmol of $NH_4OAc$), B-acetonitrile).

$^1$H NMR (400 MHz, DMSO-$d_6$): δ 8.97 (d, 1H), 7.80 (d, 1H), 7.31 (s, 1H), 7.16 (d, 1H), 6.53 (s, 1H), 5.63-5.55 (m, 1H), 5.45-5.20 (m, 3H), 5.16-5.07 (m, 1H), 4.66-4.57 (m, 1H), 3.18-3.12 (m, 1H), 2.40 (s, 3H), 2.22-2.14 (m, 1H), 2.04-1.95 (m, 2H), 1.89-1.82 (m, 1H), 1.50-1.40 (m, 1H), 0.87 (t, 3H).

## Example 2-4

*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-1-hydroxycyclopentane-1-carboxamide **4**

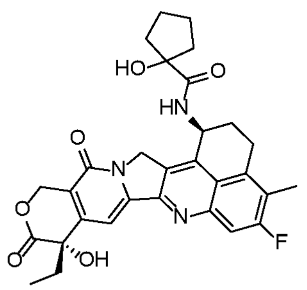

4

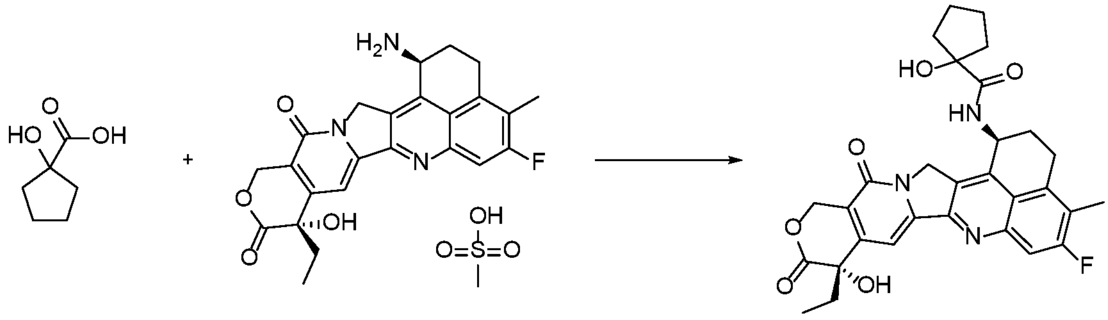

4a 1b 4

To **1b** (3.0 mg, 5.64 μmol) was added 1 mL of *N,N*-dimethylformamide. The mixture was cooled to 0-5 °C in an ice-water bath, and a drop of triethylamine was added. The reaction mixture was stirred until it became clear. To the reaction mixture were successively added 1-hydroxy-cyclopentanecarboxylic acid **4a** (2.2 mg, 16.9 μmol, prepared as disclosed in Patent Application "WO2013106717") and 4-(4,6-dimethoxy-1,3,5-triazin-2-yl)-4-methylmorpholinium chloride (4.7 mg, 16.9 μmol). After addition, the reaction mixture was stirred at 0-5 °C for 1 h, quenched with 5 mL of water, and extracted with ethyl acetate (10 mL × 3). The organic phases were combined, washed with saturated sodium chloride solution (5 mL × 2), dried over anhydrous sodium sulfate, and filtered. The filtrate was concentrated under reduced pressure, and the resulting residue was purified by thin layer chromatography with developing solvent system B to give the title product **4** (2.5 mg, 80.9% yield).

MS m/z (ESI): 548.0 [M+1].

$^1$H NMR (400 MHz, $CDCl_3$): δ 7.73-7.62 (m, 2H), 5.75-5.62 (m, 1H), 5.46-5.32 (m, 2H), 5.26-5.10 (m, 1H), 3.30-3.10 (m, 1H), 2.43 (s, 3H), 2.28-2.20 (m, 2H), 2.08-1.84 (m, 8H), 1.69-1.58 (m, 2H), 1.04-1.00 (m, 2H), 0.89 (t, 3H).

**Example 2-5**

*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-1-(hydroxymethyl)cyclopropane-1-carboxamide **5**

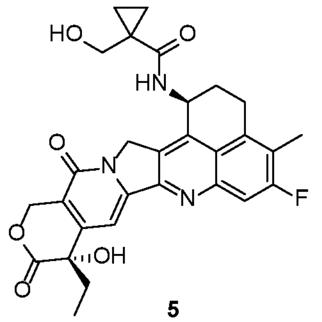

5

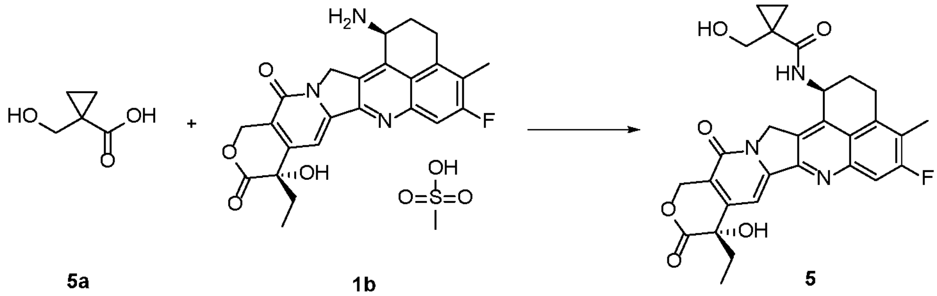

5a 1b 5

To **1b** (2.0 mg, 3.76 µmol) was added 1 mL of *N,N*-dimethylformamide. The mixture was cooled to 0-5 °C in an ice-water bath, and a drop of triethylamine was added. The reaction mixture was stirred until it became clear. To the reaction mixture were successively added 1-(hydroxymethyl)-cyclopentanecarboxylic acid **5a** (0.87 mg, 7.5 µmol, prepared as disclosed in Patent Application "WO201396771") and 4-(4,6-dimethoxy-1,3,5-triazin-2-yl)-4-methylmorpholinium chloride (2 mg, 7.24 µmol). After addition, the reaction mixture was stirred at 0-5 °C for 2 h, quenched with 5 mL of water, and extracted with ethyl acetate (8 mL × 3). The organic phases were combined, washed with saturated sodium chloride solution (5 mL × 2), dried over anhydrous sodium sulfate, and filtered. The filtrate was concentrated under reduced pressure, and the resulting residue was purified by thin layer chromatography with developing solvent system B to give the title product 5 (1.0 mg, 50% yield).

MS m/z (ESI): 533.9 [M+1].

$^1H$ NMR (400 MHz, $CDCl_3$): δ 8.07 (s, 1H), 7.23-7.18 (m, 2H), 6.71-6.64 (m, 1H), 6.55-6.51 (m, 1H), 5.36-5.27 (m, 2H), 4.67-4.61 (m, 2H), 3.53-3.48 (m, 1H), 3.30-3.22 (m, 2H), 3.18-3.13 (m, 1H), 2.71-2.61 (m, 2H), 2.35-2.28 (m, 1H), 2.04-1.91 (m, 4H), 1.53-1.40 (m, 3H), 0.91-0.75 (m, 4H).

**Example 2-6**

*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-1-(hydroxymethyl)cyclobutane-1-carboxamide **6**

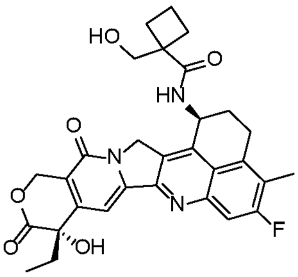

**6**

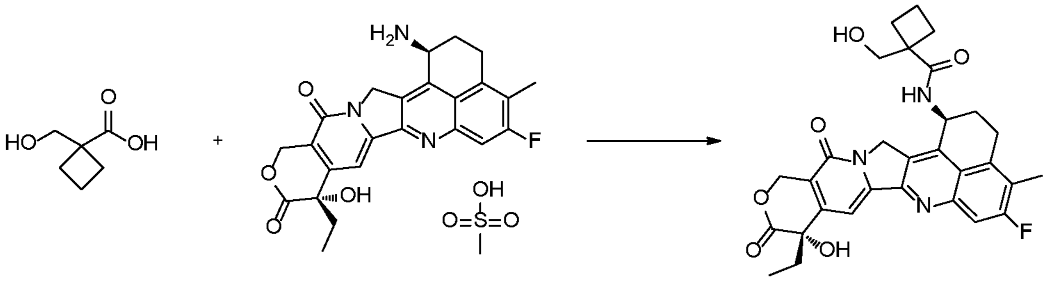

**6a** **1b** **6**

To **1b** (3.0 mg, 5.64 μmol) was added 1 mL of *N,N*-dimethylformamide. The mixture was cooled to 0-5 °C in an ice-water bath, and a drop of triethylamine was added. The reaction mixture was stirred until it became clear. To the reaction mixture were successively added 1-(hydroxymethyl)cyclobutane-1-carboxylic acid **6a** (2.2 mg, 16.9 μmol, prepared as disclosed in "Journal of the American Chemical Society, 2014, vol.136, #22, p.8138-8142*")* and 4-(4,6-dimethoxy-1,3,5-triazin-2-yl)-4-methylmorpholinium chloride (4.7 mg, 16.9 μmol). After addition, the reaction mixture was stirred at 0-5 °C for 1 h, quenched with 5 mL of water, and extracted with ethyl acetate (10 mL × 3). The organic phases were combined, washed with saturated sodium chloride solution (5 mL × 2), dried over anhydrous sodium sulfate, and filtered. The filtrate was concentrated under reduced pressure, and the resulting residue was purified by thin layer chromatography with developing solvent system B to give the title product **6** (2.1 mg, 67.9% yield).

MS m/z (ESI): 548.0 [M+1].

$^1$H NMR (400 MHz, DMSO-$d_6$): δ 7.85-7.62 (m, 1H), 6.88 (br, 1H), 5.87-5.48 (m, 2H), 5.47-5.33 (m, 1H), 5.31-5.06 (m, 1H), 4.25-3.91 (m, 2H), 3.25 (br, 1H), 2.60-2.32 (m, 3H), 2.23 (t, 1H), 2.15-1.95 (m, 3H), 1.70-1.56 (m, 2H), 1.41-1.17 (m, 9H), 1.03 (s, 1H), 0.95-0.80 (m, 2H).

**Example 2-7**

*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-1-hydroxycyclobutane-1-carboxamide 7

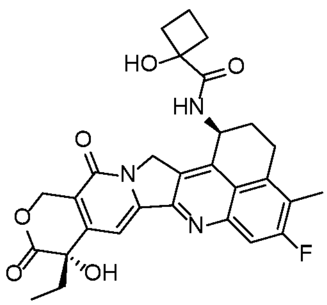

**7**

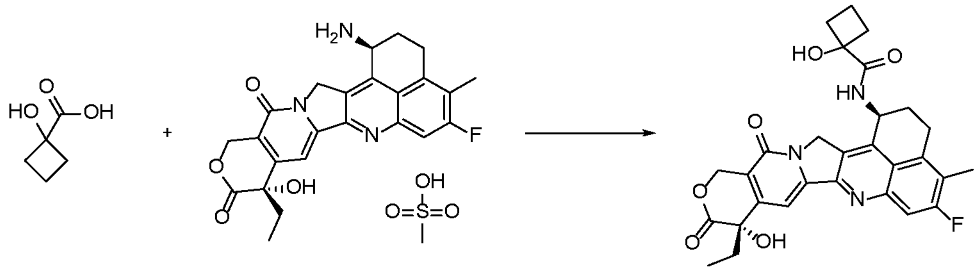

**7a** **1b** **7**

To **1b** (3.0 mg, 5.64 μmol) were added 2 mL of ethanol and 0.4 mL of *N,N*-dimethylformamide, and the mixture was cooled to 0-5 °C in an ice-water bath, followed by dropwise addition of 0.3 mL of *N*-methylmorpholine. The reaction mixture was stirred until it became clear. To the reaction mixture were successively added 1-hydroxycyclobutanecarboxylic acid **7a** (2.0 mg, 17.22 μmol, supplied by PharmaBlock), 1-hydroxybenzotriazole (2.3 mg, 17.0 μmol) and 1-(3-dimethylaminopropyl)-3-ethylcarbodiimide hydrochloride (3.2 mg, 16.7 μmol). After addition, the reaction mixture was stirred at 0-5 °C for 10 min. The ice-water bath was removed, and the reaction mixture was stirred at room temperature for 2 h and concentrated under reduced pressure. The resulting residue was purified by thin layer chromatography with developing solvent system B to give the title product 7 (2.5 mg, 83.1% yield).

MS m/z (ESI): 534.0 [M+1].

$^1H$ NMR (400 MHz, DMSO-$d_6$): δ 8.28 (d, 1H), 7.75 (d, 1H), 7.29 (s, 1H), 6.51 (s, 1H), 6.12 (s, 1H), 5.59-5.51 (m, 1H), 5.41 (s, 2H), 5.20-5.01 (m, 2H), 3.27-3.17 (m, 1H), 3.15-3.05 (m, 1H), 2.71-2.63 (m, 1H), 2.37 (s, 3H), 2.12-2.05 (m, 1H), 2.03-1.94 (m, 2H), 1.92-1.78 (m, 4H), 1.50-1.42 (m, 1H), 0.90-0.83 (m, 4H).

**Example 2-8**

1-(((*S*)-7-benzyl-20-(2,5-dioxo-2,5-dihydro-1*H*-pyrrol-1-yl)-3,6,9,12,15-pentaoxo-2,5,8,11,14-pentaazaeicosyl)oxy)-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)cyclopropane-1-carboxamide 8

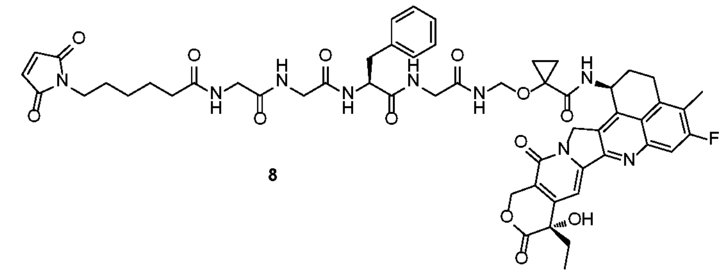

8

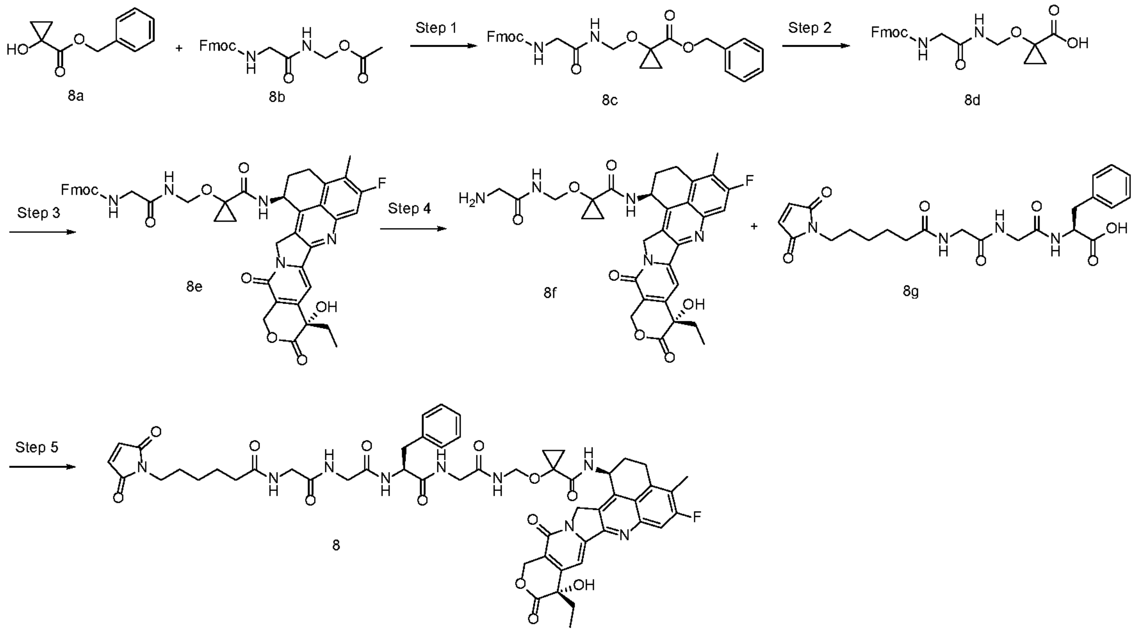


8a 8b 8c 8d 8e 8f 8g 8

Step 1

Benzyl 1-((2-((((9*H*-fluoren-9-yl)methoxy)carbonyl)amino)acetamido)methoxy)cyclopropane-1-carboxylate **8c** Benzyl 1-hydroxycyclopropane-1-carboxylate **8a** (104 mg, 0.54 mmol; prepared as disclosed in Patent Application "US2005/20645") and 2-((((9*H*-fluoren-9-yl)methoxy)carbonyl)amino)acetamido)methyl acetate **8b** (100 mg, 0.27 mmol; prepared as disclosed in Patent Application "CN105829346A") were added to a reaction flask, and 5 mL of tetrahydrofuran was added. The system was purged with argon three times, and the mixture was cooled to 0-5 °C in an ice-water bath, followed by addition of potassium *tert*-butoxide (61 mg, 0.54 mmol). The ice bath was removed, and the reaction mixture was warmed to room temperature and stirred for 10 min, followed by addition of 20 mL of ice water and by extraction with ethyl acetate (5 mL × 2) and chloroform (5 mL × 5). The organic phases were combined and concentrated. The resulting residue was dissolved in 3 mL of 1,4-dioxane, followed by addition of 0.6 mL of water, sodium bicarbonate (27 mg, 0.32 mmol) and 9-fluorenylmethyl chloroformate (70 mg, 0.27 mmol). The mixture was stirred at room temperature for 1 h. 20 mL of water was added, followed by extraction with ethyl acetate (8 mL × 3). The organic phase was washed with saturated sodium chloride solution (20 mL), dried over anhydrous sodium sulfate, and filtered. The filtrate was concentrated under reduced pressure, and the resulting residue was purified by silica gel column chromatography with developing solvent system B to give the title product **8c** (100 mg, 73.6% yield).

MS m/z (ESI): 501.0 [M+1].

Step 2

1-((2-((((9*H*-fluoren-9-yl)methoxy)carbonyl)amino)acetamido)methoxy)cyclopropane-1-carboxylicacid **8d**

**8c** (50 mg, 0.10 mmol) was dissolved in 3 mL of a solvent mixture of tetrahydrofuran and ethyl acetate (V:V = 2:1), and palladium on carbon (25 mg, 10% loading) was added. The system was purged with hydrogen three times, and the reaction mixture was stirred at room temperature for 1 h. The reaction mixture was filtered through celite, and the filter cake was rinsed with tetrahydrofuran. The filtrate was concentrated to give the title product **8d** (41 mg, 100% yield). MS m/z (ESI): 411.0 [M+1].

Step 3

(9*H*-fluoren-9-yl)methyl(2-(((1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)aminocarbonyl)cyclopropoxy)methyl)amino)-2-oxoethyl)carbamate **8e** **1b** (7 mg, 0.013 mmol) was added to a reaction flask, and 1 mL of *N,N*-dimethylformamide was added. The system was purged with argon three times, and the mixture was cooled to 0-5 °C in an ice-water bath, followed by addition of a drop of triethylamine, a solution of **8d** (7 mg, 0.017 mmol) in 0.5 mL of *N,N*-dimethylformamide, and 4-(4,6-dimethoxy-1,3,5-triazin-2-yl)-4-methylmorpholinium chloride (7 mg, 0.026 mmol). The reaction mixture was stirred in an ice bath for 35 min. 10 mL of water was added, followed by extraction with ethyl acetate (5 mL × 3). The organic phase was washed with saturated sodium chloride solution (10 mL), dried over anhydrous sodium sulfate, and filtered. The filtrate was concentrated under reduced pressure, and the resulting residue was purified by thin layer chromatography with developing solvent system B to give the title product **8e** (8.5 mg, 78.0% yield).
MS m/z (ESI): 828.0 [M+1].

Step 4

1-((2-Aminoacetylamino)methoxy)-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)cyclopropane-1-carboxamide **8f**

**8e** (4 mg, 4.84 μmol) was dissolved in 0.2 mL of dichloromethane, and 0.1 mL of diethylamine was added. The reaction mixture was stirred at room temperature for 2 h and concentrated under reduced pressure. 2 mL of toluene was added, followed by concentration under reduced pressure; the procedures were repeated twice. The residue was slurried with 3 mL of *n*-hexane, and the upper *n*-hexane layer was removed; the procedures were repeated three times. The slurry was concentrated under reduced pressure to give the crude title product **8f** (2.9 mg), which was directly used in the next step without purification.
MS m/z (ESI): 606.0 [M+1].

Step 5

1-(((*S*)-7-benzyl-20-(2,5-dioxo-2,5-dihydro-1*H*-pyrrol-1-yl)-3,6,9,12,15-pentaoxo-2,5,8,11,14-pentaazaeicosyl)oxy)-*N*-((1*S*,9*S*)-9-ethyl-5-fluoro-9-hydroxy-4-methyl-10,13-dioxo-2,3,9,10,13,15-hexahydro-1*H,*12*H*-benzo[*de*]pyrano[3',4':6,7]indolizino[1,2-*b*]quinolin-1-yl)cyclopropane-1-carboxamide **8**

Crude **8f** (2.9 mg, 4.84 μmol) was dissolved in 0.5 mL of *N,N*-dimethylformamide. The system was purged with argon three times, and the solution was cooled to 0-5 °C in an ice-water bath. A solution of (*S*)-2(-2-(-2-(6-(2,5-dioxo-1*H*-pyrrol-1-yl) hexanamido)acetylamino)acetylamino)-3-phenylpropionic acid **8g** (2.7 mg, 5.80 μmol, prepared as disclosed in Patent Application "EP2907824") in 0.3 mL of *N,N*-dimethylformamide was added, followed by addition of 4-(4,6-dimethoxy-1,3,5-triazin-2-yl)-4-methylmorpholinium chloride (2.7 mg, 9.67 μmol). The reaction mixture was stirred in an ice bath for 30 min. Then, the ice bath was removed, and the reaction mixture was warmed to room temperature, stirred for 15 min, and purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient elution, flow rate: 18 mL/min). The corresponding fractions were collected and concentrated under reduced pressure to give the title product **8** (2 mg, 39.0% yield).
MS m/z (ESI): 1060.0 [M+1].
$^1$H NMR (400 MHz, DMSO-$d_6$): δ 9.01 (d, 1H), 8.77 (t, 1H), 8.21 (t, 1H), 8.08-7.92 (m, 2H), 7.73 (d, 1H), 7.28 (s, 1H), 7.24-7.07 (m, 4H), 6.98 (s, 1H), 6.50 (s, 1H), 5.61 (q, 1H), 5.40 (s, 2H), 5.32 (t, 1H), 5.12 (q, 2H), 4.62 (t, 1H), 4.52 (t, 1H), 4.40-4.32 (m, 1H), 3.73-3.47 (m, 8H), 3.16-3.04 (m, 2H), 2.89 (dd, 1H), 2.69-2.55 (m, 2H), 2.37-2.23 (m, 4H), 2.12-1.93 (m, 4H), 1.90-1.74 (m, 2H), 1.52-1.38 (m, 4H), 1.33-1.11 (m, 5H), 0.91-0.81 (m, 4H).

**Example 2-9**

*N*-((2*R*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5,8,11,14-tetraazahexadecan-16-yl)-6-(2,5-dioxo-2,5-dihydro-1*H*-pyrrol-1-yl)hexanamide **9-A**

*N*-((2*S*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5,8,11,14-tetraazahexadecan-16-yl)-6-(2,5-dioxo-2,5-dihydro-1*H*-pyrrol-1-yl)hexanamide **9-B**

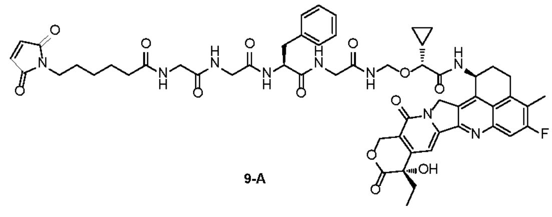

9-A

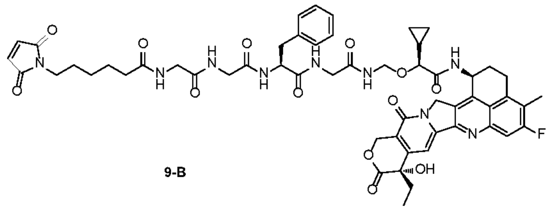

9-B

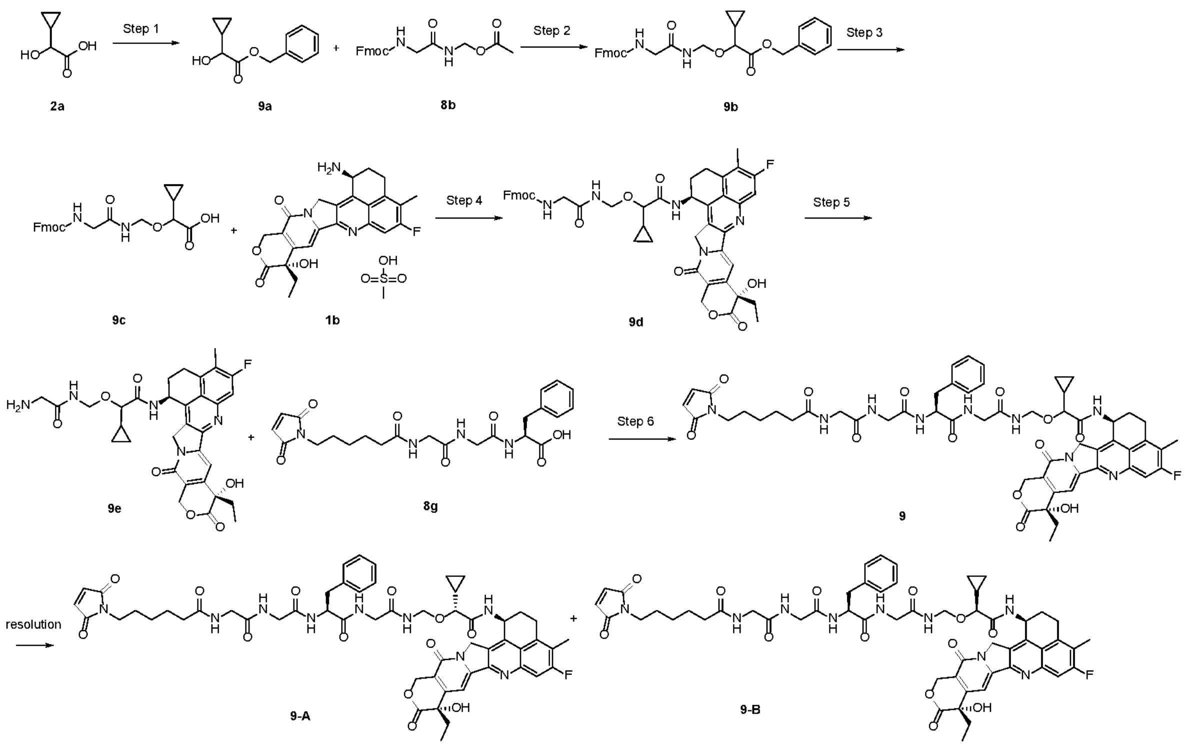


2a 9a 8b 9b 9c 1b 9d 9e 8g 9 9-A 9-B

Step 1

Benzyl 2-cyclopropyl-2-hydroxyacetate **9a**

**2a** (1.3 g, 11.2 mmol; prepared as disclosed in Patent Application "WO2013/106717") was dissolved in 50 mL of acetonitrile, and potassium carbonate (6.18 g, 44.8 mmol), benzyl bromide (1.33 mL, 11.2 mmol) and tetrabutylammonium iodide (413 mg, 1.1 mmol) were successively added. The reaction mixture was stirred at room temperature for 48 h and filtered through celite, and the filter cake was rinsed with ethyl acetate (10 mL). The filtrates were combined and concentrated under reduced pressure, and the resulting residue was purified by silica gel column chromatography with developing solvent system C to give the title product **9a** (2 g, 86.9% yield).

Step 2

Benzyl 10-cyclopropyl-1-(9*H*-fluoren-9-yl)-3,6-dioxo-2,9-dioxa-4,7-diazaundecan-11-oate **9b**

**9a** (120.9 mg, 0.586 mmol) and **8b** (180 mg, 0.489 mmol) were added to a reaction flask, and 4 mL of tetrahydrofuran was added. The system was purged with argon three times, and the reaction mixture was cooled to 0-5 °C in an ice-water bath, followed by addition of potassium *tert*-butoxide (109 mg, 0.98 mmol). The ice bath was removed, and the reaction mixture was warmed to room temperature and stirred for 40 min, followed by addition of 10 mL of ice water and by extraction with ethyl acetate (20 mL × 2) and chloroform (10 mL × 5). The organic phases were combined and concentrated. The resulting residue was dissolved in 4 mL of dioxane, and 2 mL of water, sodium bicarbonate (49.2 mg, 0.586 mmol) and 9-fluorenylmethyl chloroformate (126 mg, 0.49 mmol) were added. The mixture was stirred at room temperature for 2 h. 20 mL of water was added, followed by extraction with ethyl acetate (10 mL × 3). The organic phase was washed with saturated sodium chloride solution (20 mL), dried over anhydrous sodium sulfate, and filtered. The filtrate was concentrated under reduced pressure. The resulting residue was purified by silica gel column chromatography with developing solvent system C to give the title product **9b** (48 mg, 19% yield).
MS m/z (ESI): 515.0 [M+1].

Step 3

10-Cyclopropyl-1-(9*H*-fluoren-9-yl)-3,6-dioxo-2,9-dioxa-4,7-diazaundecan-11-oic acid **9c**

**9b** (20 mg, 0.038 mmol) was dissolved in 4.5 mL of a solvent mixture of tetrahydrofuran and ethyl acetate (V:V = 2:1), and palladium on carbon (12 mg, 10% loading, dry basis) was added. The system was purged with hydrogen three times, and the reaction mixture was stirred at room temperature for 1 h. The reaction mixture was filtered through celite, and the filter cake was rinsed with ethyl acetate. The filtrate was concentrated to give the crude title product **9c** (13 mg), which was directly used in the next step without purification.
MS m/z (ESI): 424.9 [M+1].

Step 4

(9*H*-fluoren-9-yl)methyl(2-(((1-cyclopropyl-2-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-2-oxoethoxy)methyl)amino)-2-oxoethyl)carbamate **9d**

**1b** (10 mg, 18.8 μmol) was added to a reaction flask, and 1 mL of *N,N*-dimethylformamide was added. The system was purged with argon three times, and the mixture was cooled to 0-5 °C in an ice-water bath, followed by addition of a drop of triethylamine, crude **9c** (13 mg, 30.6 μmol), and 4-(4,6-dimethoxy-1,3,5-triazin-2-yl)-4-methylmorpholinium chloride (16.9 mg, 61.2 μmol). The reaction mixture was stirred in an ice bath for 40 min. 10 mL of water was added, followed by extraction with ethyl acetate (10 mL × 3). The organic phases were combined, washed with saturated sodium chloride solution (10 mL × 2), dried over anhydrous sodium sulfate, and filtered. The filtrate was concentrated under reduced pressure. The resulting residue was purified by thin layer chromatography with developing solvent system B to give the title product **9d** (19 mg, 73.6% yield).
MS m/z (ESI): 842.1 [M+1].

Step 5

2-((2-Aminoacetamido)methoxy)-2-cyclopropyl-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)acetamide **9e**

**9d** (19 mg, 22.6 μmol) was dissolved in 2 mL of dichloromethane, and 1 mL of diethylamine was added. The reaction mixture was stirred at room temperature for 2 h and concentrated under reduced pressure. 1 mL of toluene was added, followed by concentration under reduced pressure; the procedures were repeated twice. The residue was slurried with 3 mL of *n*-hexane and let stand. Then, the supernatant was removed, and the solid was kept. The solid residue was concentrated under reduced pressure and dried using an oil pump to give the crude title product **9e** (17 mg), which was directly used in the next step without purification.
MS m/z (ESI): 638.0 [M+18].

Step 6

*N*-((2*R*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5,8,11,14-tetraazahexadecan-16-yl)-6-(2,5-dioxo-2,5-dihydro-1*H*-pyrrol-1-yl)hexanamide **9-A**

*N*-((2*S*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5,8,11,14-tetraazahexadecan-16-yl)-6-(2,5-dioxo-2,5-dihydro-1*H*-pyrrol-1-yl)hexanamide **9-B**

Crude **9e** (13.9 mg, 22.4 μmol) was dissolved in 0.6 mL of *N,N*-dimethylformamide. The system was purged with argon three times, and the solution was cooled to 0-5 °C in an ice-water bath. A solution of **8g** (21.2 mg, 44.8 μmol) in 0.3 mL of *N,N*-dimethylformamide was added, followed by addition of 4-(4,6-dimethoxy-1,3,5-triazin-2-yl)-4-methylmorpholinium chloride (18.5 mg, 67.3 μmol). The reaction mixture was stirred in an ice bath for 10 min. Then, the ice bath was removed, and the reaction mixture was warmed to room temperature and stirred for 1 h to produce compound **9.** The reaction mixture was purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient elution, flow rate: 18 mL/min). The corresponding fractions were collected and concentrated under reduced pressure to give the title products (9-A: 2.4 mg, 9-B: 1.7 mg).
MS m/z (ESI): 1074.4 [M+1].

Single-configuration compound 9-A (shorter retention time):

UPLC analysis: retention time: 1.14 min; purity: 85% (chromatography column: ACQUITY UPLC BEHC18 1.7 μm 2.1 × 50 mm; mobile phase: A-water (5 mmol of $NH_4OAc$), B-acetonitrile).
$^1$H NMR (400 MHz, DMSO-*d*$_6$): δ 8.60 (t, 1H), 8.51-8.49 (d, 1H), 8.32-8.24 (m, 1H), 8.13-8.02 (m, 2H), 8.02-7.96 (m, 1H), 7.82-7.75 (m, 1H), 7.3(s, 1H), 7.26-7.15 (m, 4H), 6.99 (s, 1H), 6.55-6.48 (m, 1H), 5.65-5.54 (m, 1H), 5.41 (s, 2H), 5.35-5.15 (m, 3H), 4.74-4.62 (m, 1H), 4.54-4.40 (m, 2H), 3.76-3.64 (m, 4H), 3.62-3.48 (m, 2H), 3.20-3.07 (m, 2H), 3.04-2.94 (m, 1H), 2.80-2.62 (m, 1H), 2.45-2.30 (m, 3H), 2.25-2.15 (m, 2H), 2.15-2.04 (m, 2H), 1.93-1.78 (m, 2H), 1.52-1.39 (m, 3H), 1.34-1.12 (m, 5H), 0.87 (t, 3H), 0.64-0.38 (m, 4H).

Single-configuration compound 9-B (longer retention time):

UPLC analysis: retention time: 1.16 min; purity: 89% (chromatography column: ACQUITY UPLC BEHC18 1.7 μm 2.1 × 50 mm; mobile phase: A-water (5 mmol of $NH_4OAc$), B-acetonitrile).
$^1$H NMR (400 MHz, DMSO-*d*$_6$): δ 8.68-8.60 (m, 1H), 8.58-8.50 (m, 1H), 8.32-8.24 (m, 1H), 8.13-8.02 (m, 2H), 8.02-7.94 (m, 1H), 7.82-7.75 (m, 1H), 7.31 (s, 1H), 7.26-7.13 (m, 3H), 6.99 (s, 1H), 6.55-6.48 (m, 1H), 5.60-5.50 (m, 1H), 5.41 (s, 2H), 5.35-5.15 (m, 2H), 4.78-4.68 (m, 1H), 4.60-4.40 (m, 2H), 3.76-3.58 (m, 4H), 3.58-3.48 (m, 1H), 3.20-3.10 (m, 2H), 3.08-2.97 (m, 2H), 2.80-2.72 (m, 2H), 2.45-2.30 (m, 3H), 2.25-2.13 (m, 2H), 2.13-2.04 (m, 2H), 2.03-1.94 (m, 2H), 1.91-1.78 (m, 2H), 1.52-1.39 (m, 3H), 1.34-1.12 (m, 4H), 0.91-0.79 (m, 3H), 0.53-0.34 (m, 4H).

**3. Preparation of ADC**

**Example 3-1 ADC-1**

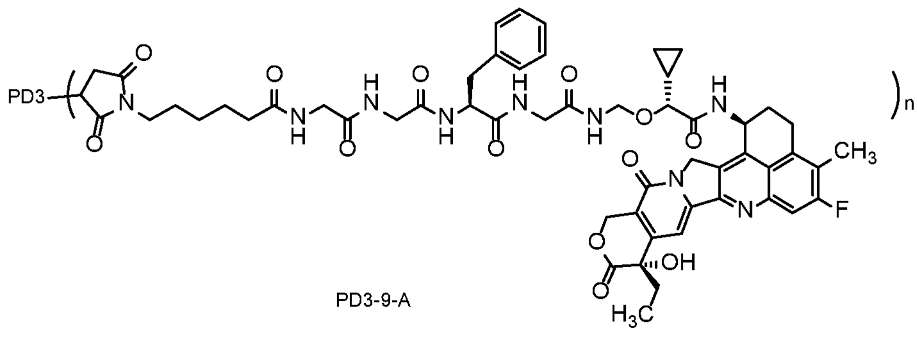

PD3-9-A

To an aqueous PBS buffer of antibody PD3 (0.05 M aqueous PBS buffer at pH 6.5; 10.0 mg/mL, 4.0 mL, 270 nmol) was added at 37 °C a prepared aqueous solution of tris(2-carboxyethyl)phosphine (TCEP) (10 mM, 67.5 μL, 675 nmol). The reaction mixture was shaken on a water bath shaker at 37 °C for 3 h before the reaction was terminated. The reaction mixture was cooled to 25 °C in a water bath.

Compound 9-A (2.9 mg, 2700 nmol) was dissolved in 180 μL of DMSO, and the resulting solution was added to the above reaction mixture, which was then shaken on a water bath shaker at 25 °C for 3 h before the reaction was terminated. The reaction mixture was desalted and purified through a Sephadex G25 gel column (elution phase: 0.05 *M* PBS buffer at pH 6.5, containing 0.001 M EDTA) to give exemplary product ADC-1 of PD3-9-A in PBS buffer (1.93 mg/mL, 18.4 mL), which was then stored at 4 °C.

Mean calculated by UV-Vis: n = 3.77.

**Example 3-2 ADC-2**

To an aqueous PBS buffer of antibody PD3 (0.05 M aqueous PBS buffer at pH 6.5; 10.0 mg/mL, 4.0 mL, 270 nmol) was added at 37 °C a prepared aqueous solution of tris(2-carboxyethyl)phosphine (TCEP) (10 mM, 143.1 μL, 1431 nmol). The reaction mixture was shaken on a water bath shaker at 37 °C for 3 h before the reaction was terminated. The reaction mixture was cooled to 25 °C in a water bath.

Compound 9-A (4.35 mg, 4050 nmol) was dissolved in 270 μL of DMSO, and the resulting solution was added to the above reaction mixture, which was then shaken on a water bath shaker at 25 °C for 3 h before the reaction was terminated. The reaction mixture was desalted and purified through a Sephadex G25 gel column (elution phase: 0.05 *M* PBS buffer at pH 6.5, containing 0.001 M EDTA) to give exemplary product **ADC-2** of PD3-9-A in PBS buffer (1.69 mg/mL, 17.8 mL), which was then stored at 4 °C. Mean calculated by UV-Vis: n = 6.59.

**Example 3-3 ADC-3**

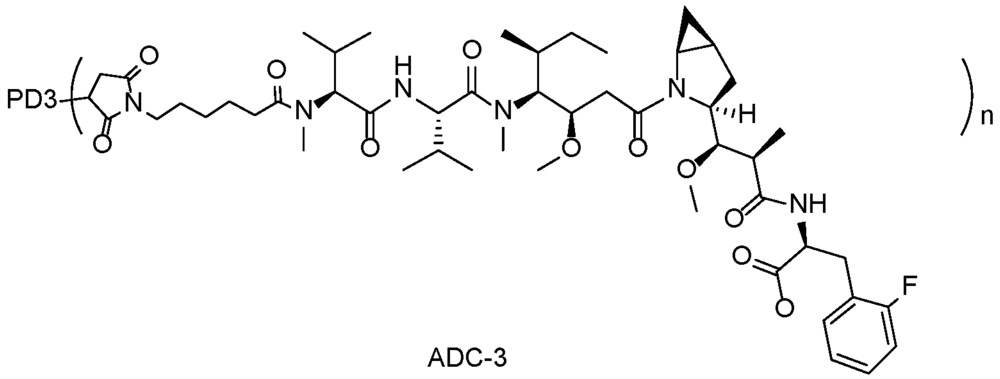

ADC-3

To an aqueous PBS buffer of antibody PD3 (0.05 M aqueous PBS buffer at pH 6.5; 10.0 mg/mL, 0.3 mL, 20.3 nmol) was added at 37 °C a prepared aqueous solution of tris(2-carboxyethyl)phosphine (TCEP) (10 mM, 5.1 μL, 51 nmol). The reaction mixture was shaken on a water bath shaker at 37 °C for 3 h before the reaction was terminated. The reaction mixture was cooled to 25 °C in a water bath.

Compound 12h (0.194 mg, 203 nmol, prepared with reference to WO2017063509) was dissolved in 20 μL of acetonitrile, and the resulting solution was added to the above reaction mixture, which was then shaken on a water bath shaker at 25 °C for 3 h before the reaction was terminated. The reaction mixture was desalted and purified through a Sephadex G25 gel column (elution phase: 0.05 MPBS buffer at pH 6.5, containing 0.001 M EDTA) to give the title product **ADC-3** in PBS buffer (4.3 mg/mL, 0.6 mL), which was then stored at 4 °C.

Mean calculated by UV-Vis: n = 4.14.

**Example 3-4 ADC-4**

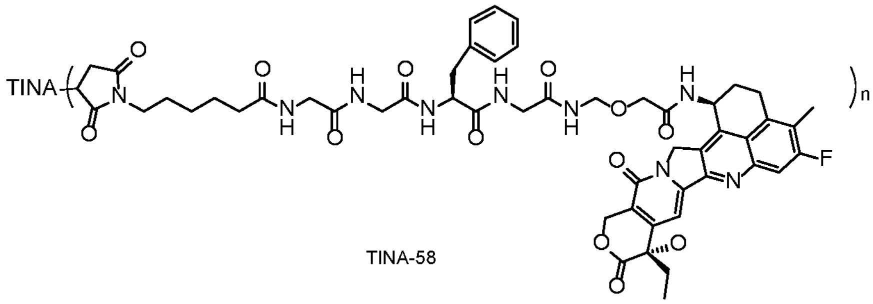

TINA-58

The synthesis was made with reference to Example 19 in WO2015098099.

To an aqueous PBS buffer of antibody TINA (0.05 M aqueous PBS buffer at pH 6.5; 10.0 mg/mL, 2.0 mL, 135.4 nmol) was added at 37 °C a prepared aqueous solution of tris(2-carboxyethyl)phosphine (TCEP) (10 mM, 33.8 μL, 338 nmol). The reaction mixture was shaken on a water bath shaker at 37 °C for 3 h before the reaction was terminated. The reaction mixture was cooled to 25 °C in a water bath.

Compound 58 (1.4 mg, 1354 nmol) was dissolved in 70 μL of DMSO, and the resulting solution was added to the above reaction mixture, which was then shaken on a water bath shaker at 25 °C for 3 h before the reaction was terminated. The reaction mixture was desalted and purified through a Sephadex G25 gel column (elution phase: 0.05 *M* PBS buffer at pH 6.5, containing 0.001 M EDTA) to give exemplary title product **ADC-4** of TINA-58 in PBS buffer (1.13 mg/mL, 15.1 mL), which was then stored at 4 °C.

Mean calculated by UV-Vis: n = 3.99.

**Example 3-5 ADC-5**

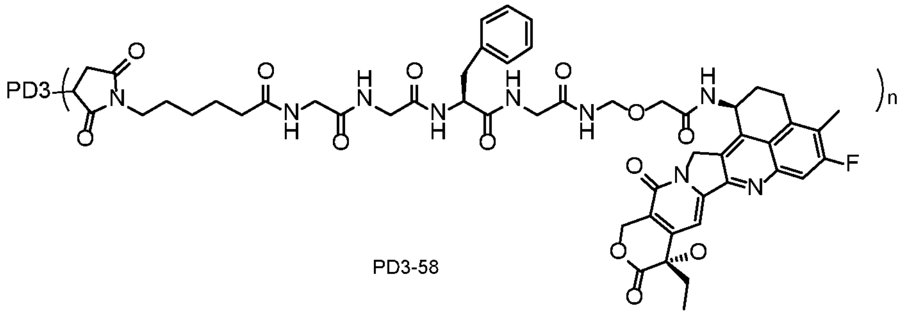

PD3-58

To an aqueous PBS buffer of antibody **PD3** (0.05 M aqueous PBS buffer at pH 6.5; 10.0 mg/mL, 1.5 mL, 101.4 nmol) was added at 37 °C a prepared aqueous solution of tris(2-carboxyethyl)phosphine (TCEP) (10 mM, 25.3 μL, 253 nmol). The reaction mixture was shaken on a water bath shaker at 37 °C for 3 h before the reaction was terminated. The reaction mixture was cooled to 25 °C in a water bath.

Compound 58 (1.05 mg, 1014 nmol, referred to Example 58 on page 163 of Patent CN104755494A) was dissolved in 60 μL of DMSO, and the resulting solution was added to the above reaction mixture, which was then shaken on a water bath shaker at 25 °C for 3 h before the reaction was terminated. The reaction mixture was desalted and purified through a Sephadex G25 gel column (elution phase: 0.05 MPBS buffer at pH 6.5) to give exemplary title product **ADC-5** of PD3-58 in PBS buffer (0.82 mg/mL, 13.5 mL), which was then stored at 4 °C.

Mean calculated by UV-Vis: n = 3.88.

**Example 3-6 ADC-6**

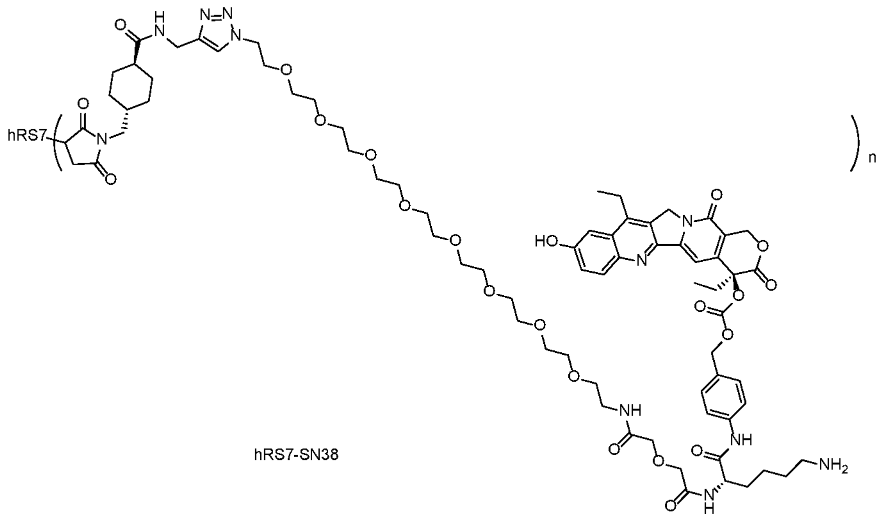

hRS7-SN38

To an aqueous PBS buffer of antibody **hRS7** (0.05 M aqueous PBS buffer at pH 6.5; 10.0 mg/mL, 1.4 mL, 94.60 nmol) was added at 37 °C a prepared aqueous solution of tris(2-carboxyethyl)phosphine (TCEP) (10 mM, 50.1 μL, 501 nmol). The reaction mixture was shaken on a water bath shaker at 37 °C for 3 h before the reaction was terminated. The reaction mixture was cooled to 25 °C in a water bath.

Compound **SN38** (synthesized with reference to Example 1 on Page 59 of Patent CN105407891A, 2.1 mg, 1419 nmol) was dissolved in 50 μL of DMSO, and the resulting solution was added to the above reaction mixture, which was then shaken on a water bath shaker at 25 °C for 3 h before the reaction was terminated. The reaction mixture was desalted and purified through a Sephadex G25 gel column (elution phase: 0.05 *M* PBS buffer at pH 6.5) to give exemplary title product **ADC-6** of **hRS7**-SN38 in PBS buffer (1.03 mg/mL, 11.5 mL), which was then stored at 4 °C.

Mean calculated by CE-SDS: n = 7.56.

### Example 3-7 ADC-7

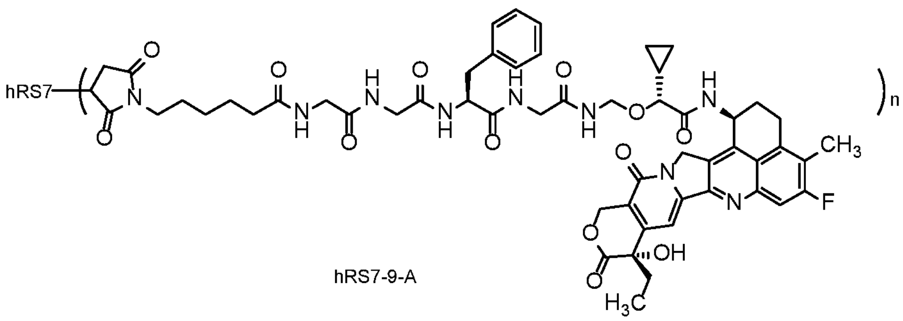

hRS7-9-A

To an aqueous PBS buffer of antibody **hRS7** (0.05 M aqueous PBS buffer at pH 6.5; 10.0 mg/mL, 2.18 mL, 147.3 nmol) was added at 37 °C a prepared aqueous solution of tris(2-carboxyethyl)phosphine (TCEP) (10 mM, 36.8 μL, 368 nmol). The reaction mixture was shaken on a water bath shaker at 37 °C for 3 h before the reaction was terminated. The reaction mixture was cooled to 25 °C in a water bath.

Compound **9-A** (1.58 mg, 1471 nmol) was dissolved in 100 μL of DMSO, and the resulting solution was added to the above reaction mixture, which was then shaken on a water bath shaker at 25 °C for 3 h before the reaction was terminated. The reaction mixture was desalted and purified through a Sephadex G25 gel column (elution phase: 0.05 *M* PBS buffer at pH 6.5) to give exemplary title product **ADC-7** of **hRS7**-9-A in PBS buffer (1.10 mg/mL, 16.4 mL), which was then stored at 4 °C.

Mean calculated by UV-Vis: n = 3.72.

**Example 3-8 ADC-8**

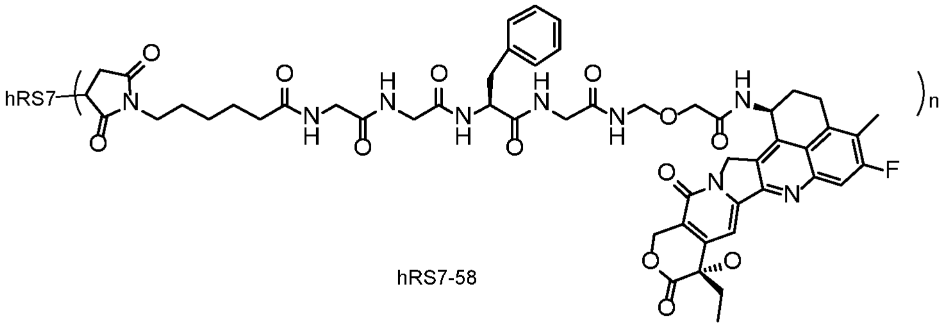

hRS7-58

To an aqueous PBS buffer of antibody **hRS7** (0.05 M aqueous PBS buffer at pH 6.5; 10.0 mg/mL, 2.18 mL, 147.3 nmol) was added at 37 °C a prepared aqueous solution of tris(2-carboxyethyl)phosphine (TCEP) (10 mM, 36.8 μL, 368 nmol). The reaction mixture was shaken on a water bath shaker at 37 °C for 3 h before the reaction was terminated. The reaction mixture was cooled to 25 °C in a water bath.

Compound 58 (1.52 mg, 1473 nmol, referred to Example 58 on page 163 of Patent CN104755494A) was dissolved in 100 μL of DMSO, and the resulting solution was added to the above reaction mixture, which was then shaken on a water bath shaker at 25 °C for 3 h before the reaction was terminated. The reaction mixture was desalted and purified through a Sephadex G25 gel column (elution phase: 0.05 *M* PBS buffer at pH 6.5) to give exemplary title product **ADC-8** of **hRS7**-58 in PBS buffer (1.02 mg/mL, 16.8 mL), which was then stored at 4 °C.

Mean calculated by UV-Vis: n = 3.93.

**Example 3-9 ADC-9**

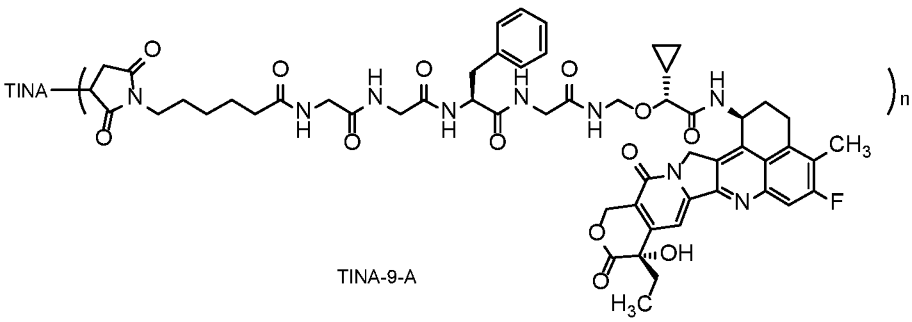

TINA-9-A

To an aqueous PBS buffer of antibody **TINA** (0.05 M aqueous PBS buffer at pH 6.5; 10.0 mg/mL, 0.95 mL, 64.2 nmol) was added at 37 °C a prepared aqueous solution of tris(2-carboxyethyl)phosphine (TCEP) (10 mM, 16.0 μL, 160 nmol). The reaction mixture was shaken on a water bath shaker at 37 °C for 3 h before the reaction was terminated. The reaction mixture was cooled to 25 °C in a water bath.

Compound **9-A** (0.69 mg, 642 nmol) was dissolved in 30 μL of DMSO, and the resulting solution was added to the above reaction mixture, which was then shaken on a water bath shaker at 25 °C for 3 h before the reaction was terminated. The reaction mixture was desalted and purified through a Sephadex G25 gel column (elution phase: 0.05 *M* PBS buffer at pH 6.5) to give exemplary title product **ADC-9** of TINA-9-A in PBS buffer (0.99 mg/mL, 7.0 mL), which was then stored at 4 °C.

Mean calculated by UV-Vis: n = 3.99.

Drug-loading analysis of ADC stock solution

An ADC is an antibody cross-linked drug, and the mechanism of treating diseases thereof is to transport toxin molecules into cells depending on the targeting performance of the antibody so as to kill the cells. The drug loading

plays a decisive role in the drug efficacy.

1. UV-Vis calculation method

The drug loading of the ADC stock solution was determined using the UV method.

**Experimental procedures**

Cuvettes containing sodium succinate buffer were placed into the reference cell and sample cell, and the absorbance of the solvent blank was subtracted. Then, a cuvette containing test solution was placed into the sample cell, and the absorbances at 280 nm and 370 nm were determined.

**Calculation for results**

The loading capacity of the ADC stock solution was determined by ultraviolet spectrophotometry (instrument: a Thermo nanodrop2000 ultraviolet spectrophotometer), based on the principle that the total absorbance of the ADC stock solution at a certain wavelength was the sum of the absorbance of the cytotoxic drug and the monoclonal antibody at certain wavelength, namely:

$$(1)\ A_{280\ nm} = \varepsilon_{mab\text{-}280} b C_{mab} + \varepsilon_{Drug\text{-}280} b C_{Drug}$$

$\varepsilon_{Drug\text{-}280}$: the mean molar extinction coefficient of the drug at 280 nm was 5100;
$C_{Drug}$: the concentration of the drug;
$\varepsilon_{mab\text{-}280}$: the mean molar extinction coefficient of the monoclonal antibody stock solution at 280 nm is 214,600;
$C_{mab}$: the concentration of the monoclonal antibody stock solution;
b: the optical path length is 1 cm.

Similarly, an equation for the total absorbance of the sample at 370 nm can be given as:

$$(2)\ A_{370\ nm} = \varepsilon_{mab\text{-}370} b C_{mab} + \varepsilon_{Drug\text{-}370} b C_{Drug}$$

$\varepsilon_{Drug\text{-}370}$: the mean molar extinction coefficient of the drug at 370 nm was 19,000;
$C_{Drug}$: the concentration of the drug;
$\varepsilon_{mab\text{-}370}$: the extinction coefficient of the monoclonal antibody stock solution at 370 nm is 0;
$C_{mab}$: the concentration of the monoclonal antibody stock solution;
b: the optical path length is 1 cm.

The drug loading can be calculated using both equations (1) and (2) as well as the attenuation coefficients of the monoclonal antibody and the drug at both wavelengths and their concentrations.

Drug loading = $C_{Drug}/C_{mab}$.

2. CE-SDS calculation method

**Reagents and instruments**

The adopted was SDS-Mw Analysis Kit produced by Beckman, Cat. # 390953, and containing SDS-MW gel separation buffer, SDS-MW sample buffer, acidic cleaning solution (0.1 mol/L hydrochloric acid solution), basic cleaning solution (0.1mol sodium hydroxide solution) and internal standard substance (10 kDa). The adopted was also the SDS kit produced by Beijing BioCEart Technology Institute, Cat. # BSYK018, and containing CE-SDS gel buffer and CE-SDS sample buffer.

Alkylation solution (0.25 mo iodoacetamide solution): about 0.046 g of iodoacetamide was weighed, 1 mL of ultrapure water was added to dissolve and mix well, and the resulting solution was stored at 2-8 °C for 7 days in dark.

Capillary electrophoresis apparatus: PA800plus from SCIEX.

Capillary: uncoated fused-silica capillary (with an internal diameter of 50 μm), cut to a total length of 30.2 cm and an effective separation length by a high-resolution method of 20 cm.

**Preparation of test sample solution**

The test sample was diluted to 1 mg/mL with SDS sample buffer. 95 μL of sample solution (1 mg/mL) was taken and added with 5 μL of iodoacetamide aquerous solution (0.8 mol/L), and the resulting solution was vortexed and mixed well. 95 μL of blank control was taken and added with 5 μL of 0.8 mol/L iodoacetamide aqueous solution, and the resulting solution was vortexed and mixed well. 75 μL of samples were taken from sample tubes and added into the sample vials, and subjected to analysis immediately.

**Determination method**

1) Pretreatment of capillary: 0.1 mol/L sodium hydroxide solution was washed under 60 psi pressure for 3 min, then washed with 0.1 mol/L hydrochloric acid solution under 60 psi pressure for 2 min, and finally washed with pure water under 70 psi pressure for 1 min. The above pretreatment should be performed before each operation.
2) Pre-filling of capillary: SDS gel separation buffer was washed under 50 psi pressure for 15 min. The above pretreatment should be performed before each operation. Sample injection: electrokinetic sample injection was performed at 10 kV of reversed polarity, and the reduced sample was injected for 20 s.

Separation: separation was performed at 15kV of reversed polarity for 40 min.
Temperature of sample chamber: 18 °C to 25 °C.
Temperature of capillary: 18 °C to 25 °C.

**Analysis of results**

Data were analyzed by using software from Beckman based on the coupling of corresponding drug on sulfydryl liberated from an opened disulfide bond in the antibody, and the proportion of the corrected peak area such as a heavy chain, a non-glycosylated heavy chain and a light chain in the sum of all corrected peak areas was calculated. Calculation formula: DAR = [4 × heavy chain (H) peak area + 2 × half antibody (H-L) peak area + 4 × double heavy chain (H-H) peak area + 2 × heavy-heavy-light chain (H-H-L) peak area]/[heavy chain (H) peak area/2 + half antibody (H-L) peak area/2 + double heavy chain (H-H) peak area + heavy-heavy-light chain (H-H-L) peak area + full antibody peak area], and the weighted average of the ADC was finally calculated.

**The activity of the antibodies of the present disclosure was validated by using a biochemical test method.**

**Test Example 1: Antibody Protein Level Binding Assay**

hTROP-2 protein was diluted to 1 μg/mL with PBS buffer at pH 7.4 (Shanghai BasalMedia Technologies Co., LTD., B320), added into a 96-well microplate at 100 μL per well, and incubated at 4 °C overnight. After the liquid was discarded, 300 μL of 5% skim milk (BD, 232100) diluted with PBS was added into each well for blocking and incubated at 37 °C for 2 h. After the blocking was completed, the blocking solution was discarded; after the plate was washed 3 times with PBST buffer (pH 7.4, PBS containing 0.1% tween-20), 100 μL of a gradient diluted antibody solution was added into each well and incubated at 37 °C for 1 h. After the incubation was completed, the plate was washed 3 times with PBST buffer, and 100 μL of 1:8000 diluted mouse anti-human IgG (H+L) (Jackson ImmunoResearch, 209-035-088) was added into each well and incubated at 37 °C for 1 h. After the plate was washed 3 times with PBST buffer, 100 μL of TMB chromogenic substrate (KPL, 5120-0077) was added into each well and incubated at room temperature for 10-15 min, and the reaction was terminated by adding 50 μL of 1 M $H_2SO_4$ into each well. The plate was detected for the absorbance at 450 nm using a microplate reader, the binding curves of antibodies to antigen were fitted with software, and the $EC_{50}$ value was calculated. The binding activity of the antibodies to the proteins is shown in Table 2.

Table 2. Antibody protein level binding activity

| Antibodies | PD3 | hRS7 | TINA |
|---|---|---|---|
| $E_{max}$ (OD value) | 1.40 | 1.37 | 1.39 |
| $EC_{50}$ (nM) | 13.6 | 18.29 | 16.14 |

The result shows that the PD3 antibody in the present application has higher binding activity to hTROP-2 protein.

**Test Example 2: Antibody Cell Level Binding Assay**

The stably transfected TROP-2-expressing CHOK1 cells were suspended in FACS buffer (2% fetal bovine serum (Gibco, 10099141) pH 7.4 PBS (Sigma, P4417-100TAB)) to give a 1 × $10^6$/mL cell suspension, which was then added to a 96-well round-bottom plate at 100 μL/well. After centrifugation and removal of the supernatant, the test antibodies that was diluted with FACS buffer to different concentrations were added at 50 μL/well. The plate was incubated in the dark in a 4 °C refrigerator for 1 h. The plate was washed 3 times with FACS buffer by centrifugation at 300 g, and Alexa Fluor 488 goat anti-human IgG (H+L) (invitrogen, A-11013) at working concentration was added. The plate was incubated in the dark in a 4 °C refrigerator for 40 min. The plate was washed 3 times with FACS buffer by centrifugation at 300 g and tested on a BD FACSCantoII flow cytometer for geometric mean fluorescence intensity (MFI). The binding $EC_{50}$ value of the antibody to the stably transfected TROP-2-expressing cells was calculated. The binding activity of the antibodies to the cells is shown in FIG. 1 and Table 3.

Table 3. Antibody cell level binding activity

| Antibodies | PD3 | hRS7 | TINA |
|---|---|---|---|
| $E_{max}$ (MFI) | 16693 | 16050 | 14217 |
| $EC_{50}$ (nM) | 1.57 | 2.31 | 3.55 |

The result shows that the PD3 antibody in the present application has higher binding activity to hTROP-2 protein-expressing cells.

**Test Example 3: Antibody Endocytosis Assay**

The purpose of the assay is that the activated DT kills cells after the DT3C protein enters the cells, indirectly reflecting endocytosis of the anti-TROP-2 antibody. The *in vitro* endocytic activity of the antibody is evaluated according to $EC_{50}$ and $E_{max}$.

DT3C is a recombinantly expressed fusion protein and is formed by fusing fragment A of diphtheria toxin (toxin part only) and fragment 3C of group G streptococcus (IgG binding part). The protein can have a high affinity for IgG part of antibody, enter cells together with the IgG part when the antibody is endocytosed, and release toxic DT under the action of intracellular furin protease. The DT can inhibit the activity of EF2-ADP ribosylation, block the protein translation process and finally cause cell death. DT3C that does not enter the cell has no activity of cell killing. The endocytic activity of the antibody is evaluated based on cell killing.

Cell suspensions were prepared with fresh cell medium containing 20% low IgG FBS at a cell density of 2 × $10^4$ cells/mL, and added into the cell culture plates at 50 μL/well and incubated with 5% carbon dioxide at 37 °C for 16 h. DT3C at 4× concentration was formulated in serum-free medium and filtered through a 0.22 μm filter to obtain a sterile solution. Antibody at 4× concentration was prepared in serum-free medium, and 80 μL of DT3C and 80 μL of antibody were mixed at a volume of 1:1, and incubated at room temperature for 30 min. 50 μL of the diluted antibody-DT 3C mixture was added to 50 μL of cells and incubated in an incubator for three days. 50 μL of CTG was added into each well and incubated for 10 min at room temperature in dark, and the chemiluminescence values were detected using Victor3. The endocytic activity of the antibody is shown in Table 4.

Table 4. Antibody endocytic activity

| Antibodies | PD3 | hRS7 | TINA |
|---|---|---|---|
| Emax | 99.1% | 98.5% | 98.7% |
| $EC_{50}$ (nM) | 0.15 | 0.18 | 0.18 |

The results show that the PD3 antibody in the present application has higher endocytosis efficiency.

**Test Example 4: Affinity Assay of Antibody**

The affinity of antibody for TROP-2 was detected in a form of a capture antibody. The antibody was affinity-captured by a Protein A (Cat. # 29127556, GE) biosensor chip conjugated with an anti-human IgG antibody (Cat. # BR-1008-39, Lot. # 10260416, GE), then an antigen hTROP-2 flowed on the surface of the chip, and a Biacore T200 instrument was used for detecting reaction signals in real time to obtain association and dissociation curves. After

dissociation was completed for each assay cycle, the chip was washed clean and regenerated with regeneration buffer, Glycine1.5 (Cat. # BR100354, GE) or 3 M $MgCl_2$ (from Human antibody capture kit, Cat. # BR100839, GE). After the assay was completed, the data were fitted with (1:1) Langmuir model using GE Biacore T200 Evaluation version 3.0 to obtain affinity values. The affinity of the antibody for the proteins is shown in Table 5.

Table 5. Affinity of antibody determined by Biacore

| Antibodies | PD3 | hRS7 | TINA |
|---|---|---|---|
| KD (M) | 6.86E-10 | 9.87E-10 | 2.15E-8 |

The results show that the PD3 antibody in the present application has a higher affinity for hTROP-2.

**Test Example 5: Cell Activity Assay of ADC Molecules**

The cells used in this assay were as follows: FaDu (+++) purchased from ATCC, Cat. # HTB-43™; HCC827(+++), purchased from ATCC, Cat. # CRL-2868; Colo205(++), purchased from Cell Bank, Chinese Academy of Sciences, Cat. # TCHu102; DMS53 (++), purchased from ATCC, Cat. # CRL-2062™; SK-OV-3(+), purchased from ATCC, Cat. # HTB-77; CHO-K1(-), purchased from ATCC, Cat. # CCL-61™; wherein "+" represents the expression amount of TROP-2 in the cell population, more "+" means that the expression amount of TROP-2 is higher, and "-" means that TROP-2 is not expressed. Cell suspensions were prepared with fresh cell medium containing 10% FBS at a density of 3703 cells/mL, and added into the 96-well cell culture plate at 135 μL/well and incubated with 5% carbon dioxide at 37 °C for 16 h. ADC samples were formulated at 5 μM with PBS. The 5 μM of initial concentration was diluted five times with PBS for a total of 8 concentrations. 15 μL of the above ADC solution was added into each well. The plate was cultured at 37 °C in 5% carbon dioxide for 6 days. 70 μL of CTG was added into each well and incubated for 10 min at room temperature in dark, the chemiluminescence values were detected using Victor3, and data analysis was performed using the GraphPad Prism software.

The result shows that ADC-1 has stronger cell killing effect, and the killing effect is positively correlated with the TROP-2 expression level on the surface of the tumor cell.

Table 6-1. Killing activity of ADC on cells with different TROP-2 expression levels

| | FaDu | | HCC827 | | Colo205 | | DMS53 | | SK-OV-3 | | CHO-K1 | |
|---|---|---|---|---|---|---|---|---|---|---|---|---|
| **Samples** | **$EC_{50}$ (nM)** | **Emax (%)** | **$EC_{50}$ (nM)** | **Emax (%)** | **$EC_{50}$ (nM)** | **Emax (%)** | **$EC_{50}$ (nM)** | **Emax (%)** | **$EC_{50}$ (nM)** | **$E_{max}$ (%)** | **$EC_{50}$ (nM)** | **$E_{max}$ (%)** |
| ADC-1 | 0.23 | 100.42 | 0.14 | 89.64 | 39.23 | 99.38 | 42.46 | 89.39 | NA | 61.91 | NA | 9.31 |
| ADC-4 | 0.53 | 101.25 | 0.27 | 88.16 | 59.8 | 99.66 | 75.58 | 89.83 | NA | 49.15 | NA | 1.71 |
| ADC-7 | 0.31 | 100.75 | 0.18 | 89.33 | 52.75 | 99.52 | 72.92 | 88.74 | NA | 66.11 | NA | 8.69 |

A second parallel comparison assay was performed in the method as described above, and the obtained results are as follows:

Table 6-2. Killing activity of ADC on cells with different TROP-2 expression levels

| | FaDu | | HCC827 | | Colo205 | | DMS53 | |
|---|---|---|---|---|---|---|---|---|
| Samples | $EC_{50}$(nM) | $E_{max}$(%) | $EC_{50}$(nM) | $E_{max}$(%) | $EC_{50}$(nM) | $E_{max}$(%) | $EC_{50}$(nM) | $E_{max}$(%) |
| ADC-1 | 0.08 | 101.6 | 0.06 | 89.57 | 12.07 | 100.34 | 15.08 | 93.24 |
| ADC-5 | 0.14 | 101.58 | 0.13 | 88.23 | 25.63 | 99.64 | 25.71 | 92.47 |

**Test Example 6: Bystander Killing Activity Study**

BxPC3 (human pancreatic cancer cells, ATCC, CRL-1687) and MiaPaCa2 cells (human pancreatic cancer cells, biocytogen, B-HCL-014) were cultured with RPMI1640+10% FBS and DMEM/high glucose+10% FBS, respectively; cells were trypsinized, neutralized with fresh medium, and centrifuged at 1000 rpm for 3 min; supernatant was discarded, and cells were resuspended with RPMI1640+10% FBS. After the cells were counted, the cell density of BxPC3 was adjusted to $6 \times 10^4$ cells/mL and the cell density of MiaPaCa2-luc was adjusted to $1.5 \times 10^4$ cells/mL. 500 μL of BxPC3 cells and 500 μL of MiaPaCa2-luc cells were added into each well of 12-well plate 1. 500 μL of MiaPaCa2-luc cells and 500 μL of RPMI1640 medium containing 10% FBS serum were added into a 12-well plate 2. The plate was cultured at 37 °C in 5% carbon dioxide for 24 h.

The ADC samples were formulated into 40× concentration of intermediate solutions (0.2 μM). 25 μL of the above samples were taken and added into the corresponding well of the 12-well plate. Solvent control group was set. The plate was cultured at 37 °C in 5% carbon dioxide for 6 days. The cells in the 12-well plate were trypsinized, neutralized with fresh medium, and centrifuged at 1000 rpm for 3 min. The supernatant was discarded, and the cells were resuspended in 1 mL of FACS buffer (PBS + 2.5% FBS). 20 μL of the cells were taken and stained with 20 μL of trypan blue and counted. The cells in the plate 1 were centrifuged at 1000 rpm for 3 min, the supernatant was discarded, the cells were resuspended in 100 μL of FACS Buffer, 2 μL of TROP-2 (EGP-1) monoclonal antibody (MR54) was added, and the cells were incubated on ice for 30 min. The cells were centrifuged at 2000 rpm at 4 °C for 1 min, the supernatant was discarded, and the cells were resuspended in 150 μL of FACS buffer. Detection was performed using BD FACSVerse. Data was analyzed by Flowjo 7.6. The results of the bystander killing activity study are shown in FIG. 2.

The results show that ADC-1 in the present disclosure has a clear bystander killing effect, and ADCs do not kill the TROP-2 negative MiaPaCa2 cells, but ADC-1 also kills TROP-2 negative cells when the TROP-2 expressing BxPC3 cells are mixed with the negative cells MiaPaCa2.

Biological Evaluation ***of In Vivo* Activity**

**Test Example 7: *In Vivo* Efficacy Evaluation of Fadu Cell CDX Mouse Model**

Fadu cells ($3 \times 10^6$) were subcutaneously inoculated in the right flank of Balb/c nude mice, after 10 days of inoculation, the nude mice with heavy weight, too big tumor and too small tumor were removed after the tumor volume was about 245 $mm^3$, and the remaining mice were randomized into 5 groups of 8 mice according to tumor volume. Each mouse was intraperitoneally injected with an ADC at 0.1 mL/10 g body weight at days 0 and 8, making a total of 2 injections with a dose of 1 mg/kg. The tumor volumes and body weights were measured twice a week and the results were recorded for 21 days. Data were recorded using Excel statistical software: the mean values were calculated as avg; the SD values were calculated as STDEV; the SEM values were calculated as STDEV/SQRT (number of animals per group); GraphPad Prism software was used for plot, and statistical analysis of the data was performed using Two-way ANOVA or One-way ANOVA.

Tumor volume (V) was calculated as: $V = 1/2 \times L_{long} \times L_{short}^2$

Relative tumor proliferation rate T/C(%) = (T - To)/(C - Co) × 100, wherein T and C are the tumor volumes of animals at the end of the experiment in the treatment group and control group, respectively; To and Co are the tumor volumes of animals at the beginning of the experiment in the treatment group and control group, respectively.

Tumor inhibition rate TGI (%) = 1 - T/C (%).

The results are shown in Table 7 and FIG. 3, which indicate that ADC-1 has a strong tumor-inhibiting effect on FaDu xenograft tumors at a dose of 1 mpk.

Table 7. Efficacy of ADC on FaDu xenograft tumors of tumor-bearing nude mice

| Group | Mean tumor volume ($mm^3$) | | Mean tumor volume ($mm^3$) | | Tumor inhibition rate TGI (%) |
|---|---|---|---|---|---|
| | D0 | SEM | D21 | SEM | |
| Blank control | 246.8 | 29.04 | 2245.04 | 275.14 | N/A |
| ADC-4 1mg/kg | 244.41 | 29.62 | 1180.96 | 193.55 | 53 |
| ADC-1 1mg/kg | 251.19 | 23.45 | 238.54 | 107.62 | 101 |
| ADC-2 1mg/kg | 270.48 | 21.36 | 0 | 0 | 114 |
| PD3 30mg/kg | 246.02 | 25 | 2407.37 | 207.71 | -8 |

**Test Example 8: *In Vivo* Efficacy Evaluation of SKOV3 Cell CDX Mouse Model**

SKOV3 cells ($5 \times 10^6$) were subcutaneously inoculated in the right flank of Balb/c nude mice, after 23 days of inoculation, the nude mice with heavy weight, too big tumor and too small tumor were removed after the tumor volume was about 180 $mm^3$, and the remaining mice were randomized into 5 groups of 8 mice according to tumor volume. Each mouse was intraperitoneally injected with an ADC at 0.1 mL/10 g body weight for a total of 2 injections, and the dose is shown in following table. The tumor volumes and body weights were measured twice a week and the results were recorded. Data were recorded using Excel statistical software: the mean values were calculated as avg; the SD values were calculated as STDEV; the SEM values were calculated as STDEV/SQRT (number of animals per group); GraphPad Prism software was used for plot, and statistical analysis of the data was performed using Two-way ANOVA or One-way ANOVA.
Tumor volume (V) was calculated as:

$$V = 1/2 \times L_{long} \times L_{short}^2$$

Relative tumor proliferation rate T/C(%) = (T - To)/(C - Co) $\times$ 100, wherein T and C are the tumor volumes of animals at the end of the experiment in the treatment group and control group, respectively; To and Co are the tumor volumes of animals at the beginning of the experiment in the treatment group and control group, respectively.

$$\text{Tumor inhibition rate TGI (\%)} = 1 - \text{T/C (\%)}.$$

The results are shown in Table 8 and FIG. 4, which indicate that ADC-1 has a strong tumor-inhibiting effect on SKOV3 xenograft tumors at different doses, and the tumor-inhibiting effect is dose-dependent.
The effect is dose-dependent.

Table 8. Efficacy of ADC on SKOV3 xenograft tumors of tumor-bearing nude mice

| Group | Mean tumor volume ($mm^3$) | | Mean tumor volume ($mm^3$) | | Tumor inhibition rate TGI (%) |
|---|---|---|---|---|---|
| | D0 | SEM | D21 | SEM | |
| Blank control | 183.26 | 7.6 | 1081.36 | 132.88 | N/A |
| ADC-110mg/kg | 185.48 | 9.62 | 302.93 | 39.34 | 87 |
| ADC-1 3mg/kg | 185.09 | 10.07 | 519.9 | 38.93 | 63 |
| ADC-1 1mg/kg | 183.29 | 8.87 | 795.34 | 124.81 | 32 |
| ADC-4 10mg/kg | 183.83 | 8.9 | 938.99 | 148.98 | 16 |
| ADC-4 3mg/kg | 184.17 | 8.2 | 873.38 | 50.84 | 23 |
| ADC-4 1mg/kg | 185.17 | 8.38 | 1110.52 | 159.73 | -3 |
| PD3 30mg/kg | 182.85 | 8.38 | 1384.28 | 93.87 | -34 |

**Test Example 9: *In Vivo* Efficacy Evaluation of Colo205 Cell CDX Mouse Model**

Colo205 cells ($5 \times 10^6$) were subcutaneously inoculated in the right flank of Balb/c nude mice, after 10 days of inoculation, the nude mice with heavy weight, too big tumor and too small tumor were removed after the tumor volume was about 245 $mm^3$, and the remaining mice were randomized into 6 groups of 8 mice according to tumor volume.

Each mouse was intraperitoneally injected with an ADC at 0.1 mL/10 g body weight at day 0 (D0) and day 10, making a total of 2 injections with a dose of 10 mg/kg. The tumor volumes and body weights were measured twice a week and the results were recorded for 28 days (D28).

Data were recorded using Excel statistical software: the mean values were calculated as avg; the SD values were calculated as STDEV; the SEM values were calculated as STDEV/SQRT (number of animals per group); GraphPad Prism software was used for plot, and statistical analysis of the data was performed using Two-way ANOVA or One-way ANOVA.

Tumor volume (V) was calculated as:

$$V = 1/2 \times L_{long} \times L_{short}^2$$

Relative tumor proliferation rate T/C(%) = (T - To)/(C - Co) $\times$ 100, wherein T and C are the tumor volumes of animals at the end of the experiment in the treatment group and control group, respectively; To and Co are the tumor volumes of animals at the beginning of the experiment in the treatment group and control group, respectively.

$$\text{Tumor inhibition rate TGI (\%)} = 1 - \text{T/C (\%)}.$$

The results are shown in Table 9 and FIG. 5, which indicate that ADC-9 and ADC-7 conjugated with compound 9-A have a strong tumor-inhibiting effect on Colo205 xenograft tumors at a dose of 1 mpk.

Table 9. Efficacy of ADC on FaDu xenograft tumors of tumor-bearing nude mice

| Group | Mean tumor volume ($mm^3$) | | Mean tumor volume ($mm^3$) | | Tumor inhibition rate TGI (%) |
|---|---|---|---|---|---|
| | D0 | SEM | D28 | SEM | |
| Blank control | 210.30 | 24.91 | 1355.56 | 117.46 | N/A |
| ADC-6 10mg/kg | 194.93 | 26.56 | 1344.84 | 95.93 | 0 |
| ADC-7 10mg/kg | 194.64 | 21.03 | 35.13 | 12.80 | **113** |
| ADC-8 10mg/kg | 199.09 | 23.41 | 475.66 | 175.24 | 76 |
| ADC-9 10mg/kg | 206.70 | 21.21 | 99.86 | 32.55 | **109** |
| ADC-4 10mg/kg | 201.52 | 27.85 | 513.83 | 118.53 | 73 |

## Claims

1. A ligand-drug conjugate of general formula (Pc-L-Y-D) or a pharmaceutically acceptable salt thereof:

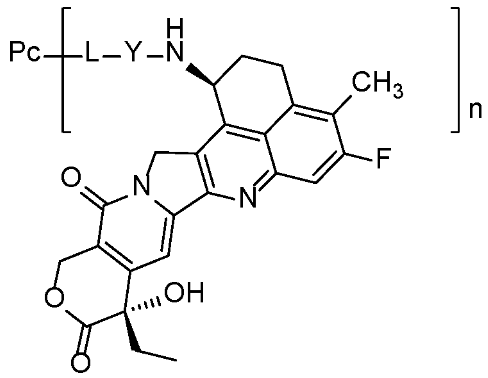

(Pc-L-Y-D)

wherein:

Y is selected from the group consisting of $-O-(CR^aR^b)_m-CR^1R^2-C(O)-$, $-O-CR^1R^2-(CR^aR^b)_m-$, $-O-CR^1R^2-$, $-NH-(CR^aR^b)_m-CR^1R^2-C(O)-$ and $-S-(CR^aR^b)_m-CR^1R^2-C(O)-$;
$R^a$ and $R^b$ are identical or different and are each independently selected from the group consisting of hydrogen, deuterium, halogen, alkyl, haloalkyl, deuterated alkyl, alkoxy, hydroxy, amino, cyano, nitro, hydroxyalkyl, cycloalkyl and heterocyclyl; or, $R^a$ and $R^b$, together with carbon atoms connected thereto, form cycloalkyl or heterocyclyl;
$R^1$ is selected from the group consisting of halogen, haloalkyl, deuterated alkyl, cycloalkyl, cycloalkylalkyl, alkoxyalkyl, heterocyclyl, aryl and heteroaryl; $R^2$ is selected from the group consisting of hydrogen, halogen, haloalkyl, deuterated alkyl, cycloalkyl, cycloalkylalkyl, alkoxyalkyl, heterocyclyl, aryl and heteroaryl; or, $R^1$ and $R^2$, together with carbon atoms connected thereto, form cycloalkyl or heterocyclyl;
or, $R^a$ and $R^2$, together with carbon atoms connected thereto, form cycloalkyl or heterocyclyl;
m is an integer from 0 to 4;
n is a decimal or an integer from 1 to 10;
L is a linker unit;
Pc is an anti-TROP-2 antibody or an antigen-binding fragment thereof.

**2.** The ligand-drug conjugate of general formula (Pc-L-Y-D) or the pharmaceutically acceptable salt thereof according to claim 1, wherein the anti-TROP-2 antibody or the antigen-binding fragment thereof comprises a heavy chain variable region and a light chain variable region, wherein the heavy chain variable region comprises an HCDR1, an HCDR2 and an HCDR3 having sequences identical to those of an HCDR1, an HCDR2 and an HCDR3 of a heavy chain variable region set forth in SEQ ID NO: 3, and the light chain variable region comprises an LCDR1, an LCDR2 and an LCDR3 having sequences identical to those of an LCDR1, an LCDR2 and an LCDR3 of a light chain variable region set forth in SEQ ID NO: 4.

**3.** The ligand-drug conjugate of general formula (Pc-L-Y-D) or the pharmaceutically acceptable salt thereof according to claim 1 or 2, wherein the anti-TROP-2 antibody or the antigen-binding fragment thereof comprises a heavy chain variable region and a light chain variable region, wherein the heavy chain variable region comprises an HCDR1, an HCDR2 and an HCDR3 set forth in SEQ ID NO: 5, SEQ ID NO: 6 and SEQ ID NO: 7, respectively, and the light chain variable region comprises an LCDR1, an LCDR2 and an LCDR3 set forth in SEQ ID NO: 8, SEQ ID NO: 9 and SEQ ID NO: 10, respectively.

**4.** The ligand-drug conjugate of general formula (Pc-L-Y-D) or the pharmaceutically acceptable salt thereof according to any one of claims 1 to 3, wherein the anti-TROP-2 antibody is a murine antibody, a chimeric antibody, a humanized antibody or a human antibody.

**5.** The ligand-drug conjugate of general formula (Pc-L-Y-D) or the pharmaceutically acceptable salt thereof according to any one of claims 1 to 4, wherein the anti-TROP-2 antibody or the antigen-binding fragment thereof comprises a heavy chain variable region and a light chain variable region, wherein the heavy chain variable region has an amino acid sequence set forth in SEQ ID NO: 3 or having at least 90% identity thereto, and/or the light chain variable region has an amino acid sequence set forth in SEQ ID NO: 4 or having at least 90% identity thereto.

**6.** The ligand-drug conjugate of general formula (Pc-L-Y-D) or the pharmaceutically acceptable salt thereof according to any one of claims 1 to 5, wherein the anti-TROP-2 antibody or the antigen-binding fragment thereof comprises a heavy chain variable region set forth in SEQ ID NO: 3 and a light chain variable region set forth in SEQ ID NO: 4.

**7.** The ligand-drug conjugate of general formula (Pc-L-Y-D) or the pharmaceutically acceptable salt thereof according to any one of claims 1 to 6, wherein the anti-TROP-2 antibody or the antigen-binding fragment thereof comprises a heavy chain constant region and a light chain constant region of the antibody; preferably, the heavy chain constant region is selected from the group consisting of human IgG1, IgG2, IgG3 and IgG4 constant regions, and the light chain constant region is selected from the group consisting of human antibody κ and λ chain constant regions; and more preferably, the anti-TROP-2 antibody comprises a heavy chain constant region set forth in SEQ ID NO: 11 and a light chain constant region set forth in SEQ ID NO: 12.

**8.** The ligand-drug conjugate of general formula (Pc-L-Y-D) or the pharmaceutically acceptable salt thereof according to any one of claims 1 to 7, wherein the anti-TROP-2 antibody comprises a heavy chain set forth in SEQ ID NO: 13 and a light chain set forth in SEQ ID NO: 14.

**9.** The ligand-drug conjugate of general formula (Pc-L-Y-D) or the pharmaceutically acceptable salt thereof according to any one of claims 1 to 8, wherein n is a decimal or an integer from 2 to 10, preferably from 4 to 8.

**10.** The ligand-drug conjugate of general formula (Pc-L-Y-D) or the pharmaceutically acceptable salt thereof according to any one of claims 1 to 9,
wherein:

Y is $-O-(CR^aR^b)_m-CR^1R^2-C(O)-$;
$R^a$ and $R^b$ are identical or different and are each independently selected from the group consisting of hydrogen, deuterium, halogen and alkyl;
$R^1$ is haloalkyl or $C_{3-6}$ cycloalkyl;
$R^2$ is selected from the group consisting of hydrogen, haloalkyl and $C_{3-6}$ cycloalkyl;
or, $R^1$ and $R^2$, together with carbon atoms connected thereto, form $C_{3-6}$ cycloalkyl;
m is 0 or 1.

**11.** The ligand-drug conjugate of general formula (Pc-L-Y-D) or the pharmaceutically acceptable salt thereof according to any one of claims 1 to 10, wherein Y is selected from the group consisting of:

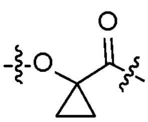 , 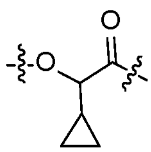 , 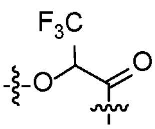 , 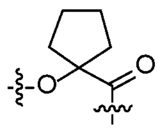 , 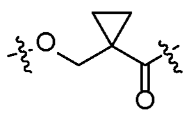 , 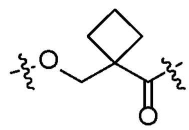 ,

and

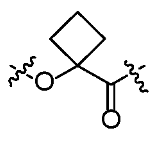 ;

wherein an O-terminus of Y is connected to the linker unit -L-.

**12.** The ligand-drug conjugate of general formula (Pc-L-Y-D) or the pharmaceutically acceptable salt thereof according to any one of claims 1 to 11, wherein the linker unit -L-is $-L^1-L^2-L^3-L^4-$, wherein

$L^1$ is selected from the group consisting of -(succinimidyl-3-yl-*N*)-W-C(O)-, $-CH_2-C(O)-NR^3-W-C(O)-$ and -C(O)-W-C(O)-, wherein W is selected from the group consisting of $C_{1-8}$ alkyl, $C_{1-8}$ alkyl-cycloalkyl and linear heteroalkyl of 1 to 8 chain atoms, and the heteroalkyl comprises 1 to 3 heteroatoms selected from the group consisting of N, O and S, wherein the $C_{1-8}$ alkyl, cycloalkyl and linear heteroalkyl are each independently

optionally further substituted with one or more substituents selected from the group consisting of halogen, hydroxy, cyano, amino, alkyl, chloroalkyl, deuterated alkyl, alkoxy and cycloalkyl;

$L^2$ is selected from the group consisting of $-NR^4(CH_2CH_2O)pCH_2CH_2C(O)-$, - $NR^4(CH_2CH_2O)pCH_2C(O)-$, $-S(CH_2)pC(O)-$ and a chemical bond, wherein p is an integer from 1 to 20;

$L^3$ is a peptide residue consisting of 2 to 7 amino acid residues, wherein the amino acid residues are selected from the group consisting of amino acid residues formed from amino acids from phenylalanine, glycine, valine, lysine, citrulline, serine, glutamic acid and aspartic acid, and are optionally further substituted with one or more substituents selected from the group consisting of halogen, hydroxy, cyano, amino, alkyl, chloroalkyl, deuterated alkyl, alkoxy and cycloalkyl;

$L^4$ is selected from the group consisting of $-NR^5(CR^6R^7)_t-$, $-C(O)NR^5$, $-C(O)NR^5(CH_2)_t-$ and a chemical bond, wherein t is an integer from 1 to 6;

$R^3$, $R^4$ and $R^5$ are identical or different and are each independently selected from the group consisting of hydrogen, alkyl, haloalkyl, deuterated alkyl and hydroxyalkyl;

$R^6$ and $R^7$ are identical or different and are each independently selected from the group consisting of hydrogen, halogen, alkyl, haloalkyl, deuterated alkyl and hydroxyalkyl.

**13.** The ligand-drug conjugate of general formula (Pc-L-Y-D) or the pharmaceutically acceptable salt thereof according to any one of claims 1 to 12, wherein the linker unit -L-is $-L^1-L^2-L^3-L^4-$, wherein

$L^1$ is

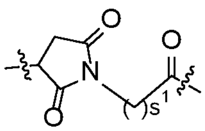

,

and $s^1$ is an integer from 2 to 8;

$L^2$ is a chemical bond;

$L^3$ is a tetrapeptide residue, preferably a tetrapeptide residue of GGFG;

$L^4$ is $-NR^5(CR^6R^7)t-$, wherein $R^5$, $R^6$ and $R^7$ are identical or different and are each independently hydrogen or alkyl, and t is 1 or 2;

wherein the $L^1$ terminus is connected to Pc, and the $L^4$ terminus is connected to Y.

**14.** The ligand-drug conjugate of general formula (Pc-L-Y-D) or the pharmaceutically acceptable salt thereof according to any one of claims 1 to 13, wherein -L- is:

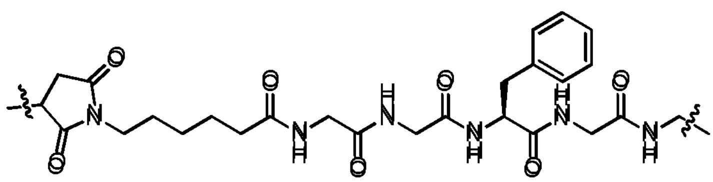

.

**15.** The ligand-drug conjugate of general formula (Pc-L-Y-D) or the pharmaceutically acceptable salt thereof according to any one of claims 1 to 14, wherein -L-Y- is optionally selected from the group consisting of:

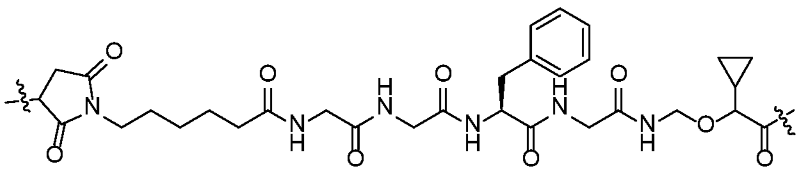

,

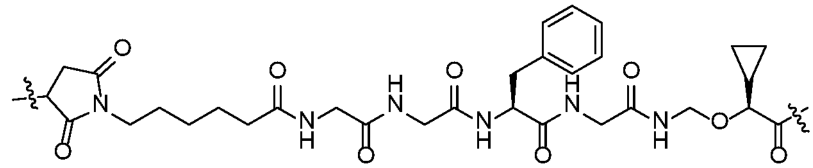

and

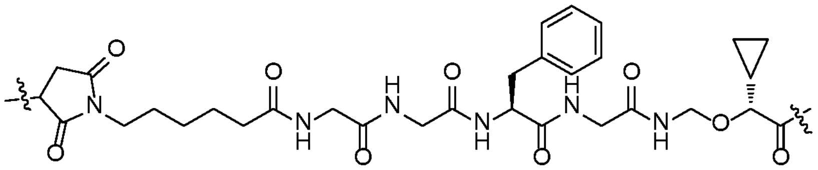

.

**16.** The ligand-drug conjugate of general formula (Pc-L-Y-D) or the pharmaceutically acceptable salt thereof according to any one of claims 1 to 12, wherein the ligand-drug conjugate of general formula (Pc-L-Y-D) or the pharmaceutically acceptable salt thereof is a ligand-drug conjugate of general formula ($Pc-L_a-Y-D$) or a pharmaceutically acceptable salt thereof,

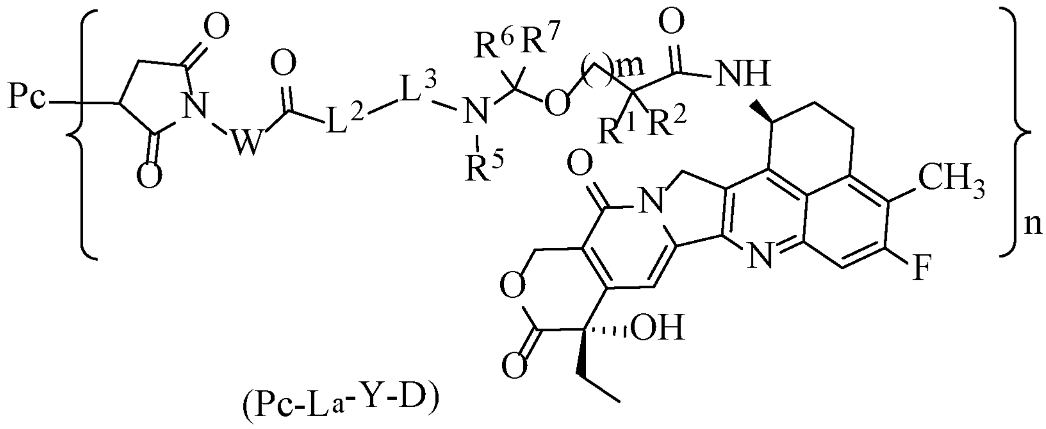

($Pc-L_a-Y-D$)

wherein Pc, n, m, W, $L^2$, $L^3$, $R^1$, $R^2$, $R^5$, $R^6$ and $R^7$ are as defined in any one of claims 1 to 12.

**17.** The ligand-drug conjugate of general formula (Pc-L-Y-D) or the pharmaceutically acceptable salt thereof according to any one of claims 1 to 13 and claim 16, wherein the ligand-drug conjugate of general formula (Pc-L-Y-D) or the pharmaceutically acceptable salt thereof is a ligand-drug conjugate of general formula ($Pc-L_b-Y-D$) or a pharmaceutically acceptable salt thereof,

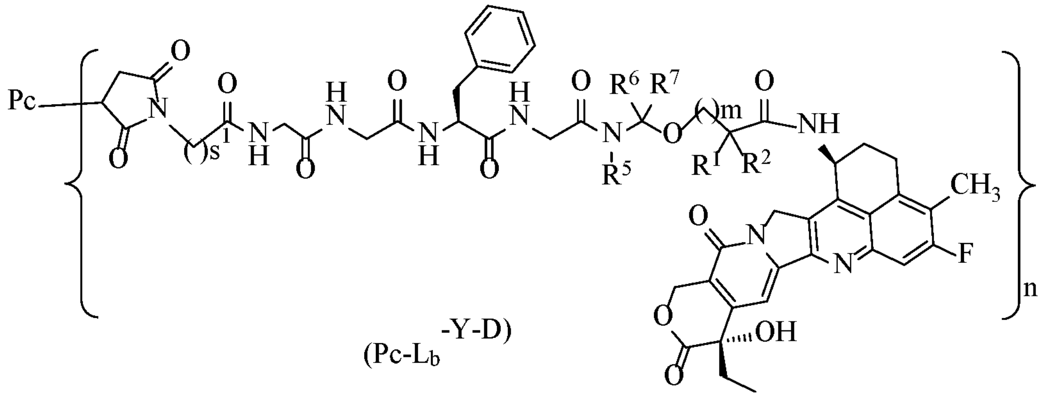

($Pc-L_b-Y-D$)

wherein:

$s^1$ is an integer from 2 to 8;
Pc, $R^1$, $R^2$, $R^5$-$R^7$, m and n are as defined in any one of claims 1 to 13.

**18.** The ligand-drug conjugate of general formula (Pc-L-Y-D) or the pharmaceutically acceptable salt thereof according to any one of claims 1 to 17, wherein the ligand-drug conjugate is selected from the group consisting of:

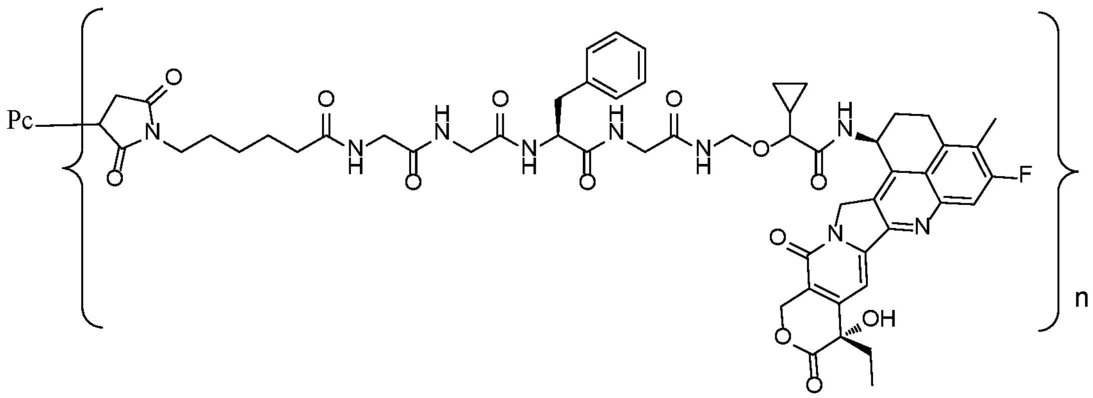

,

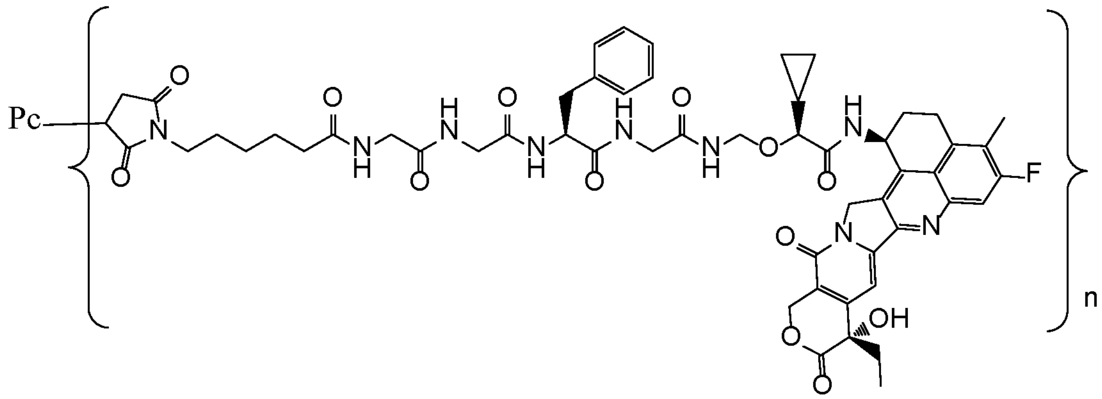

and

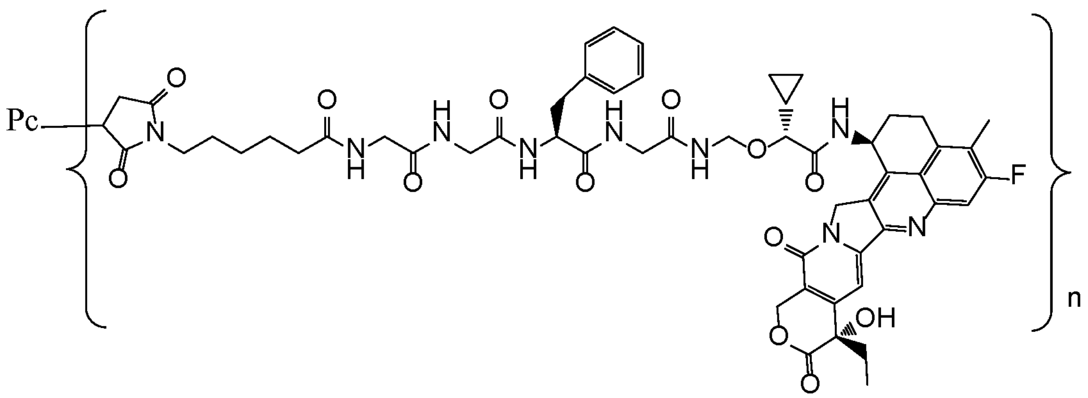

wherein Pc and n are as defined in any one of claims 1 to 17.

**19.** The ligand-drug conjugate of general formula (Pc-L-Y-D) or the pharmaceutically acceptable salt thereof according to any one of claims 1 to 18, wherein the ligand-drug conjugate is:

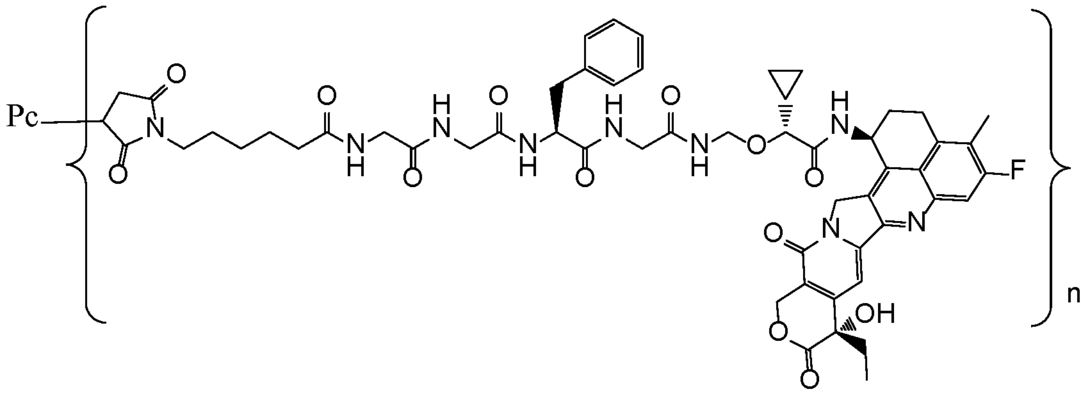

wherein:

n is a decimal or an integer from 1 to 8, preferably from 4 to 8, and more preferably from 4 to 6;
Pc is an anti-TROP-2 antibody comprising a heavy chain set forth in SEQ ID NO: 13 and a light chain set forth in SEQ ID NO: 14.

**20.** An anti-TROP-2 antibody or an antigen-binding fragment thereof, comprising a heavy chain variable region and a light chain variable region, wherein the heavy chain variable region comprises an HCDR1, an HCDR2 and an HCDR3 having sequences identical to those of an HCDR1, an HCDR2 and an HCDR3 of a heavy chain variable region set forth in SEQ ID NO: 3, and the light chain variable region comprises an LCDR1, an LCDR2 and an LCDR3 having sequences identical to those of an LCDR1, an LCDR2 and an LCDR3 of a light chain variable region set forth in SEQ ID NO: 4.

**21.** The anti-TROP-2 antibody or the antigen-binding fragment thereof according to claim 20, comprising a heavy chain variable region and a light chain variable region, wherein the heavy chain variable region comprises an HCDR1, an HCDR2 and an HCDR3 set forth in SEQ ID NO: 5, SEQ ID NO: 6 and SEQ ID NO: 7, respectively, and the light chain variable region comprises an LCDR1, an LCDR2 and an LCDR3 set forth in SEQ ID NO: 8, SEQ ID NO: 9 and SEQ ID NO: 10, respectively.

**22.** The anti-TROP-2 antibody or the antigen-binding fragment thereof according to claim 20 or 21, wherein the anti-TROP-2 antibody is a murine antibody, a chimeric antibody, a humanized antibody or a human antibody.

**23.** The anti-TROP-2 antibody or the antigen-binding fragment thereof according to claims 20 to 22, comprising a heavy chain variable region and a light chain variable region, wherein: the heavy chain variable region has an amino acid sequence set forth in SEQ ID NO: 3 or having at least 90% identity thereto, and the light chain variable region has an amino acid sequence set forth in SEQ ID NO: 4 or having at least 90% identity thereto.

**24.** The anti-TROP-2 antibody or the antigen-binding fragment thereof according to claims 20 to 23, wherein the anti-TROP-2 antibody or the antigen-binding fragment thereof comprises a heavy chain constant region and a light chain constant region of the antibody; preferably, the heavy chain constant region is selected from the group consisting of human IgG1, IgG2, IgG3 and IgG4 constant regions, and the light chain constant region is selected from the group consisting of human antibody $\kappa$ and $\lambda$ chain constant regions; and
more preferably, the anti-TROP-2 antibody comprises a heavy chain constant region set forth in SEQ ID NO: 11 and a light chain constant region set forth in SEQ ID NO: 12.

**25.** The anti-TROP-2 antibody or the antigen-binding fragment thereof according to claims 20 to 24, wherein the anti-TROP-2 antibody comprises a heavy chain set forth in SEQ ID NO: 13 and a light chain set forth in SEQ ID NO: 14.

**26.** A nucleic acid molecule encoding the anti-TROP-2 antibody or the antigen-binding fragment thereof according to any one of claims 20 to 25.

**27.** A host cell comprising the nucleic acid molecule according to claim 26.

**28.** A method for preparing a ligand-drug conjugate of general formula (Pc-$L_a$-Y-D) or a pharmaceutically acceptable

salt thereof, comprising the following steps:

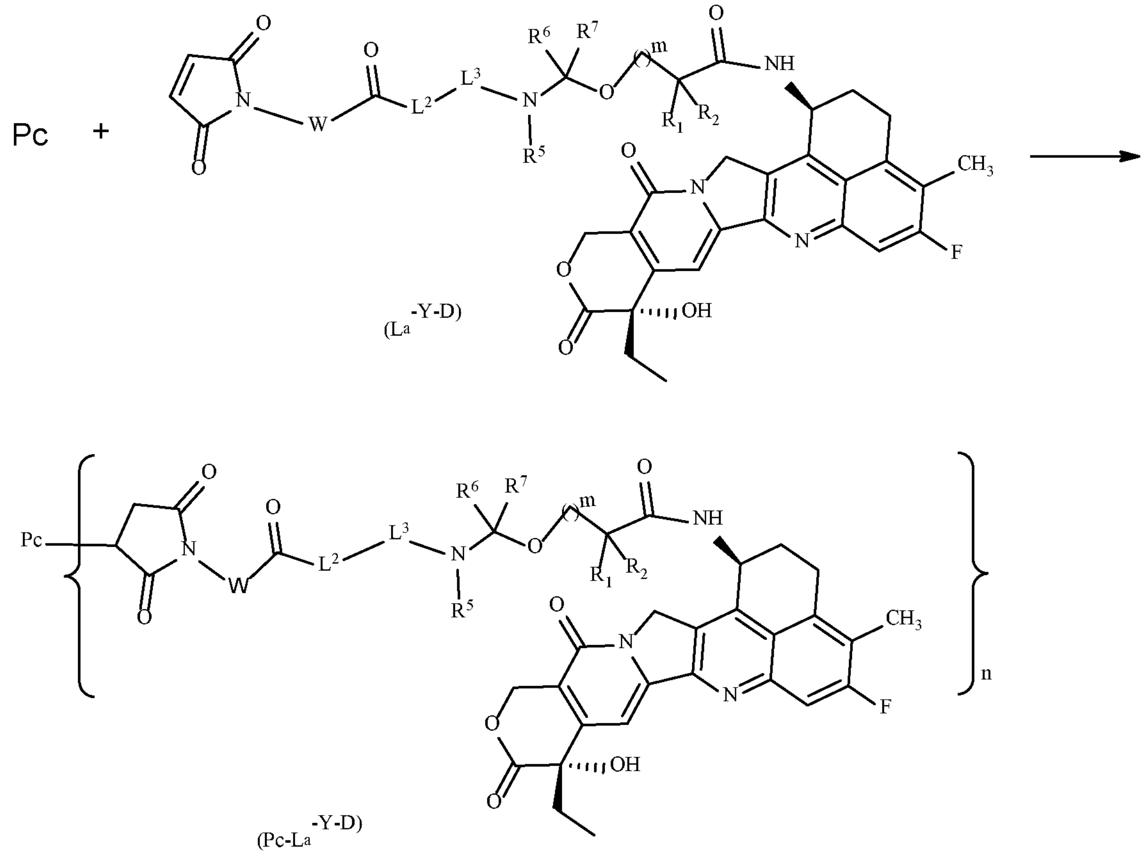

$(L_a\text{-}Y\text{-}D)$

$(Pc\text{-}L_a\text{-}Y\text{-}D)$

subjecting reduced Pc' and a compound of general formula ($L_a$-Y-D) to a coupling reaction to give a compound of general formula (Pc-$L_a$-Y-D);
wherein:

Pc is an anti-TROP-2 antibody or an antigen-binding fragment thereof; preferably, Pc is the anti-TROP-2 antibody or the antigen-binding fragment thereof according to any one of claims 20 to 25;
n, m, W, $L^2$, $L^3$, $R^1$, $R^2$, $R^5$, $R^6$ and $R^7$ are as defined in claim 16.

**29.** A pharmaceutical composition, comprising:

the antibody-drug conjugate or the pharmaceutically acceptable salt thereof according to any one of claims 1 to 19, or the anti-TROP-2 antibody or the antigen-binding fragment thereof according to any one of claims 20 to 25, and
one or more pharmaceutically acceptable excipients, diluents or carriers.

**30.** Use of the antibody-drug conjugate or the pharmaceutically acceptable salt thereof according to any one of claims 1 to 19, the anti-TROP-2 antibody or the antigen-binding fragment thereof according to any one of claims 20 to 25, or the pharmaceutical composition according to claim 29 in preparing a medicament for the treatment of a TROP-2-mediated disease or condition.

**31.** Use of the antibody-drug conjugate or the pharmaceutically acceptable salt thereof according to any one of claims 1 to 19, the anti-TROP-2 antibody or the antigen-binding fragment thereof according to any one of claims 20 to 25, or the pharmaceutical composition according to claim 29 in preparing a medicament for the treatment of tumors.

**32.** Use of the antibody-drug conjugate or the pharmaceutically acceptable salt thereof according to any one of claims 1 to 19, the anti-TROP-2 antibody or the antigen-binding fragment thereof according to any one of claims 20 to 25, or the pharmaceutical composition according to claim 29 in preparing a medicament for the treatment and/or prevention of tumors and cancers, wherein the tumors and cancers are preferably head and neck squamous cell carcinoma, head and neck cancer, brain cancer, neuroglioma, glioblastoma multiforme, neuroblastoma, central nervous system carcinoma, neuroendocrine tumor, throat cancer, pharyngeal squamous cell carcinoma, oral squamous cell carcinoma, nasopharyngeal cancer, esophageal cancer, thyroid cancer, malignant pleural mesothelioma, lung cancer, breast cancer, liver cancer, hepatobiliary cancer, pancreatic cancer, stomach cancer, gastrointestinal cancer, intestinal cancer, colon cancer, colorectal cancer, kidney cancer, clear cell renal cell carcinoma, ovarian cancer, endometrial cancer, cervical cancer, bladder cancer, prostate cancer, testicular cancer, skin cancer, melanoma, leukemia, lymphoma, bone cancer, chondrosarcoma, myeloma, multiple myeloma, myelodysplastic syndrome, Krukenberg tumor, myeloproliferative tumor, squamous cell carcinoma, Ewing’s sarcoma, urothelial carcinoma or Merkel cell carcinoma; more preferably, the lymphoma is selected from the group consisting of Hodgkin’s lymphoma, non-Hodgkin’s lymphoma, diffuse large B-cell lymphoma, follicular lymphoma, primary mediastinal large B-cell lymphoma, mantle cell lymphoma, small lymphocytic lymphoma, large B-cell lymphoma rich in T-cells/histiocytes and lymphoplasmacytic lymphoma, the lung cancer is selected from the group consisting of non-small cell lung cancer and small cell lung cancer, and the leukemia is selected from the group consisting of chronic myeloid leukemia, acute myeloid leukemia, lymphocytic leukemia, lymphoblastic leukemia, acute lymphoblastic leukemia, chronic lymphocytic leukemia and myeloid cell leukemia.

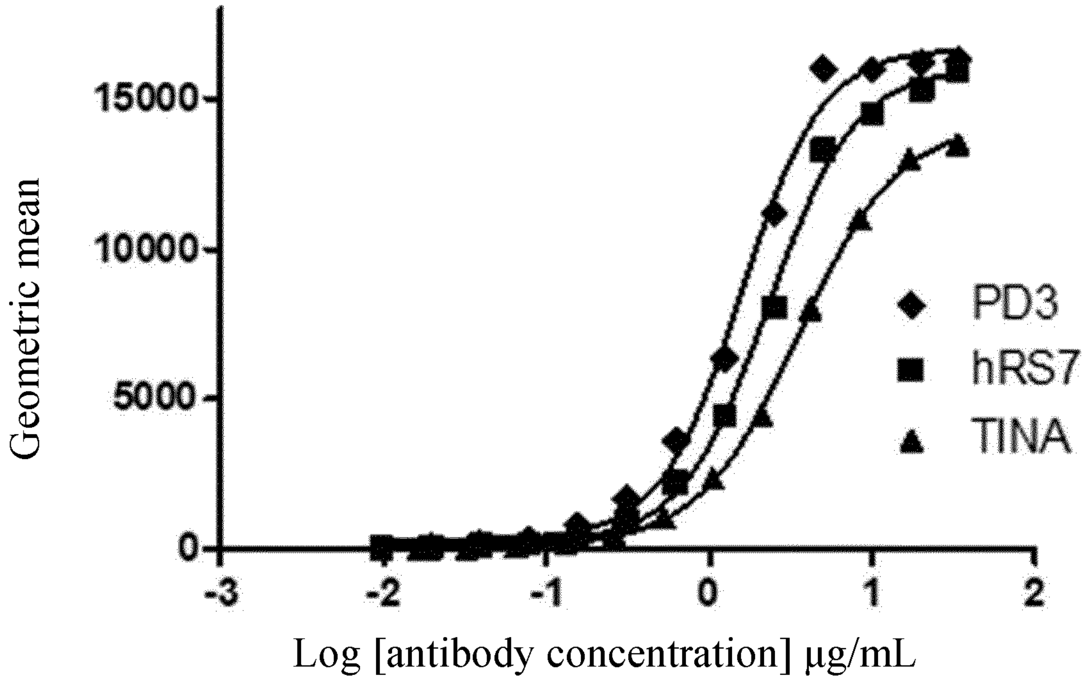
15000
10000
5000
0
-3
-2
-1
0
1
2
Geometric mean
Log [antibody concentration] µg/mL
PD3
hRS7
TINA

**FIG. 1**

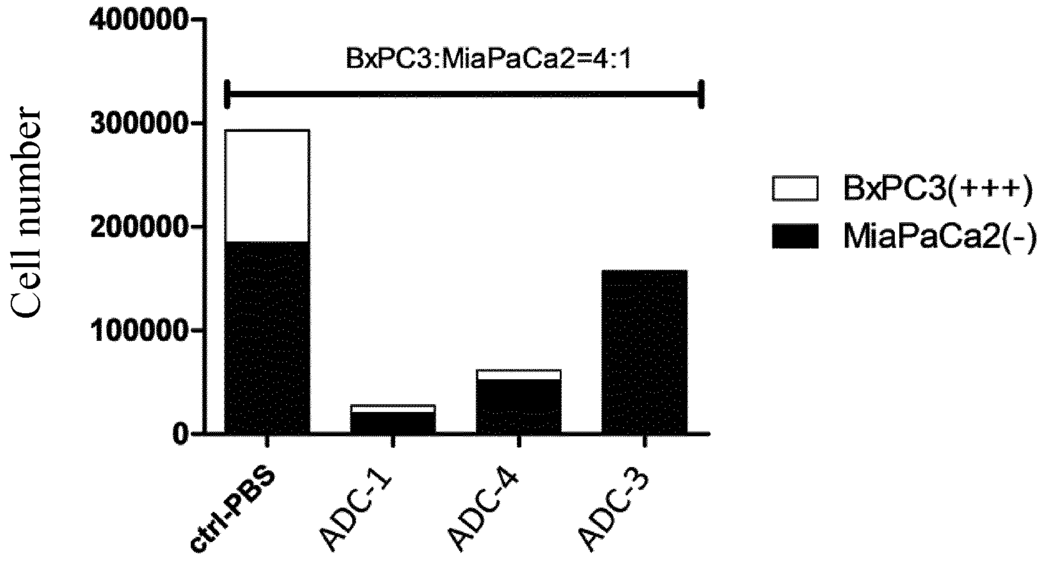
400000
300000
200000
100000
0
Cell number
BxPC3:MiaPaCa2=4:1
BxPC3(+++)
MiaPaCa2(-)
ctrl-PBS
ADC-1
ADC-4
ADC-3

**FIG. 2**

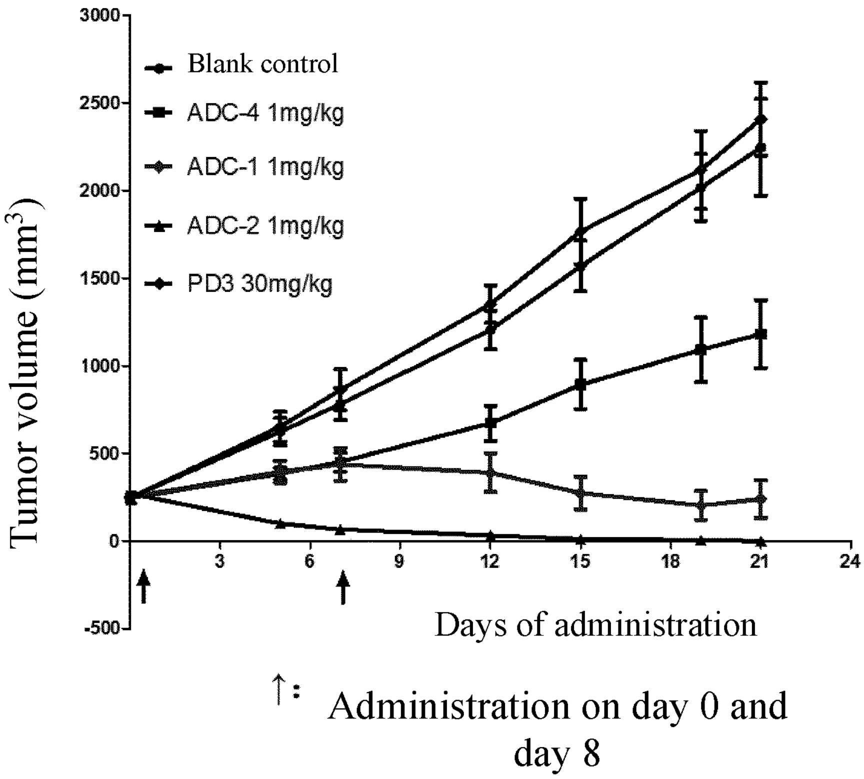
3000
2500
2000
1500
1000
500
0
-500
Tumor volume (mm³)
Blank control
ADC-4 1mg/kg
ADC-1 1mg/kg
ADC-2 1mg/kg
PD3 30mg/kg
3
6
9
12
15
18
21
24
Days of administration
↑: Administration on day 0 and day 8

**FIG. 3**

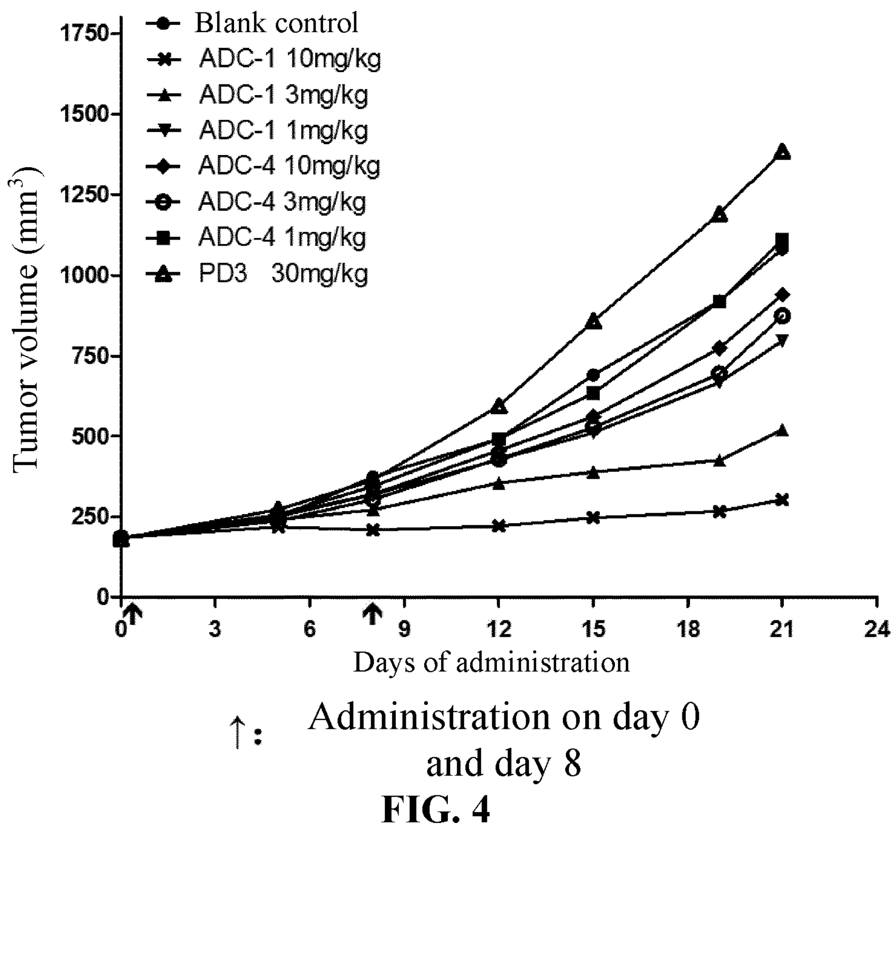
1750
1500
1250
1000
750
500
250
0
Tumor volume (mm³)
Blank control
ADC-1 10mg/kg
ADC-1 3mg/kg
ADC-1 1mg/kg
ADC-4 10mg/kg
ADC-4 3mg/kg
ADC-4 1mg/kg
PD3 30mg/kg
0
3
6
9
12
15
18
21
24
Days of administration
↑: Administration on day 0 and day 8

**FIG. 4**

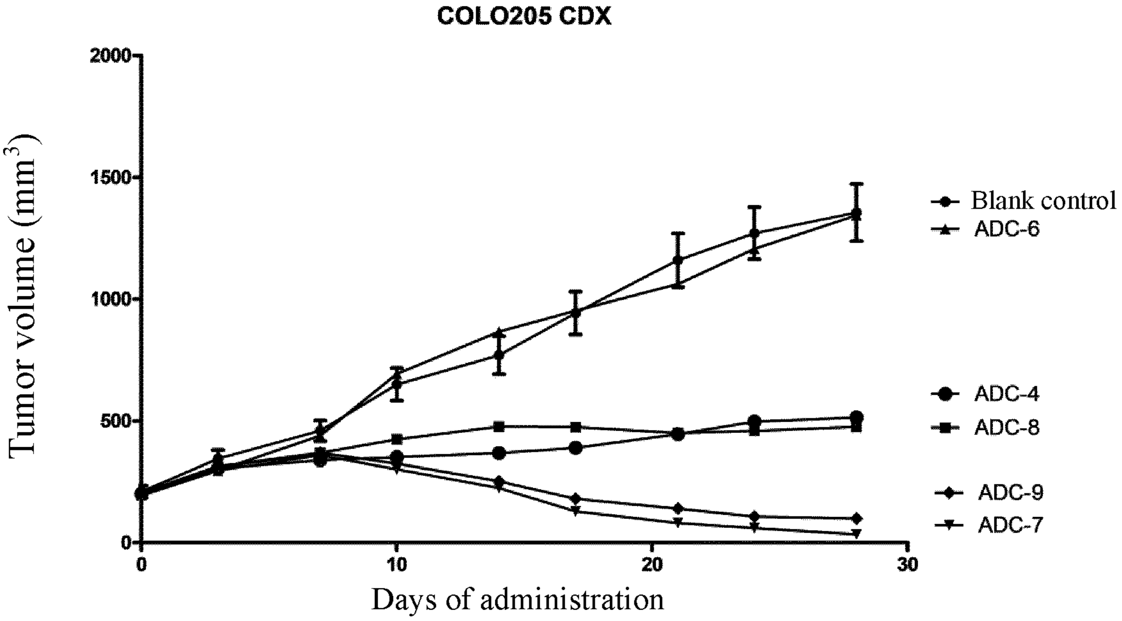
COLO205 CDX
Tumor volume (mm3)
2000
1500
1000
500
0
0
10
20
30
Days of administration
Blank control
ADC-6
ADC-4
ADC-8
ADC-9
ADC-7

**FIG. 5**

**INTERNATIONAL SEARCH REPORT**

International application No.
**PCT/CN2021/073279**

**A. CLASSIFICATION OF SUBJECT MATTER**

C07K 7/02(2006.01)i; C07K 5/062(2006.01)i; A61K 39/395(2006.01)i; A61K 31/506(2006.01)i; C07D 491/22(2006.01)i; A61P 35/00(2006.01)i

According to International Patent Classification (IPC) or to both national classification and IPC

**B. FIELDS SEARCHED**

Minimum documentation searched (classification system followed by classification symbols)

C07K, C07D, A61K, A61P

Documentation searched other than minimum documentation to the extent that such documents are included in the fields searched

Electronic data base consulted during the international search (name of data base and, where practicable, search terms used)

CNABS, CNTXT, DWPI, SIPOABS, CATXT, USTXT, EPTXT, WOTXT, CPEA, CNKI, PUBMED, WEB OF SCIENCE, ELSEVIER, 百度学术, GenBank, 中国专利生物序列检索系统: TROP-2, 抗体, EGP-1, GAT33, TACSTD2, 依喜替康, 依沙替康, 缀合物, 偶联物, 癌症, 肿瘤, anti-TROP-2, exatecan, cancer, tumor, carcinoma, conjugate, search based on sequence SEQ ID NOs: 3-14

**C. DOCUMENTS CONSIDERED TO BE RELEVANT**

| Category* | Citation of document, with indication, where appropriate, of the relevant passages | Relevant to claim No. |
|---|---|---|
| X | Thomas MC et al. "Humanized Anti-Trop-2 IgG-SN-38 Conjugate for Effective Treatment of Diverse Epithelial Cancers: Preclinical Studies in Human Cancer Xenograft Models and Monkeys" *Clinical Cancer Research,* Vol. 17, No. 10, 03 March 2011 (2011-03-03), pages 3157-3169 see abstract, figure 1A | 1-32 |
| X | David MG et al. "Trop-2 is a novel target for solid cancer therapy with sacituzumab govitecan (IMMU-132), an antibody-drug conjugate (ADC)" *ONCOTARGET,* Vol. 6, No. 26, 18 June 2015 (2015-06-18), pages 22496-22512 see abstract, figures 1A and 1B | 1-32 |
| X | Thomas MC et al. "Sacituzumab Govitecan (IMMU-132), an Anti-Trop-2/SN-38 Antibody-Drug Conjugate: Characterization and Efficacy in Pancreatic, Gastric, and other Cancers" *Bioconjugate Chemistry,* Vol. 26, No. 5, 27 April 2015 (2015-04-27), pp. 919-931, see abstract | 1-32 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 April 2021** | **29 April 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

INTERNATIONAL SEARCH REPORT

International application No.
PCT/CN2021/073279

C. DOCUMENTS CONSIDERED TO BE RELEVANT

| Category* | Citation of document, with indication, where appropriate, of the relevant passages | Relevant to claim No. |
|---|---|---|
| X | WO 2019114666 A1 (SICHUAN KELUN BIOTECH BIOPHARMACEUTICAL CO., LTD.) 20 June 2019 (2019-06-20) see description page 19 line 3, page 38 line 8 to page 40 line 15, page 79 line 20 to page 80 line 4, page 85 line 1 to page 86 line 11 | 1-32 |
| X | WO 2015047510 A1 (IMMUNOMEDICS INC.) 02 April 2015 (2015-04-02) see description paragraphs [0007]-[0011] and [0200]-[204] | 1-32 |
| X | CN 110526978 A (BIO-THERA SOLUTIONS, LTD.) 03 December 2019 (2019-12-03) see description paragraphs [0097]-[0099] and [0173]-[0177], sequence table SEQ ID NOs: 1-4 | 20-27 |

**INTERNATIONAL SEARCH REPORT**

International application No.
**PCT/CN2021/073279**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/CN2021/073279**

| Patent document cited in search report | | | Publication date (day/month/year) | Patent family member(s) | | | Publication date (day/month/year) |
|---|---|---|---|---|---|---|---|
| WO | 2019114666 | A1 | 20 June 2019 | KR | 20200099123 | A | 21 August 2020 |
| | | | | EP | 3725798 | A1 | 21 October 2020 |
| | | | | CN | 111295389 | A | 16 June 2020 |
| | | | | CA | 3080236 | A1 | 20 June 2019 |
| | | | | US | 2020347075 | A1 | 05 November 2020 |
| WO | 2015047510 | A1 | 02 April 2015 | EP | 3049443 | A1 | 03 August 2016 |
| | | | | CA | 2920192 | A1 | 02 April 2015 |
| | | | | EP | 3049443 | A4 | 05 April 2017 |
| CN | 110526978 | A | 03 December 2019 | AU | 2018267660 | C1 | 12 November 2020 |
| | | | | CN | 111087471 | A | 01 May 2020 |
| | | | | JP | 2020503243 | A | 30 January 2020 |
| | | | | EP | 3464381 | A1 | 10 April 2019 |
| | | | | AU | 2018267660 | A1 | 28 February 2019 |
| | | | | CN | 111018992 | A | 17 April 2020 |
| | | | | WO | 2019029715 | A1 | 14 February 2019 |
| | | | | AU | 2018267660 | B2 | 07 May 2020 |
| | | | | CN | 107446050 | A | 08 December 2017 |
| | | | | US | 2019048095 | A1 | 14 February 2019 |
| | | | | CN | 109078181 | A | 25 December 2018 |
| | | | | CN | 109078181 | B | 05 November 2019 |
| | | | | EP | 3464381 | A4 | 11 December 2019 |
| | | | | CA | 3038423 | A1 | 14 February 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202010073438
- US 20050238649 A1
- US 5208020 A
- WO 03074566 A
- WO 2015098099 A1
- CN 2019107873 W
- EP 0737686 A1
- WO 2013106717 A
- WO 201396771 A
- US 200520645 B
- CN 105829346 A
- EP 2907824 A
- WO 2017063509 A
- WO 2015098099 A
- CN 104755494 A
- CN 105407891 A


### Non-patent literature cited in the description


- **CHARI et al.** *Cancer Research,* 1992, vol. 52, 127-131
- *J. biol. chem,* 1968, vol. 243, 3558
- **WARD et al.** *Nature,* 1989, vol. 341, 544-546
- **BIRD et al.** *Science,* 1988, vol. 242, 423-426
- **HUSTON et al.** *Proc. Natl. Acad. Sci USA,* 1988, vol. 85, 5879-5883
- **HOLLIGER et al.** *Proc. Natl. Acad. Sci. USA,* 1993, vol. 90, 6444-6448
- **ALFTHAN et al.** *Protein Eng.,* 1995, vol. 8, 725-731
- **CHOI et al.** *Eur. J. Immunol.,* 2001, vol. 31, 94-106
- **HU et al.** *Cancer Res.,* 1996, vol. 56, 3055-3061
- **KIPRIYANOV et al.** *J. Mol. Biol.,* 1999, vol. 293, 41-56
- **ROOVERS et al.** *Cancer Immunol.,* 2001
- **KABAT et al.** Sequences of Proteins of Immunological Interest. Public Health Service, National Institutes of Health, 1991
- **AL-LAZIKANI et al.** *JMB,* 1997, vol. 273, 927-948
- **LEFRANC M.P.** *Immunologist,* 1999, vol. 7, 132-136
- **LEFRANC, M.P. et al.** *Dev. Comp. Immunol.,* 2003, vol. 27, 55-77
- Epitope Mapping Protocols in Methods in Molecular B iology. 1996, vol. 66
- Antibodies: A Laboratory Manual. Cold Spring Harbor Press
- **LEFRANC, G.** The Immunoglobulin FactsBook. Academic Press
- **SAMBROOK et al.** Molecular Cloning: A Laboratory Manual. Cold Spring Harbor Laboratory Press
- **AUSUBEL et al.** CurrentProtocols in Molecular Biology. Greene Publishing Association
- *Tetrahedron Letters,* 1984, vol. 25 (12), 1269-72
- *Journal of the American Chemical Society,* 2014, vol. 136 (22), 8138-8142